(19) Europäisches Patentamt
European Patent Office
Office européen des brevets

(11) EP 4 450 495 A1

(12) EUROPEAN PATENT APPLICATION
published in accordance with Art. 153(4) EPC

(43) Date of publication:
23.10.2024 Bulletin 2024/43

(21) Application number: 22919900.5

(22) Date of filing: 28.10.2022

(51) International Patent Classification (IPC):
C07D 401/14 (2006.01) C07D 471/04 (2006.01)
C07D 491/04 (2006.01) C07D 491/08 (2006.01)
C07D 491/10 (2006.01) C07D 519/00 (2006.01)
A61P 35/00 (2006.01) A61K 31/506 (2006.01)

(52) Cooperative Patent Classification (CPC):
A61K 31/506; A61K 31/517; A61K 31/519; A61P 35/00; C07D 401/14; C07D 405/14; C07D 471/04; C07D 491/04; C07D 491/08; C07D 491/10; C07D 519/00

(86) International application number:
PCT/CN2022/128264

(87) International publication number:
WO 2023/134266 (20.07.2023 Gazette 2023/29)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 17.01.2022 PCT/CN2022/072306
12.05.2022 PCT/CN2022/092375

(71) Applicant: Suzhou Puhe Biopharma Co., Ltd.
Suzhou, Jiangsu 215124 (CN)

(72) Inventors:
- LIU, Bin
Suzhou, Jiangsu 215124 (CN)
- GAO, Feng
Suzhou, Jiangsu 215124 (CN)
- YUAN, Shuai
Suzhou, Jiangsu 215124 (CN)
- ZHAO, Hui
Suzhou, Jiangsu 215124 (CN)
- PENG, Xingzhe
Suzhou, Jiangsu 215124 (CN)
- LIU, Chong
Beijing 102206 (CN)
- SHAO, Ning
Beijing 102206 (CN)
- GUO, Yongqi
Suzhou, Jiangsu 215124 (CN)
- WU, Yongyong
Suzhou, Jiangsu 215124 (CN)
- WU, Zhuo
Suzhou, Jiangsu 215124 (CN)

(74) Representative: Cabinet Becker et Associés
25, rue Louis le Grand
75002 Paris (FR)

# (54) 2-PIPERIDYL OR 2-PYRAZOLYL SUBSTITUTED PYRIMIDINE COMPOUND SERVING AS EGFR INHIBITOR

(57) A 2-piperidyl or 2-pyrazolyl substituted pyrimidine compound, which is a compound represented by formula (I) or a pharmaceutically acceptable salt, an isotopic variant, a tautomer, a stereoisomer, a prodrug, a polymorph, a hydrate or a solvate thereof. A pharmaceutical composition comprising the compound, and a use of the pharmaceutical composition in the treatment of diseases mediated by an EGFR protein and mutants thereof.

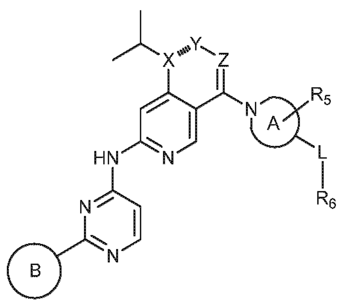

(I)

EP 4 450 495 A1

## Description

### TECHNICAL FIELD

The present disclosure belongs to the field of medicine, and specifically relates to pyrimidine compounds substituted with 2-piperidinyl or 2-pyrazolyl as EGFR inhibitors.

### BACKGROUND

Lung cancer is one of the most common malignant tumors, with approximately 1.6 million new cases of lung cancer worldwide each year. Lung cancer has two types: small cell lung cancer and non-small cell lung cancer (NSCLC), of which non-small cell lung cancer accounts for approximately 85% of all lung cancers (Nature Reviews Disease Primers, 2015, 1,15009). Epidermal growth factor receptor (EGFR) is the most common driver gene for non-small cell lung cancer, with a positive rate of 17% in all non-small cell lung cancers, close to 30% to 40% in domestic patients of China, and as high as about 60% in lung adenocarcinoma.

EGFR is a transmembrane glycoprotein that belongs to the ErbB family of tyrosine kinase receptors. EGFR is abnormally activated by various mechanisms, such as receptor overexpression, mutation, ligand-dependent receptor dimerization, and ligand-independent activation. The continuous activation of its kinase activity initiates downstream signal transduction of cell proliferation, differentiation, and survival. Small molecule inhibitors of EGFR kinase can inhibit the activation of tyrosine kinase, inhibit the proliferation of tumor cells, promote the apoptosis of tumor cells and other biological effects, and are a hot area for lung cancer development.

Osimertinib is a drug developed for first- and second-generation drug-resistant mutations of EGFR (del19 or L858R) accompanied by T790M mutations. It has shown very significant efficacy in clinical practice, but patients will also develop drug resistance as treatment continues. In 2015 (Nature Medicine, 2015, 21, 560-562), the drug resistance data of 15 patients taking osimertinib were first reported, among which EGFR C797S mutation was one of main mechanisms leading to drug resistance to osimertinib, accounting for about 40%. In addition, the latest literature reports show that among patients with resistance after second-line treatment with osimertinib, 22-25% have C797S mutations (Nature Cancer, 2021, 377-391). Therefore, there is an urgent clinical need to develop new small molecule inhibitors targeting C797S mutations to provide patients with safer and more effective fourth-generation EGFR inhibitors.

On the other hand, although the first-generation EGFR inhibitor gefitinib and the third-generation EGFR inhibitor osimertinib both produced good therapeutic effects in the first-line population with EGFR activating mutations, in subgroup analysis, the benefits of the L858R population were much lower than those of the del19 population, with PFS of 14.4 and 21.4 months, respectively (The new England journal of medicine, 2018, 378, 113-125).

### SUMMARY

In the present disclosure, we used the main mutation types that appear in the clinic, such as L858R/C797S, L858R/T790M/C797S and L858R, to carry out enzymatic evaluation and verify it at the constructed Ba/F3 cell level, and finally discovered a series of new chemical entities with strong biological activity.

In one aspect, the present disclosure provides a compound of formula (I), or a pharmaceutically acceptable salt, isotopic variant, tautomer, stereoisomer, prodrug, polymorph, hydrate or solvate thereof:

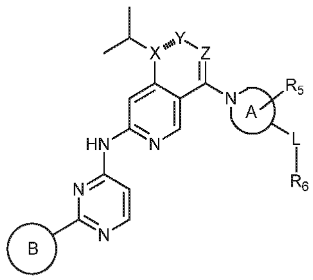

(I)

wherein:

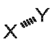 represents 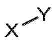 or 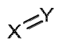; wherein when 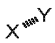 is 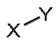, X is selected from CH and N, and Y is selected from $CH_2$, C=O and C=S; and when 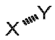 is 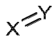, X is C, and Y is CH;

Z is selected from CH and N;
ring A is 4- to 8-membered heterocyclyl;
ring B is selected from

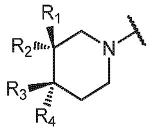 and 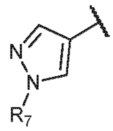 ;

L is selected from a chemical bond, -S(=O)(=M)-, $-N(R_N)-S(=O)(=M)-$, $-S(=O)(=M)-N(R_N)-$, $-N(R_N)-C(=O)-$, $-C(=O)-N(R_N)-$, $-CH(R_N)-C(=O)-$, $-C(=O)-CH(R_N)-$, $-CH(R_N)-S(=O)(=M)-$ and $-S(=O)(=M)-CH(R_N)-$;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, $-C_{1-6}$ alkylene-$C_{3-6}$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl;
provided that, when L is $-CH(R_N)-S(=O)(=M)-$, and the ring where X, Y and Z are located is a benzene ring, B is

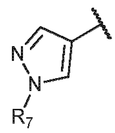 .

In another aspect, the present disclosure provides a pharmaceutical composition comprising a compound of the present disclosure, and optionally pharmaceutically acceptable excipients.
In another aspect, the present disclosure provides a pharmaceutical composition comprising a compound of the present disclosure and pharmaceutically acceptable excipients, which also contain other therapeutic agents.
In another aspect, the present disclosure provides use of the compound of the present disclosure in the manufacture of a medicament for treating and/or preventing a disease mediated by EGFR protein and mutants thereof.
In another aspect, the present disclosure provides a method for treating and/or preventing a disease mediated by EGFR protein and mutants thereof in a subject, comprising administering a compound or composition of the present disclosure to the subject.
In another aspect, the present disclosure provides a compound or a composition of the present disclosure, for use in treating and/or preventing a disease mediated by EGFR protein and mutants thereof.
In a specific embodiment, the EGFR mutant is one or more of the L858R mutant, del19 mutant, T790M mutant, and C797S mutant; optionally, the disease is selected from lung cancer, colon cancer, urothelial carcinoma, breast cancer, prostate cancer, brain cancer, ovarian cancer, gastric cancer, pancreatic cancer, head and neck cancer, bladder cancer and mesothelioma, alternatively non-small cell lung cancer.
Other objects and advantages of the present disclosure will be apparent to those skilled in the art from the following detailed embodiments, examples and claims.

## Definition

### Chemical definitions

Definitions of specific functional groups and chemical terms are described in more detail below.
When a range of values is listed, it is intended to encompass each value and sub-range within the range. For

example, "$C_{1-6}$ alkyl" is intended to include $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-6}$ alkyl" refers to a radical of a straight or branched, saturated hydrocarbon group having 1 to 6 carbon atoms. In some embodiments, $C_{1-4}$ alkyl and $C_{1-2}$ alkyl are alternative. Examples of $C_{1-6}$ alkyl include methyl ($C_1$), ethyl ($C_2$), n-propyl ($C_3$), iso-propyl ($C_3$), n-butyl ($C_4$), tert-butyl ($C_4$), sec-butyl ($C_4$), iso-butyl ($C_4$), n-pentyl ($C_5$), 3-pentyl ($C_5$), pentyl ($C_5$), neopentyl ($C_5$), 3-methyl-2-butyl ($C_5$), tert-pentyl (Cs) and n-hexyl ($C_6$). The term "$C_{1-6}$ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me ($-CH_3$), Et ($-CH_2CH_3$), iPr ($-CH(CH_3)_2$), nPr ($-CH_2CH_2CH_3$), n-Bu ($-CH_2CH_2CH_2CH_3$) or i-Bu ($-CH_2CH(CH_3)_2$).

"$C_{2-6}$ alkenyl" refers to a radical of a straight or branched hydrocarbon group having 2 to 6 carbon atoms and at least one carbon-carbon double bond. In some embodiments, $C_{2-4}$ alkenyl is alternative. Examples of $C_{2-6}$ alkenyl include vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"$C_{2-6}$ alkynyl" refers to a radical of a straight or branched hydrocarbon group having 2 to 6 carbon atoms, at least one carbon-carbon triple bond and optionally one or more carbon-carbon double bonds. In some embodiments, $C_{2-4}$ alkynyl is alternative. Examples of $C_{2-6}$ alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"$C_{1-6}$ alkylene" refers to a divalent group formed by removing another hydrogen of the $C_{1-6}$ alkyl, and can be substituted or unsubstituted. In some embodiments, $C_{1-4}$ alkylene, $C_{2-4}$ alkylene, and $C_{1-3}$ alkylene are alternative. The unsubstituted alkylene groups include, but are not limited to, methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Examples of substituted alkylene groups, such as those substituted with one or more alkyl (methyl) groups, include, but are not limited to, substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $- CH_2C(CH_3)_2CH_2-$, $-CH_2CH_2C(CH_3)_2-$), etc.

"$C_{0-6}$ alkylene" refers to a chemical bond and the above-mentioned $C_{1-6}$ alkylene.

"$C_{2-6}$ alkenylene" refers to a $C_{2-6}$ alkenyl group wherein another hydrogen is removed to provide a divalent radical of alkenylene, and which may be substituted or unsubstituted. In some embodiments, $C_{2-4}$ alkenylene is yet alternative. Exemplary unsubstituted alkenylene groups include, but are not limited to, ethenylene (-CH=CH-) and propenylene (e.g., $-CH=CHCH_2-$, $-CH_2-CH=CH-$). Exemplary substituted alkenylene groups, e.g., substituted with one or more alkyl (methyl) groups, include but are not limited to, substituted ethylene ($-C(CH_3)=CH-$, $- CH=C(CH_3)-$), substituted propylene (e.g., $-C(CH_3)=CHCH_2-$, $-CH=C(CH_3)CH_2-$, $- CH=CHCH(CH_3)-$, $-CH=CHC(CH_3)_2-$, $-CH(CH_3)-CH=CH-$, $-C(CH_3)_2-CH=CH-$, $-CH_2-C(CH_3)=CH-$, $-CH_2-CH=C(CH_3)-$), and the like.

"$C_{2-6}$ alkynylene" refers to a $C_{2-6}$ alkynyl group wherein another hydrogen is removed to provide a divalent radical of alkynylene, and which may be substituted or unsubstituted. In some embodiments, $C_{2-4}$ alkynylene is yet alternative. Exemplary alkynylene groups include, but are not limited to, ethynylene (-C=C-), substituted or unsubstituted propynylene ($-C\equiv CCH_2-$), and the like.

"Halo" or "halogen" refers to fluorine (F), chlorine (Cl), bromine (Br) and iodine (I).

Thus, "$C_{1-6}$ haloalkyl" refers to the above "$C_{1-6}$ alkyl", which is substituted by one or more halogen. In some embodiments, $C_{1-4}$ haloalkyl is yet alternative, and still alternatively $C_{1-2}$ haloalkyl. Exemplary haloalkyl groups include, but are not limited to, $-CF_3$, $-CH_2F$, $-CHF_2$, $- CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, and the like. The haloalkyl can be substituted at any available point of attachment, for example, with 1 to 5 substituents, 1 to 3 substituents or 1 substituent.

"$C_{3-10}$ cycloalkyl" refers to a radical of a non-aromatic cyclic hydrocarbon group having from 3 to 10 ring carbon atoms and zero heteroatoms. In some embodiments, $C_{5-7}$ cycloalkyl, $C_{3-6}$ cycloalkyl and $C_{3-5}$ cycloalkyl are yet alternative, and still alternatively $C_{5-6}$ cycloalkyl. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl can be substituted with one or more

substituents, for example, with 1 to 5 substituents, 1 to 3 substituents or 1 substituent.

"3- to 12-membered heterocyclyl" refers to a radical of 3- to 12-membered non-aromatic ring system having ring carbon atoms and 1 to 5 ring heteroatoms, wherein each of the heteroatoms is independently selected from nitrogen, oxygen, sulfur, boron, phosphorus and silicon. In the heterocyclyl containing one or more nitrogen atoms, the point of attachment can be a carbon or nitrogen atom as long as the valence permits. In some embodiments, 4- to 8-membered heterocyclyl is alternative, which is a radical of 4- to 8-membered non-aromatic ring system having ring carbon atoms and 1 to 5 ring heteroatoms. In some embodiments, 5- to 8-membered heterocyclyl is alternative, which is a radical of 5- to 8-membered non-aromatic ring system having ring carbon atoms and 1 to 5 ring heteroatoms. In some embodiments, 3- to 8-membered heterocyclyl is alternative, which is a radical of 3- to 8-membered non-aromatic ring system having ring carbon atoms and 1 to 4 ring heteroatoms. 3- to 6-membered heterocyclyl is alternative, which is a radical of 3- to 6-membered non-aromatic ring system having ring carbon atoms and 1 to 3 ring heteroatoms. 4- to 7-membered heterocyclyl is alternative, which is a radical of 4- to 7-membered non-aromatic ring system having ring carbon atoms and 1 to 3 ring heteroatoms. 4- to 6-membered heterocyclyl is alternative, which is a radical of 4- to 6-membered non-aromatic ring system having ring carbon atoms and 1 to 3 ring heteroatoms. 5- to 6-membered heterocyclyl is more alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc. The heterocyclyl can be substituted with one or more substituents, for example, with 1 to 5 substituents, 1 to 3 substituents or 1 substituent.

"$C_{6-10}$ aryl" refers to a radical of monocyclic or polycyclic (e.g., bicyclic) 4n+2 aromatic ring system having 6-10 ring carbon atoms and zero heteroatoms (e.g., having 6 or 10 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5- to 14-membered heteroaryl" refers to a radical of 5- to 14-membered monocyclic or bicyclic 4n+2 aromatic ring system (e.g., having 6, 10 or 14 shared $\pi$ electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 10-membered heteroaryl groups are alternative, which are radicals of 5- to 10-membered monocyclic or bicyclic 4n+2 aromatic ring systems having ring carbon atoms and 1-4 ring heteroatoms. In some embodiments, 5- to 9-membered heteroaryl groups are alternative, which are radicals of 5- to 9-membered monocyclic or bicyclic 4n+2 aromatic ring systems having ring carbon atoms and 1-4 ring heteroatoms. In other embodiments, 5- to 6-membered heteroaryl groups are yet alternative, which are radicals of 5- to 6-membered monocyclic 4n+2 aromatic ring systems having ring carbon atoms and 1-4 ring heteroatoms. Exemplary 5-membered heteroaryl groups containing one heteroatom include, but are not limited to, pyrrolyl, furyl and

thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4- oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"Cycloalkylene", "heterocyclylene", "arylene" or "heteroarylene" is a divalent group formed by removing another hydrogen from "cycloalkyl", "heterocyclyl", "aryl" or "heteroaryl" as defined above, and may be substituted or unsubstituted. For example, "$C_{5-7}$ cycloalkylene" refers to a divalent group formed by removing another hydrogen from $C_{5-7}$ cycloalkyl, "5- to 8-membered heterocyclylene" refers to a divalent group formed by removing another hydrogen from 5- to 8-membered heterocyclyl, "$C_{6-10}$ arylene" refers to a divalent group formed by removing another hydrogen from $C_{6-10}$ aryl, and "5- to 6-membered heteroarylene" refers to a divalent group formed by removing another hydrogen from 5- to 6-membered heteroaryl.

Alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, - NO2, -N3, -$SO_2$H, -SO3H, -OH, -$OR^{aa}$, -$ON(R^{bb})_2$, -$N(R^{bb})_2$, -$N(R^{bb})_3^+X^-$, -$N(OR^{cc})R^{bb}$, -SH, -$SR^{aa}$, -$SSR^{cc}$, -$C(=O)R^{aa}$, -$CO_2H$, -CHO, -$C(OR^{cc})_2$, -$CO_2R^{aa}$, -$OC(=O)R^{aa}$, -$OCO_2R^{aa}$, -$C(=O)N(R^{bb})_2$, -$OC(=O)N(R^{bb})_2$, -$NR^{bb}C(=O)R^{aa}$, -$NR^{bb}CO_2R^{aa}$, -$NR^{bb}C(=O)N(R^{bb})_2$, -$C(=NR^{bb})R^{aa}$, - $C(=NR^{bb})OR^{aa}$, -$OC(=NR^{bb})R^{aa}$, -$OC(=NR^{bb})OR^{aa}$, -$C(=NR^{bb})N(R^{bb})_2$, -$OC(=NR^{bb})N(R^{bb})_2$, - $NR^{bb}C(=NR^{bb})N(R^{bb})_2$, -$C(=O)NR^{bb}SO_2R^{aa}$, -$NR^{bb}SO_2R^{aa}$, -$SO_2N(R^{bb})_2$, -$SO_2R^{aa}$, -$SO_2OR^{aa}$, - $OSO_2R^{aa}$, -$S(=O)R^{aa}$, -$OS(=O)R^{aa}$, -$Si(R^{aa})_3$, -$OSi(R^{aa})_3$, -$C(=S)N(R^{bb})_2$, -$C(=O)SR^{aa}$, -$C(=S)SR^{aa}$, -$SC(=S)SR^{aa}$, -$SC(=O)SR^{aa}$, -$OC(=O)SR^{aa}$, -$SC(=O)OR^{aa}$, -$SC(=O)R^{aa}$, -$P(=O)_2R^{aa}$, -$OP(=O)_2R^{aa}$, -$P(=O)(R^{aa})_2$, -$OP(=O)(R^{aa})_2$, -$OP(=O)(OR^{cc})_2$, -$P(=O)_2N(R^{bb})_2$, -$OP(=O)_2N(R^{bb})_2$, -$P(=O)(NR^{bb})_2$, -$OP(=O)(NR^{bb})_2$, -$NR^{bb}P(=O)(OR^{cc})_2$, -$NR^{bb}P(=O)(NR^{bb})_2$, -$P(R^{cc})_2$, -$P(R^{cc})_3$, -$OP(R^{cc})$2, -$OP(R^{cc})_3$, -$B(R^{aa})_2$, -$B(OR^{cc})_2$, -$BR^{aa}(OR^{cc})$, alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 $R^{dd}$ groups;

or two geminal hydrogen on a carbon atom are replaced with =O, =S, =$NN(R^{bb})_2$, =$NNR^{bb}C(=O)R^{aa}$, =$NNR^{bb}C(=O)OR^{aa}$, =$NNR^{bb}S(=O)_2R^{aa}$, =$NR^{bb}$ or =$NOR^{cc}$ groups;

each of the $R^{aa}$ is independently selected from alkyl, haloalkyl, alkenyl, alkynyl, cycloalkyl, heterocyclyl, aryl and heteroaryl, or two of the $R^{aa}$ groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{bb}$ is independently selected from hydrogen, -OH, -$OR^{aa}$, -$N(R^{cc})_2$, -CN, - $C(=O)R^{aa}$, -$C(=O)N(R^{cc})$2, -$CO_2R^{aa}$, -$SO_2R^{aa}$, -$C(=NR^{cc})OR^{aa}$, -$C(=NR^{cc})N(R^{cc})_2$, -$SO_2N(R^{cc})_2$, - $SO_2R^{cc}$, -$SO_2OR^{cc}$, -$SOR^{aa}$, -$C(=S)N(R^{cc})$2, -$C(=O)SR^{cc}$, -$C(=S)SR^{cc}$, -$P(=O)_2R^{aa}$, -$P(=O)(R^{aa})_2$, - $P(=O)_2N(R^{cc})_2$, -$P(=O)(NR^{cc})_2$, alkyl, haloalkyl, alkenyl, alkynyl, cycloalkyl, heterocyclyl, aryl and heteroaryl, or two $R^{bb}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, cycloalkyl, heterocyclyl, aryl and heteroaryl, or two $R^{cc}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups;

each of the $R^{dd}$ is independently selected from halogen, -CN, -$NO_2$, -$N_3$, -$SO_2H$, -$SO_3H$, - OH, -$OR^{ee}$, -$ON(R^{ff})$2 , -$N(R^{ff})$2, -$N(R^{ff})_3^+X^-$, -$N(OR^{ee})R^{ff}$, -SH, -$SR^{ee}$, -$SSR^{ee}$, -$C(=O)R^{ee}$, -$CO_2H$, -$CO_2R^{ee}$, -$OC(=O)R^{ee}$, -$OCO_2R^{ee}$, -$C(=O)N(R^{ff})_2$, -$OC(=O)N(R^{ff})_2$, -$NR^{ff}C(=O)R^{ee}$, -$NR^{ff}CO_2R^{ee}$, -$NR^{ff}C(=O)N(R^{ff})_2$, -$C(=NR^{ff})OR^{ee}$, -$OC(=NR^{ff})R^{ee}$, -$OC(=NR^{ff})OR^{ee}$, -$C(=NR^{ff})N(R^{ff})_2$, - $OC(=NR^{ff})N(R^{ff})_2$, -$NR^{ff}C(=NR^{ff})N(R^{ff})_2$, -$NR^{ff}SO_2R^{ee}$, -$SO_2N(R^{ff})_2$, -$SO_2R^{ee}$, -$SO_2OR^{ee}$, - $OSO_2R^{ee}$, -$S(=O)R^{ee}$, -$Si(R^{ee})_3$, -$OSi(R^{ee})_3$, -$C(=S)N(R^{ff})_2$, -$C(=O)SR^{ee}$, -$C(=S)SR^{ee}$, -$SC(=S)SR^{ee}$, -$P(=O)_2R^{ee}$, -$P(=O)(R^{ee})_2$, -$OP(=O)(R^{ee})_2$, -$OP(=O)(OR^{ee})_2$, alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl and heteroaryl

is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, cycloalkyl, heterocyclyl, aryl and heteroaryl, or two $R^{ff}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{gg}$ groups;

each of the $R^{gg}$ is independently selected from halogen, -CN, $-NO_2$, $-N_3$, $-SO_2H$, $-SO_3H$, - OH, $-OC_{1-6}$ alkyl, $-ON(C_{1-6}$ alkyl$)_2$, $-N(C_{1-6}$ alkyl$)_2$, $-N(C_{1-6}$ alkyl$)_3{}^+X^-$, $-NH(C_{1-6}$ alkyl$)_2{}^+X^-$, - $NH_2(C_{1-6}$ alkyl$)^+X^-$, $-NH_3{}^+X^-$, $-N(OC_{1-6}$ alkyl$)(C_{1-6}$ alkyl), $-N(OH)(C_{1-6}$ alkyl), -NH(OH), -SH, - $SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), - $OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, - $NHC(=O)NH(C_{1-6}$ alkyl), $-NHC(=O)NH_2$, $-C(=NH)O(C_{1-6}$ alkyl), $-OC(=NH)(C_{1-6}$ alkyl), - $OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, - $OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, - $NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, - $C(=S)N(C_{1-6}$ alkyl$)_2$, $C(=S)NH(C_{1-6}$ alkyl), $C(=S)NH_2$, $-C(=O)S(C_{1-6}$ alkyl), $-C(=S)SC_{1-6}$ alkyl, - $SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, and $C_5$-$C_{10}$ heteroaryl; or two geminal $R^{gg}$ substituents may combine to form =O or =S; wherein $X^-$ is a counter-ion.

Exemplary substituents on nitrogen atoms include, but are not limited to, hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, - $C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, - $C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{cc}$ groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

Other Definitions

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "humam", "patient" and "subject" can be used interchangeably herein.

"Disease," "disorder," and "condition" can be used interchangeably herein.

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

## DETAILED DESCRIPTION

"Compound(s) of the present disclosure" herein refers to the following compounds of formula (I) (including sub-formulae, such as formula (II), formula (V-1), etc.), a pharmaceutically acceptable salt, enantiomer, diastereomer, solvate, hydrate or isotopic variant thereof, and mixtures thereof.

In one embodiment, the present disclosure relates to a compound of formula (I), or a pharmaceutically acceptable salt, isotopic variant, tautomer, stereoisomer, prodrug, polymorph, hydrate or solvate thereof:

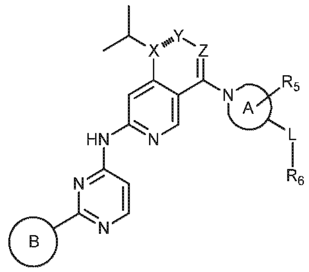

(I)

wherein:

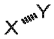 represents 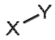 or 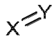; wherein when 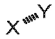 is 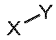, X is selected from CH and N, and Y is selected from $CH_2$, C=O and C=S; and when 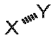 is 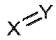, X is C, and Y is CH;

Z is selected from CH and N;

ring A is 4- to 8-membered heterocyclyl;

ring B is selected from

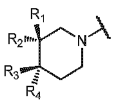 , 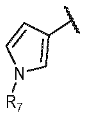 and 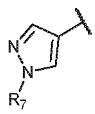 ;

L is selected from a chemical bond, -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and -S(=O)(=M)-CH(Rrr)-;

$R_1$ and $R_2$ are independently selected from H, halogen, $C_{1-6}$ alkyl and $C_{1-6}$ haloalkyl;

$R_3$ and $R_4$ are independently selected from H, $OR_a$, $C_{1-6}$ alkyl and $C_{1-6}$ haloalkyl;

$R_5$ is selected from H, halogen, $C_{1-6}$ alkyl and $C_{1-6}$ haloalkyl;

$R_6$ is selected from -$C_{0-6}$ alkylene-$OR_a$, -$C_{0-6}$ alkylene-$NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;

$R_7$ is selected from H, -$C_{1-6}$ alkylene-CN, -$C_{1-6}$ alkylene-$OR_a$, -$C_{1-6}$ alkylene-$NR_bR_c$, $C_{1-6}$ alkyl, $C_{1-6}$ haloalkyl and -S(=O)(=M)-Rs;

wherein M is O or NH;

$R_N$ is selected from H, $C_{1-6}$ alkyl and $C_{1-6}$ haloalkyl;

or $R_N$ and $R_6$ are linked to form $C_{1-6}$ alkylene, $C_{2-6}$ alkenylene or $C_{2-6}$ alkynylene;

Rs is selected from -$C_{0-6}$ alkylene-$OR_a$, -$C_{0-6}$ alkylene-$NR_bR_c$, $C_{1-6}$ alkyl, -$C_{1-6}$ alkylene-$C_{3-6}$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;

$R_a$, $R_b$ and $R_c$ are independently selected from H, $C_{1-6}$ alkyl, $C_{1-6}$ haloalkyl, $C_{3-6}$ cycloalkyl and 4- to 6-membered heterocyclyl; or $R_b$ and $R_c$ together with the nitrogen atom to which they are attached form 4- to 7-membered heterocyclyl;

each group in X, Y, Z, ring A, ring B, L, $R_1$-$R_8$, $R_N$, $R_a$, $R_b$ and $R_c$ may be substituted by one or more deuterium atoms, up to full deuteration;

provided that, when L is -CH($R_N$)-S(=O)(=M)-, and the ring where X, Y and Z are located is a benzene ring, B is

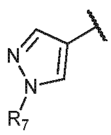

.

X, Y, Z

In one embodiment, 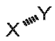 is 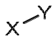, X is selected from CH and N, and Y is selected from $CH_2$, C=O and C=S, alternatively C=O; in another embodiment, 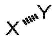 is 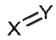, X is C, and Y is CH.

In one embodiment, Z is CH; in another embodiment, Z is N.

In a more specific embodiment, the ring where X, Y and Z are located is selected from:

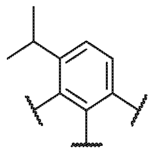, 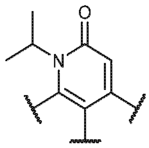 and 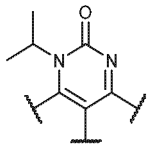.

Ring A

In one embodiment, ring A is 4- to 8-membered heterocyclyl; in another embodiment, ring A is selected from:

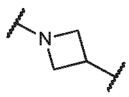, 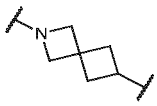 and 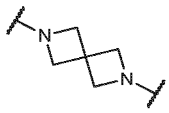.

Ring B

In one embodiment, ring B is

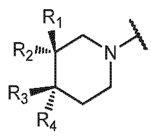;

in another embodiment, ring B is

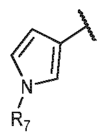;

in another embodiment, ring B is

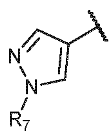.

L

In one embodiment, L is a chemical bond; in another embodiment, L is -S(=O)(=M)-; in another embodiment, L is -N($R_N$)-S(=O)(=M)-; in another embodiment, L is -S(=O)(=M)-N($R_N$)-; in another embodiment, L is -N($R_N$)-C(=O)-; in another embodiment, L is -C(=O)-N($R_N$)-; in another embodiment, L is -CH($R_N$)-C(=O)-; in another embodiment, L is -C(=O)-CH($R_N$)-; in another embodiment, L is -CH($R_N$)-S(=O)(=M)-; in another embodiment, L is -S(=O)(=M)-CH($R_N$)-.

$R_1$, $R_2$, $R_3$ and $R_4$

In one embodiment, $R_1$ and $R_2$ are independently selected from H, halogen, $C_{1-6}$ alkyl and $C_{1-6}$ haloalkyl; in another embodiment, one of $R_1$ and $R_2$ is halogen, and the other is selected from H, halogen and $C_{1-6}$ alkyl; in another embodiment, one of $R_1$ and $R_2$ is F, and the other is selected from H, F and Me; in another embodiment, one of $R_1$ and $R_2$ is F, and the other is H.

In one embodiment, one of $R_3$ and $R_4$ is $OR_a$, and the other is selected from H and $C_{1-6}$ alkyl; in another embodiment, one of $R_3$ and $R_4$ is OH or OMe, and the other is selected from H and Me.

$R_5$

In one embodiment, $R_5$ is H; in another embodiment, $R_5$ is halogen; in another embodiment, $R_5$ is $C_{1-6}$ alkyl; in another embodiment, $R_5$ is $C_{1-6}$ haloalkyl; in another embodiment, $R_5$ is selected from H and Me.

$R_6$

In one embodiment, $R_6$ is -$C_{0-6}$ alkylene-$OR_a$; in another embodiment, $R_6$ is $OR_a$; in another embodiment, $R_6$ is -$C_{0-6}$ alkylene-$NR_bR_c$; in another embodiment, $R_6$ is $NR_bR_c$; in another embodiment, $R_6$ is $C_{1-6}$ alkyl; in another embodiment, $R_6$ is $C_{1-6}$ haloalkyl; in another embodiment, $R_6$ is $C_{3-6}$ cycloalkyl; in another embodiment, $R_6$ is 4- to 6-membered heterocyclyl; in another embodiment, $R_6$ is $C_{6-10}$ aryl; in another embodiment, $R_6$ is 5- to 6-membered heteroaryl.

$R_7$

In one embodiment, $R_7$ is H; in another embodiment, $R_7$ is -$C_{1-6}$ alkylene-CN; in another embodiment, $R_7$ is -$C_{1-6}$ alkylene-$OR_a$; in another embodiment, $R_7$ is -$C_{1-6}$ alkylene-$NR_bR_c$; in another embodiment, $R_7$ is $C_{1-6}$ alkyl; in another embodiment, $R_7$ is $C_{1-6}$ haloalkyl; in another embodiment, $R_7$ is -S(=O)(=M)-Rs.

M

In one embodiment, M is O; in another embodiment, M is NH.

$R_N$

In one embodiment, $R_N$ is H; in another embodiment, $R_N$ is $C_{1-6}$ alkyl; in another embodiment, $R_N$ is $C_{1-6}$ haloalkyl; in another embodiment, $R_N$ and $R_6$ are linked to form $C_{1-6}$ alkylene; in another embodiment, $R_N$ and $R_6$ are linked to form $C_{2-6}$ alkenylene; in another embodiment, $R_N$ and $R_6$ are linked to form $C_{2-6}$ alkynylene.

$R_8$

In one embodiment, $R_8$ is -$C_{0-6}$ alkylene-$OR_a$; in another embodiment, $R_8$ is $OR_a$; in another embodiment, $R_8$ is -$C_{0-6}$ alkylene-$NR_bR_c$; in another embodiment, $R_8$ is $NR_bR_c$; in another embodiment, $R_8$ is $C_{1-6}$ alkyl; in another embodiment, $R_8$ is -$C_{1-6}$ alkylene-$C_{3-6}$ cycloalkyl; in another embodiment, $R_8$ is $C_{1-6}$ haloalkyl; in another embodiment, $R_8$ is $C_{3-6}$ cycloalkyl; in another embodiment, $R_8$ is 4- to 6-membered heterocyclyl; in another embodiment, $R_8$ is $C_{6-10}$ aryl; in another embodiment, $R_8$ is 5- to 6-membered heteroaryl.

Any technical solution in any one of the above specific embodiments, or any combination thereof, may be combined with any technical solution in other specific embodiments or any combination thereof. For example, any technical solution of X or any combination thereof may be combined with any technical solution of Y, Z, ring A, ring B, L, $R_5$, $R_6$ or any combination thereof. The present disclosure is intended to include all combination of such technical solutions, which are not exhaustively listed here to save space.

In a more specific embodiment, the present disclosure provides a compound of formula (I), or a pharmaceutically acceptable salt, isotopic variant, tautomer, stereoisomer, prodrug, polymorph, hydrate or solvate thereof:

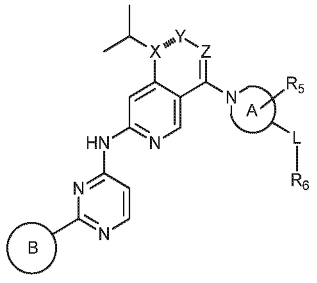

(I)

wherein:

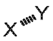 represents 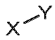 or 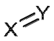; wherein when 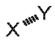 is 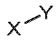, X is selected from CH and N, and Y is selected from $CH_2$, C=O and C=S; and when 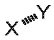 is 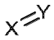, X is C, and Y is CH;
Z is selected from CH and N;
ring A is 4- to 8-membered heterocyclyl;
ring B is selected from

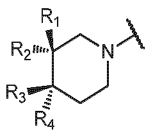 and 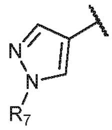 ;

L is selected from a chemical bond, -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and -S(=O)(=M)-CH($R_N$)-;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, -$C_{1-6}$ alkylene-$C_{3-6}$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl;
provided that, when L is -CH($R_N$)-S(=O)(=M)-, and the ring where X, Y and Z are located is a benzene ring, B is

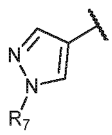 .

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein the ring where X, Y and Z are located is selected from:

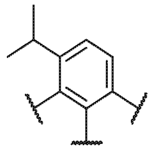, 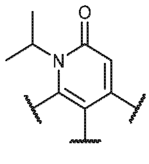 and 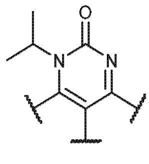.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein ring A is selected from:

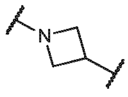, 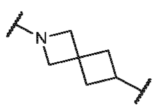 and 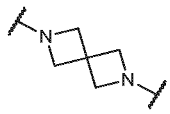.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein ring B is

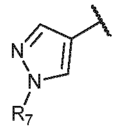.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein ring B is

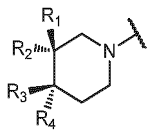.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein L is selected from -S(=O)(=M)-, -N($R_N$)-S(=O)(=M)-, -N($R_N$)-C(=O)-, -CH($R_N$)-C(=O)- and -CH($R_N$)-S(=O)(=M)-; alternatively, L is - N($R_N$)-S(=O)(=M)- or -CH($R_N$)-S(=O)(=M)-.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein one of $R_1$ and $R_2$ is halogen, and the other is selected from H, halogen and $C_{1-6}$ alkyl; alternatively, one of $R_1$ and $R_2$ is F, and the other is selected from H, F and Me; alternatively, one of $R_1$ and $R_2$ is F, and the other is H.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein one of $R_3$ and $R_4$ is $OR_a$, and the other is selected from H and $C_{1-6}$ alkyl; alternatively, one of $R_3$ and $R_4$ is OH or OMe, and the other is selected from H and Me.

In a more specific embodiment, the present disclosure provides a compound of the above formula (I), or a pharmaceutically acceptable salt, isotopic variant, tautomer, stereoisomer, prodrug, polymorph, hydrate or solvate thereof, wherein $R_5$ is selected from H and Me.

In a more specific embodiment, the present disclosure provides a compound of the above formula (I), or a pharmaceutically acceptable salt, isotopic variant, tautomer, stereoisomer, prodrug, polymorph, hydrate or solvate thereof, which has the following structure:

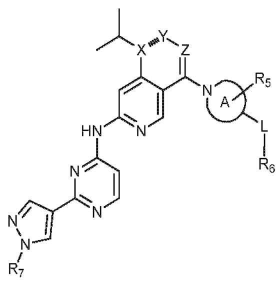
(II),
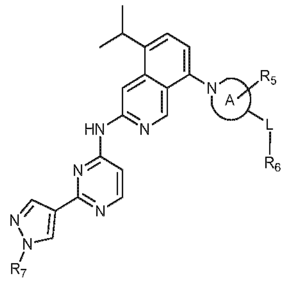
(III),
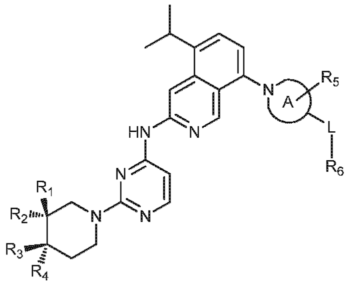
(IV),
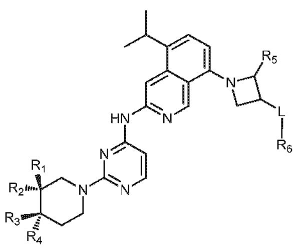
(V),
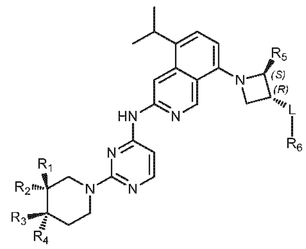
(V-1),
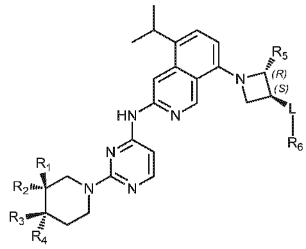
(V-2),
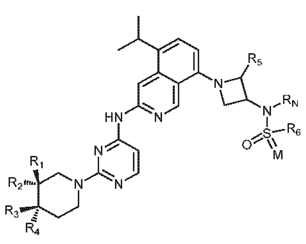
(VI),
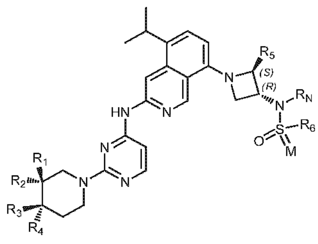
(VI-1),
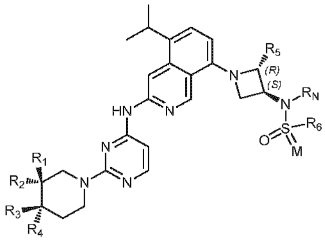
(VI-2),
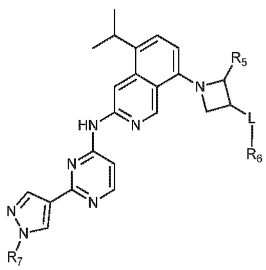
(VII),
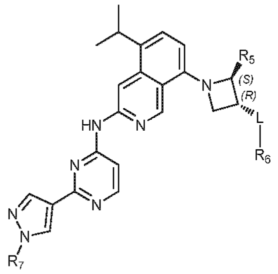
(VII-1) or
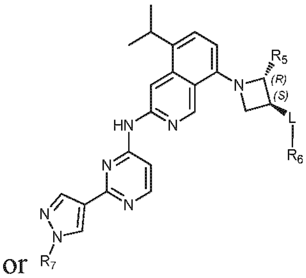
(VII-2),

wherein each group is as defined above.

In a more specific embodiment, the present disclosure provides a compound of formula (II), or a pharmaceutically acceptable salt, isotopic variant, tautomer, stereoisomer, prodrug, polymorph, hydrate or solvate thereof:

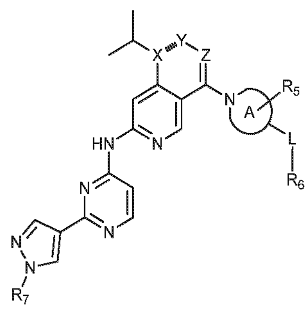
(II)

wherein:

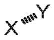 represents 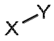 or 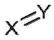; wherein when 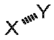 is 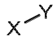, X is selected from CH and N, and Y is selected from $CH_2$, C=O and C=S; and when 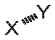 is 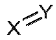, X is C, and Y is CH;

Z is selected from CH and N;

ring A is 4- to 8-membered heterocyclyl;

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and - S(=O)(=M)-CH($R_N$)-;

$R_5$ is selected from H, halogen, $C_{1-6}$ alkyl and $C_{1-6}$ haloalkyl;

$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;

$R_7$ is selected from H, $C_{1-6}$ alkyl, $C_{1-6}$ haloalkyl and -S(=O)(=M)-Rs;

wherein M is O or NH;

$R_N$ is selected from H, $C_{1-6}$ alkyl and $C_{1-6}$ haloalkyl;

or $R_N$ and $R_6$ are linked to form $C_{1-6}$ alkylene, $C_{2-6}$ alkenylene or $C_{2-6}$ alkynylene;

$R_8$ is selected from $OR_a$, $NR_bR_c$, $C_{1-6}$ alkyl, -$CH_2$-$C_{3-6}$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;

$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides a compound of formula (III), or a pharmaceutically acceptable salt, isotopic variant, tautomer, stereoisomer, prodrug, polymorph, hydrate or solvate thereof:

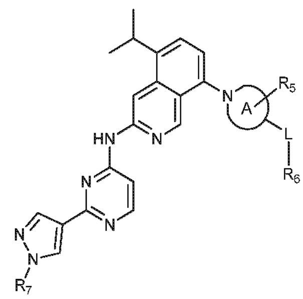

(III)

wherein:

ring A is 4- to 8-membered heterocyclyl;

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and - S(=O)(=M)-CH($R_N$)-;

$R_5$ is selected from H, halogen, $C_{1-6}$ alkyl and $C_{1-6}$ haloalkyl;

$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;

$R_7$ is selected from H, $C_{1-6}$ alkyl, $C_{1-6}$ haloalkyl and -S(=O)(=M)-Rs;

wherein M is O or NH;

$R_N$ is selected from H, $C_{1-6}$ alkyl and $C_{1-6}$ haloalkyl;

or $R_N$ and $R_6$ are linked to form $C_{1-6}$ alkylene, $C_{2-6}$ alkenylene or $C_{2-6}$ alkynylene;

$R_8$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;

$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

ring A is selected from

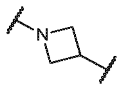, 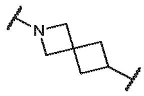 and 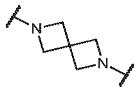;

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and - S(=O)(=M)-CH($R_N$)-;
$R_5$ is selected from H and Me;
$R_6$ is selected from Me, Et, Pr, $CH_2CF_3$, cyclopropyl, OH, $NH_2$, NHMe, $NMe_2$, furan-2-yl and pyridin-4-yl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from Me, Et, Pr, $CH_2CF_3$, cyclopropyl, OH, $NH_2$, NHMe, $NMe_2$, pyran-4-yl, furan-2-yl and pyridin-4-yl;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

ring A is 4- to 8-membered heterocyclyl;
L is selected from -S(=O)(=M)-, -CH($R_N$)-C(=O)- and -CH($R_N$)-S(=O)(=M)-;
$R_5$ is H, $C_{1-6}$ alkyl or $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl and 4- to 6-membered heterocyclyl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, $C_{1-6}$ haloalkyl, $C_{3-6}$ cycloalkyl and 4- to 6-membered heterocyclyl;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

ring A is 4- to 8-membered heterocyclyl;
L is selected from -S(=O)(=M)-, -CH($R_N$)-C(=O)- and -CH($R_N$)-S(=O)(=M)-;
$R_5$ is selected from H and $C_{1-6}$ alkyl;
$R_6$ is selected from $NR_bR_c$, $C_{1-6}$ alkyl and $C_{1-6}$ haloalkyl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and pyran-4-yl;
$R_b$ and $R_c$ are independently selected from H, $C_{1-6}$ alkyl and $C_{1-6}$ haloalkyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

ring A is selected from

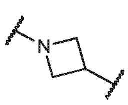 and 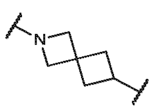;

L is selected from -S(=O)(=M)-, -CH($R_N$)-C(=O)- and -CH($R_N$)-S(=O)(=M)-;
$R_5$ is H;
$R_6$ is selected from Me and $NH_2$;
$R_7$ is selected from Hand -S(=O)(=M)-Rs;
wherein M is O;
$R_N$ is selected from H, $C_{1-6}$ alkyl and $C_{1-6}$ haloalkyl;
$R_8$ is selected from Me, Et, Pr, cyclopropyl and pyran-4-yl.

In a more specific embodiment, the present disclosure provides a compound of formula (IV), or a pharmaceutically acceptable salt, isotopic variant, tautomer, stereoisomer, prodrug, polymorph, hydrate or solvate thereof:

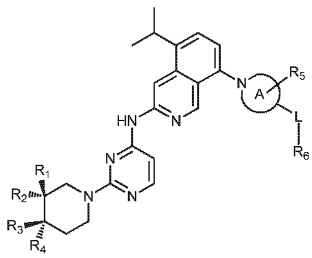

(IV)

wherein:

ring A is 4- to 8-membered heterocyclyl;
L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)- and -C(=O)-CH($R_N$)-;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

ring A is selected from

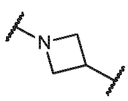, 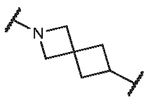 and 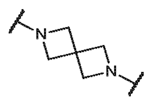;

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)- and -C(=O)-CH($R_N$)-;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;

$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides a compound of formula (V), (V-1) or (V-2), or a pharmaceutically acceptable salt, isotopic variant, tautomer, stereoisomer, prodrug, polymorph, hydrate or solvate thereof:

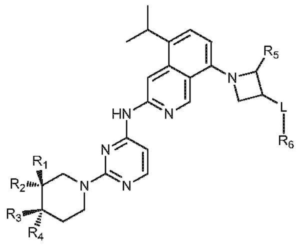 (V), 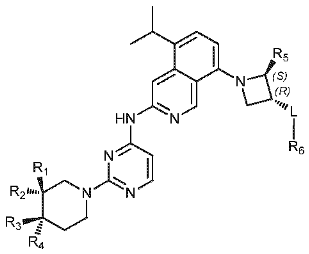 (V-1) or 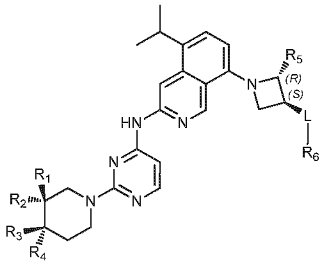 (V-2)

wherein:

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)- and -C(=O)-CH($R_N$)-;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

L is selected from -S(=O)(=M)-, -N($R_N$)-S(=O)(=M)-, -N($R_N$)-C(=O)- and -CH($R_N$)-C(=O)-;
one of $R_1$ and $R_2$ is halogen, and the other is selected from H, halogen and $C_{1-6}$ alkyl;
one of $R_3$ and $R_4$ is $OR_a$, and the other is selected from H and $C_{1-6}$ alkyl;
$R_5$ is selected from H, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides a compound of formula (VI), (VI-1) or (VI-2), or a pharmaceutically acceptable salt, isotopic variant, tautomer, stereoisomer, prodrug, polymorph, hydrate or solvate thereof:

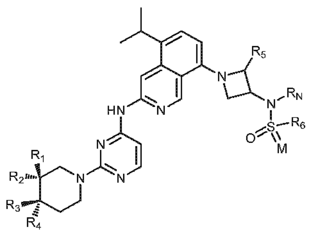 (VI), 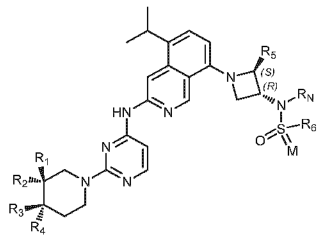 (VI-1) or 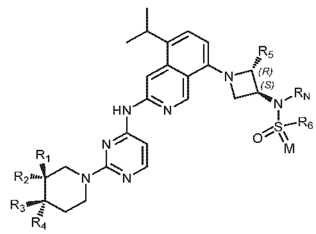 (VI-2)

wherein:

M is O or NH;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
wherein $R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

M is O or NH;
one of $R_1$ and $R_2$ is halogen, and the other is selected from H, halogen and $C_{1-6}$ alkyl;
one of $R_3$ and $R_4$ is $OR_a$, and the other is selected from H and $C_{1-6}$ alkyl;
$R_5$ is selected from H and $C_{1-6}$ alkyl;
$R_6$ is selected from $OR_a$, $NR_bR_c$, $C_{1-6}$ alkyl, $C_{1-6}$ haloalkyl, $C_{3-6}$ cycloalkyl and 5- to 6-membered heteroaryl;
$R_N$ is selected from H and $C_{1-6}$ alkyl;
or $R_N$ and $R_6$ are linked to form $C_{1-6}$ alkylene;
wherein $R_a$, $R_b$ and $R_c$ are independently selected from H, $C_{1-6}$ alkyl and $C_{1-6}$ haloalkyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

M is O;
one of $R_1$ and $R_2$ is F, and the other is selected from H, F and Me;
one of $R_3$ and $R_4$ is OH or OMe, and the other is selected from H and Me;
$R_5$ is selected from H and Me;
$R_6$ is selected from Me, Et, Pr, $CH_2CF_3$, cyclopropyl, $NMe_2$, furan-2-yl and pyridin-4-yl;
$R_N$ is selected from H and Me;
or $R_N$ and $R_6$ are linked to form $C_{1-4}$ alkylene.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

M is O or NH;
one of $R_1$ and $R_2$ is selected from halogen, $C_{1-6}$ alkyl and $C_{1-6}$ haloalkyl, and the other is H;
one of $R_3$ and $R_4$ is selected from $OR_a$, $C_{1-6}$ alkyl and $C_{1-6}$ haloalkyl, and the other is H;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_N$ is selected from H, $C_{1-6}$ alkyl and $C_{1-6}$ haloalkyl;

or $R_N$ and $R_6$ are linked to form $C_{1-6}$ alkylene, $C_{2-6}$ alkenylene or $C_{2-6}$ alkynylene;
wherein $R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

M is O or NH;
one of $R_1$ and $R_2$ is selected from halogen and $C_{1-6}$ alkyl, and the other is H;
one of $R_3$ and $R_4$ is $OR_a$, and the other is H;
$R_5$ is selected from H, $C_{1-6}$ alkyl and $C_{1-6}$ haloalkyl;
$R_6$ is selected from $C_{1-6}$ alkyl, $C_{1-6}$ haloalkyl, $C_{3-6}$ cycloalkyl and 4- to 6-membered heterocyclyl;
$R_N$ is selected from H, $C_{1-6}$ alkyl and $C_{1-6}$ haloalkyl;
wherein $R_a$ is selected from H, $C_{1-6}$ alkyl and $C_{1-6}$ haloalkyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:

M is O;
one of $R_1$ and $R_2$ is halogen (alternatively F), and the other is H;
one of $R_3$ and $R_4$ is $OR_a$, and the other is H;
$R_5$ is selected from H and $C_{1-4}$ alkyl;
$R_6$ is selected from $C_{1-4}$ alkyl and $C_{1-4}$ haloalkyl;
$R_N$ is selected from H, $C_{1-4}$ alkyl and $C_{1-4}$ haloalkyl; alternatively, $C_{1-4}$ alkyl;
wherein $R_a$ is selected from H and $C_{1-4}$ alkyl.

In a more specific embodiment, the present disclosure provides a compound of formula (VII), (VII-1) or (VII-2), or a pharmaceutically acceptable salt, isotopic variant, tautomer, stereoisomer, prodrug, polymorph, hydrate or solvate thereof:

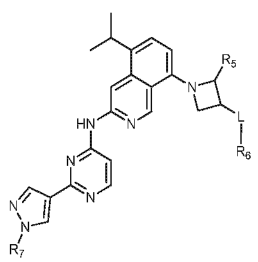 (VII), 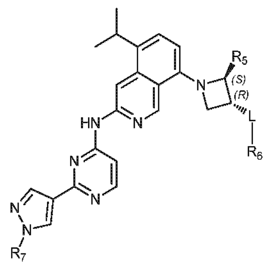 (VII-1) or 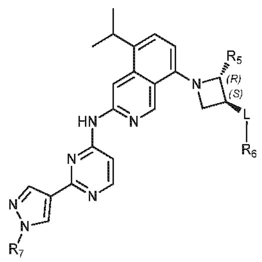 (VII-2)

wherein:

L is selected from -S(=O)(=M)-, -N($R_N$)-S(=O)(=M)-, -S(=O)(=M)-N($R_N$)-, -N($R_N$)-C(=O)-, -C(=O)-N($R_N$)-, -CH($R_N$)-C(=O)-, -C(=O)-CH($R_N$)-, -CH($R_N$)-S(=O)(=M)- and-S(=O)(=M)-CH($R_N$)-;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:
wherein:

L is selected from $-N(R_N)-S(=O)(=M)-$ and $-CH(R_N)-S(=O)(=M)-$;
$R_5$ is selected from $C_{1-6}$ alkyl and $C_{1-6}$ haloalkyl;
$R_6$ is selected from $C_{1-6}$ alkyl, $C_{1-6}$ haloalkyl, $C_{3-6}$ cycloalkyl and 4- to 6-membered heterocyclyl;
$R_7$ is -S(=O)(=M)-Rs;
wherein M is O or NH;
$R_N$ is selected from H, $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and 4- to 6-membered heterocyclyl, which are optionally substituted with one or more $R_a$;
$R_a$ is selected from H, $C_{1-6}$ alkyl and $C_{1-6}$ haloalkyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:
wherein:

L is selected from $-N(R_N)-S(=O)(=M)-$ and $-CH_2-S(=O)(=M)-$;
$R_5$ is selected from $C_{1-6}$ alkyl and $C_{1-6}$ haloalkyl;
$R_6$ is selected from $C_{1-6}$ alkyl and $C_{1-6}$ haloalkyl;
$R_7$ is -S(=O)(=M)-Rs;
wherein M is O or NH;
$R_N$ is selected from $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and 4- to 6-membered heterocyclyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:
wherein:

L is selected from $-N(R_N)-S(=O)_2-$ and $-CH_2-S(=O)_2-$;
$R_5$ is selected from $C_{1-6}$ alkyl and $C_{1-6}$ haloalkyl;
$R_6$ is selected from $C_{1-6}$ alkyl and $C_{1-6}$ haloalkyl;
$R_7$ is $-S(=O)_2-R_8$;
wherein $R_N$ is selected from $C_{1-6}$ alkyl and $C_{1-6}$ haloalkyl;
$R_8$ is selected from $C_{1-6}$ alkyl, $C_{1-6}$ haloalkyl and $C_{3-6}$ cycloalkyl.

In a more specific embodiment, the present disclosure provides the above-mentioned compound, or a pharmaceutically acceptable salt, isotopic variant, tautomer, stereoisomer, prodrug, polymorph, hydrate or solvate thereof, wherein:
wherein:

L is selected from $-N(R_N)-S(=O)_2-$ and $-CH_2-S(=O)_2-$;
$R_5$ is $C_{1-4}$ alkyl;
$R_6$ is $C_{1-4}$ alkyl;
$R_7$ is $-S(=O)_2-R_8$;
wherein $R_N$ is $C_{1-4}$ alkyl;
$R_8$ is selected from $C_{1-4}$ alkyl and $C_{3-6}$ cycloalkyl.

In a more specific embodiment, the present disclosure provides a compound, or a tautomer, stereoisomer, prodrug, crystalline form, pharmaceutically acceptable salt, hydrate or solvate thereof, wherein the compound is selected from:

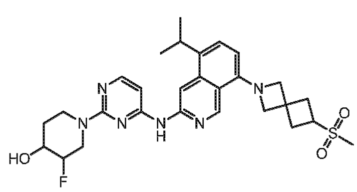,
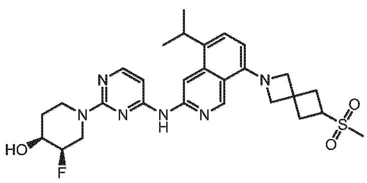,
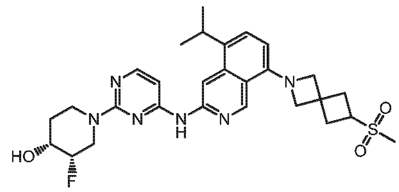,

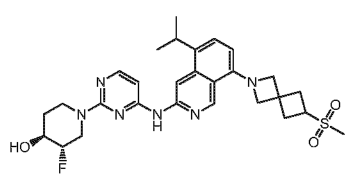,
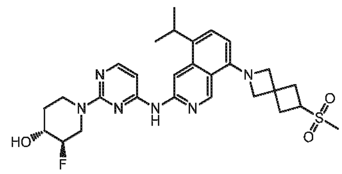,
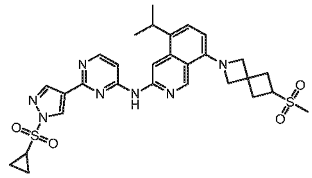,

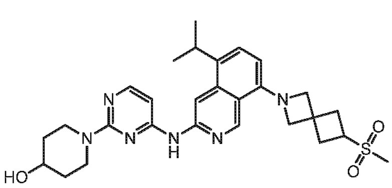,
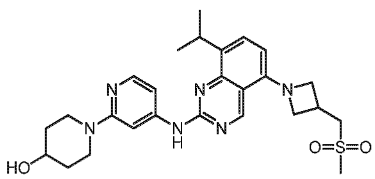,
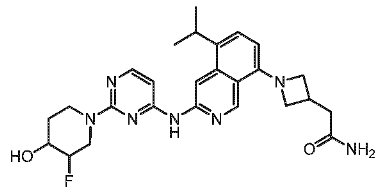,

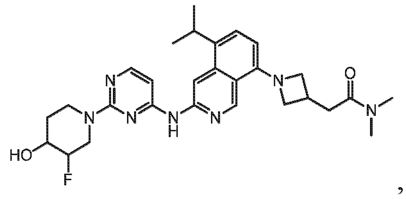,
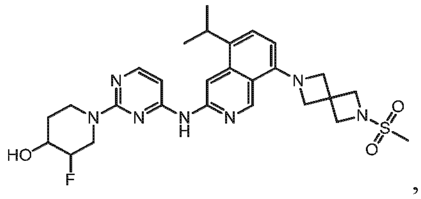,
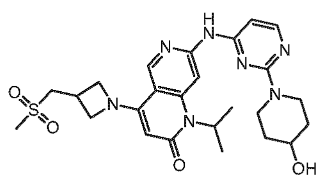,

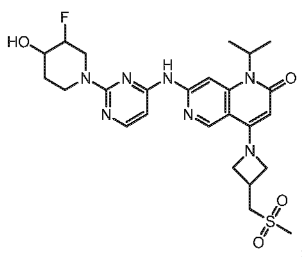,
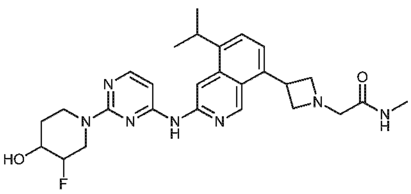,
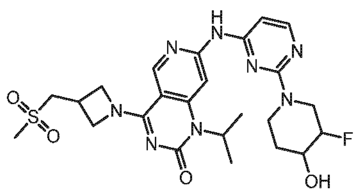,

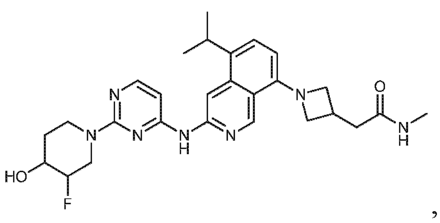,
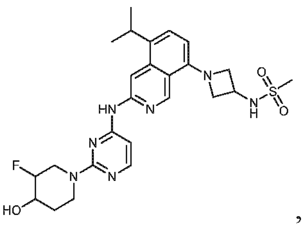,
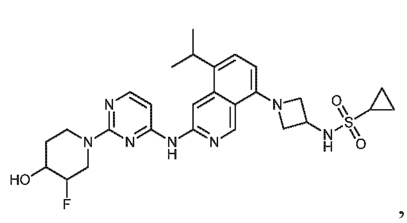,

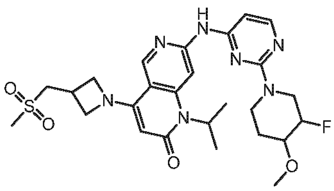, 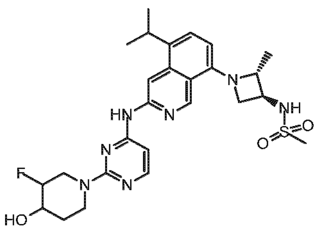, 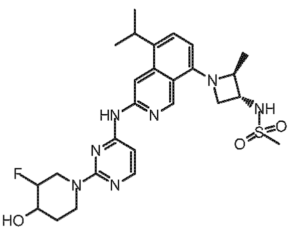,

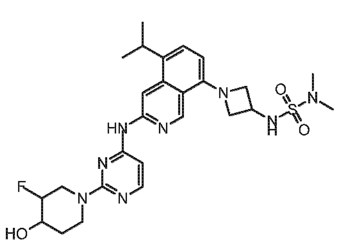, 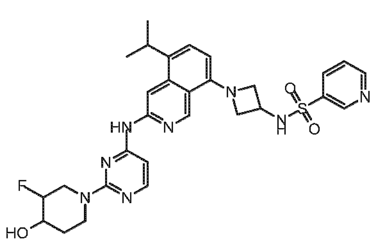, 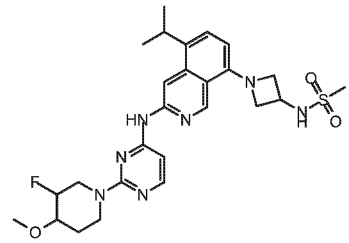,

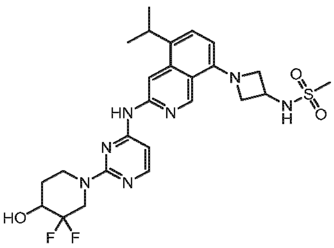, 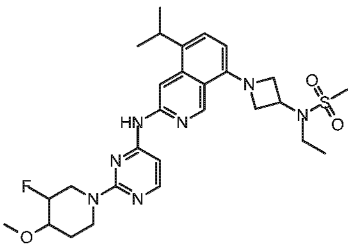, 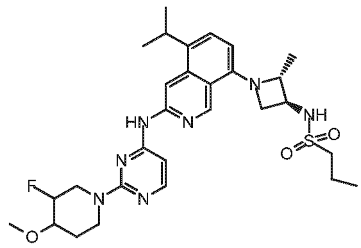,

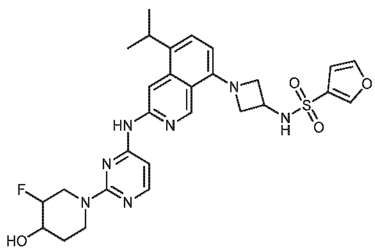, 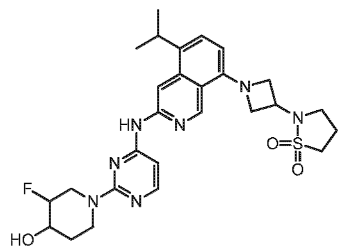, 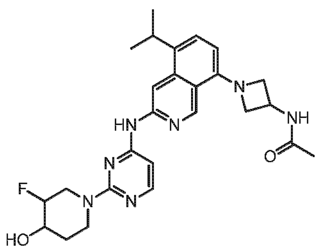,

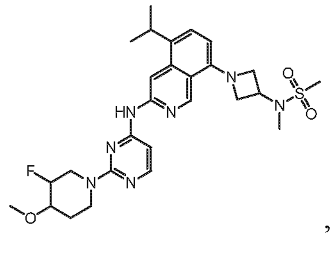, 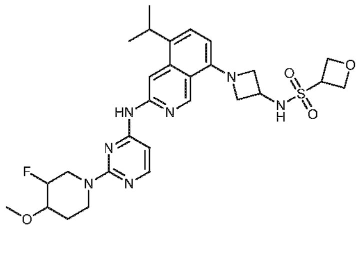, 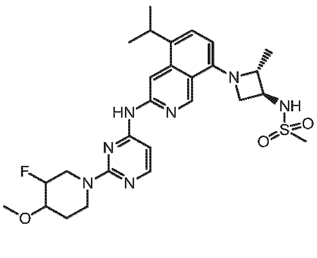,

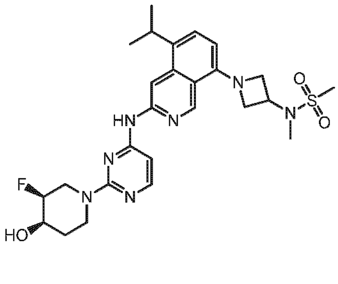, 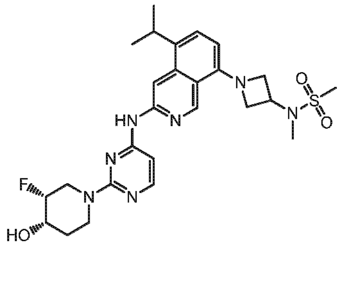, 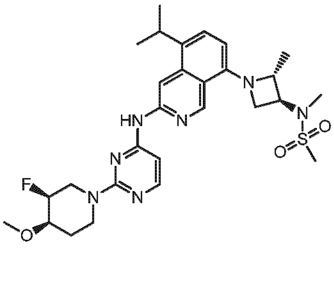,

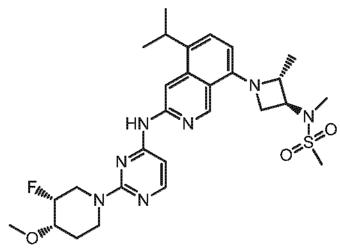,
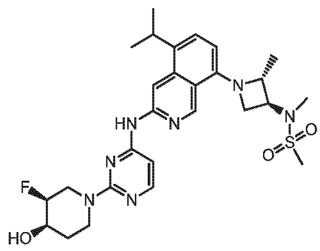,
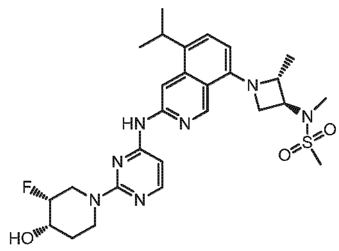,
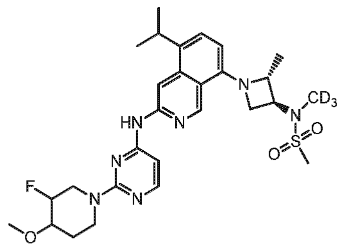,
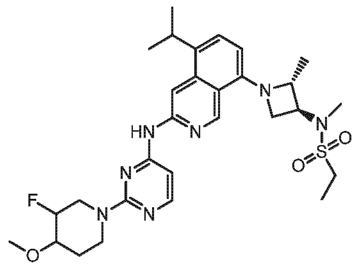,
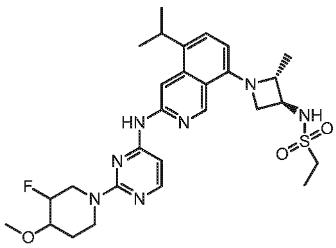,
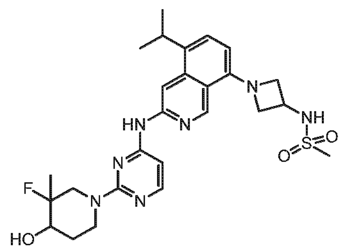,
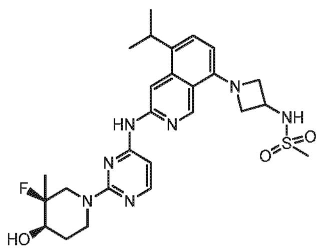,
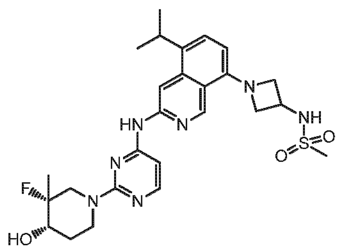,
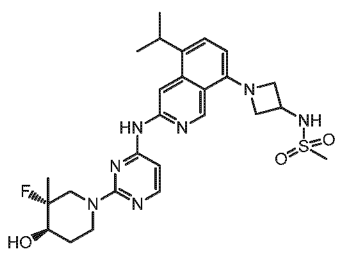,
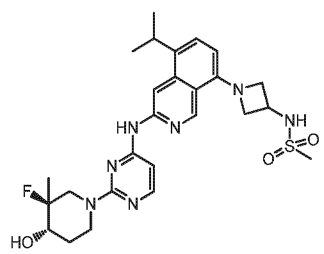,
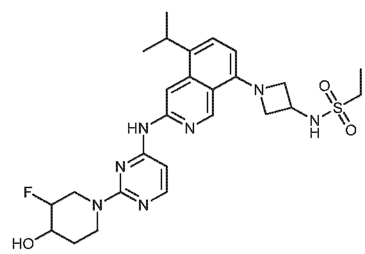,
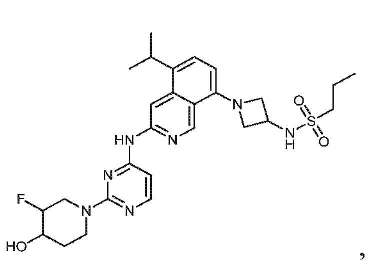,
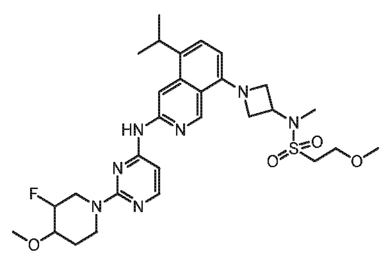,
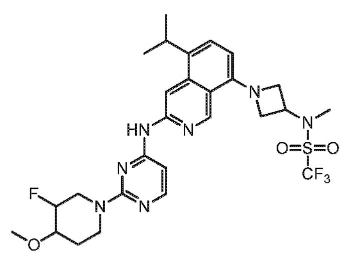,

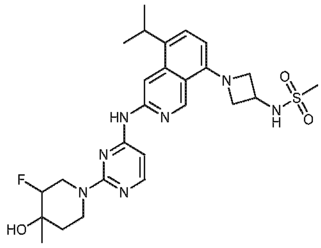,
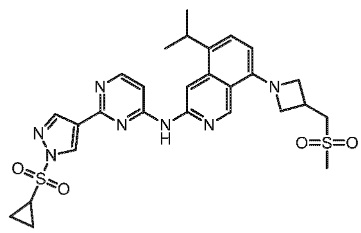,
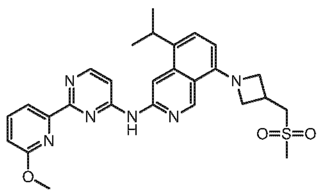,

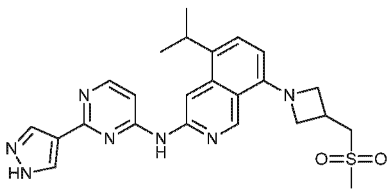,
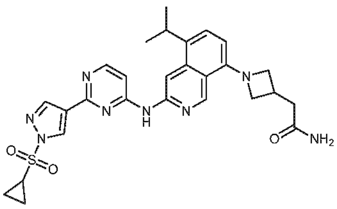,
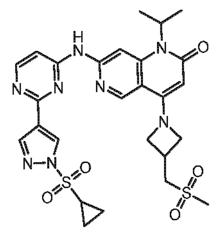,

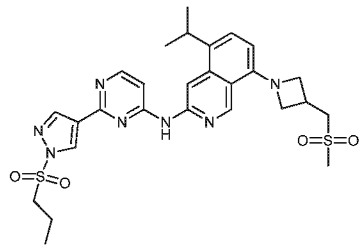,
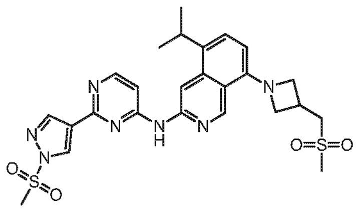,
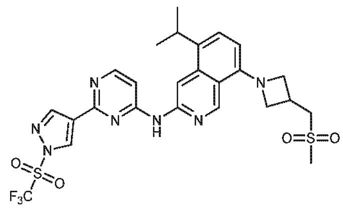,

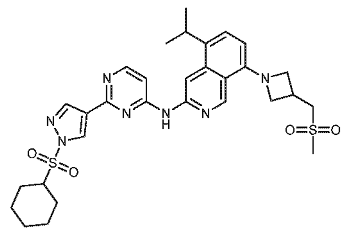,
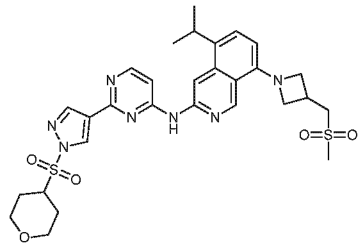,
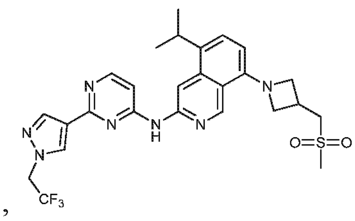,

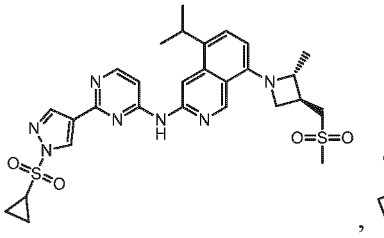,
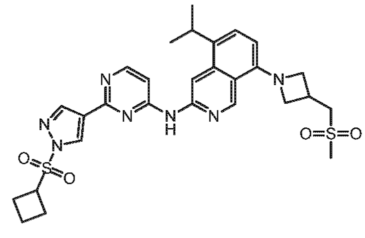,
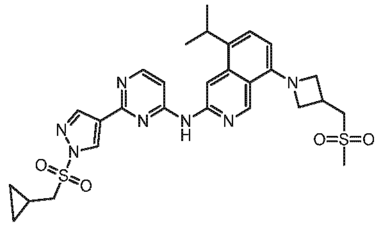,

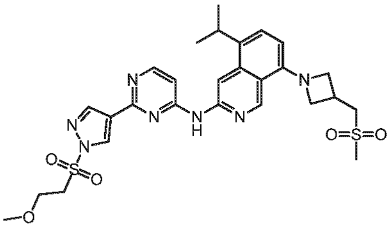,
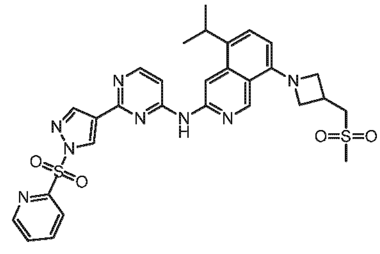,
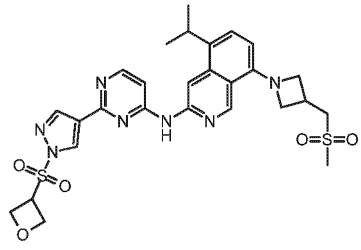,

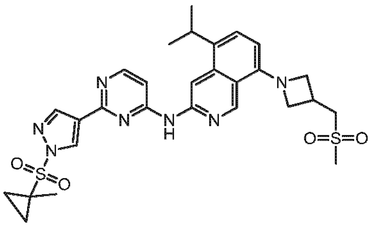, 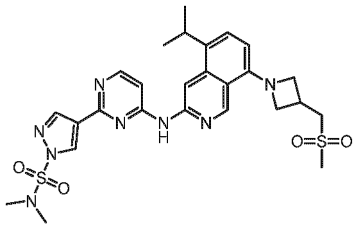, 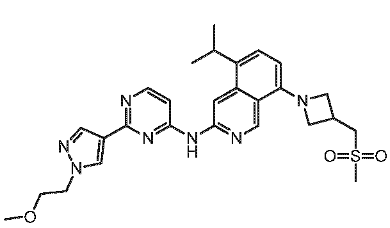,

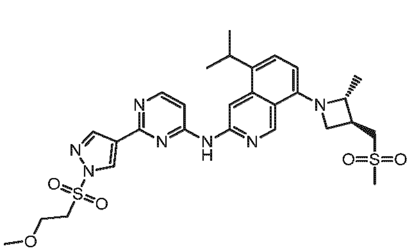, 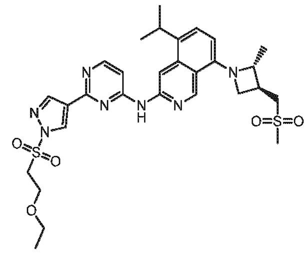, 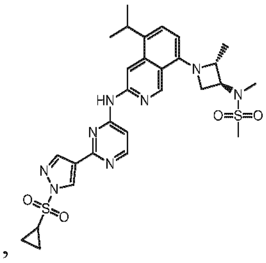,

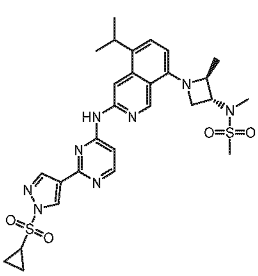, 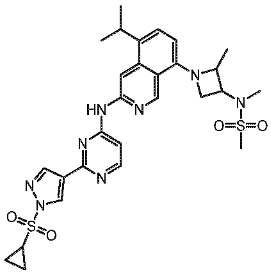, 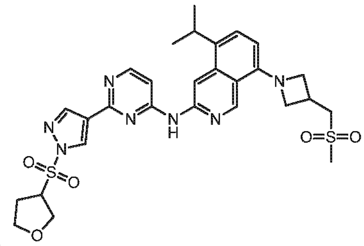,

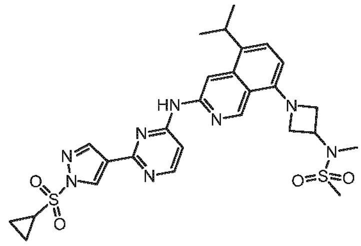, 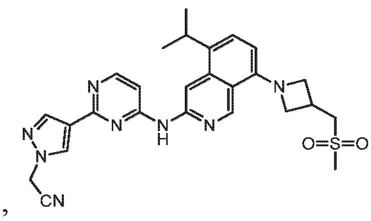, 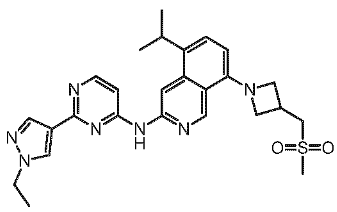,

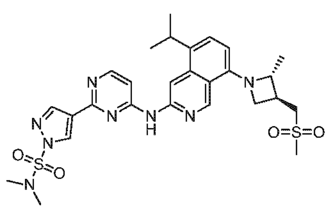, 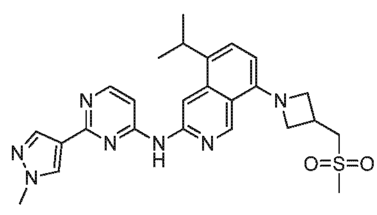, 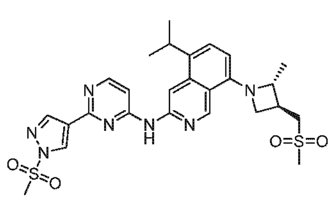,

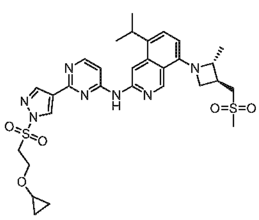, 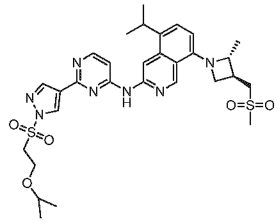, 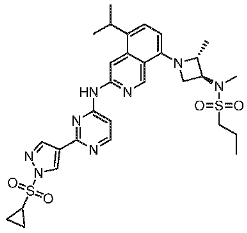, 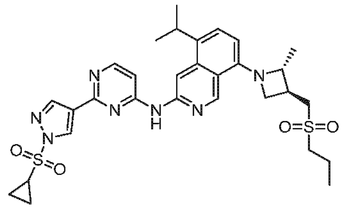

and

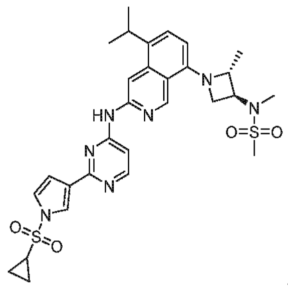

.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high-pressure liquid chromatography (HPLC) and the formation and crystallization of chiral salts; or alternative isomers can be prepared by asymmetric synthesis.

The compounds of the present disclosure may also exist as tautomers. For compounds that exist in different tautomeric forms, a compound is not limited to any specific tautomer, but is intended to encompass all tautomeric forms.

It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotope variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are yet alternative, because they are easy to prepare and detect. Furthermore, replaced

by heavier isotopes, such as deuterium, which is $^2H$, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects *in vivo* by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Pharmaceutical compositions and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the compound of the present disclosure. In certain embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable excipient for use in the present disclosure refers to a nontoxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an

extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g.*, within the therapeutic window over the extended period of time.

The pharmaceutical compostions of the present disclosure may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g.*, through an IV drip) allows a much faster delivery which quickly raises the concentration of the active ingredient in the blood to an effective level. In other embodiments, the pharmaceutical composition may be administered as a continuous infusion, *e.g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's

Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ- cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, *e.g.*, for example, sulfobutyl ether β-cyclodextrin, also known as Captisol. See, *e.g.,* U.S. 5,376,645. In certain embodiments, the formulation comprises hexapropyl-β-cyclodextrin (*e.g.,* 10-50% in water).

## Examples

The reagents used in the present disclosure are commercial reagents purchased directly or synthesized by common methods well known in the art.

The specific reaction routes or steps exemplified below are used in the present disclosure:

### Example 1

### Preparation of key intermediates

Synthesis of intermediate **a1**

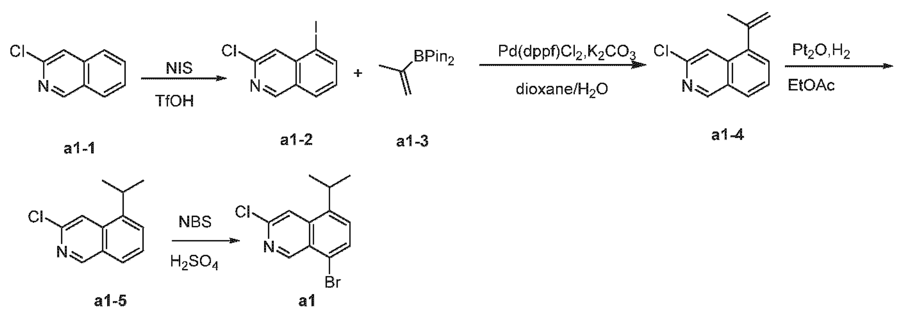


a1-1 a1-2 a1-3 a1-4 a1-5 a1

Step 1: 3-Chloroisoquinoline **a1-1** (3.5 g, 21.4 mmol) was dissolved in 50 mL of trifluoromethanesulfonic acid at -10°C under nitrogen protection. N-iodosuccinimide NIS (7.21 g, 32.1 mmol) was added in batches and the mixture was stirred for 4 hours. The reaction was completed by TLC monitoring. The reaction solution was quenched by slowly adding 100 mL of ammonia water, extracted with ethyl acetate, washed with saturated brine, dried over anhydrous sodium sulfate, and filtered. The filtrate was concentrated. The residue was separated by column chromatography (PE/EA=100/1) to obtain a white solid **a1-2** (4.1 g), yield: 60%, LCMS: ESI-MS (m/z): 289.8 $[M + H]^+$.

1H NMR (400 MHz, $CDCl_3$) $\delta$ 8.95 (s, 1H), 8.26 (dd, *J* = 7.4, 1.1 Hz, 1H), 7.98 - 7.94 (m, 1H), 7.93 (d, *J* = 0.7 Hz, 1H), 7.33 (dd, *J* = 8.2, 7.4 Hz, 1H).

Step 2: Under nitrogen protection, the intermediate **a1-2** (4.1 g, 14.2 mmol) from the previous step and $K_2CO_3$ (5.87 g, 42.3 mmol) were mixed in 110 mL of a mixture of 1,4-dioxane and water (v/v=10/1), and Pd(dppf)$Cl_2$ (1.04 g, 1.42 mmol) was added. The mixture was heated to 90°C. Isopropenylboronic acid pinacol ester **a1-3** (3.6 g, 21.2 mmol) was added to the reaction solution, and the reaction solution was reacted at 60°C for another 3 hours. The reaction was stopped. The reaction solution was filtered, and the filtrate was concentrated. 50 mL of water was added to the system, and the mixture was extracted with ethyl acetate, dried over anhydrous sodium sulfate, and filtered. The filtrate was concentrated, and separated by flash column chromatography to obtain a yellow solid **a1-4** (1.9 g), yield: 53%, LC-MS: $M+H^+$= 204.0.

1H NMR (400 MHz, DMSO-*d6*) $\delta$ 9.21 (s, 1H), 8.09 (dd, *J* = 6.2, 3.1 Hz, 1H), 7.85 (s, 1H), 7.68 - 7.65 (m, 2H), 5.49 (t, *J* = 1.6 Hz, 1H), 5.03 (s, 1H), 2.14 (s, 3H).

Step 3: 3-Chloro-5-(prop-1-en-2-yl)isoquinoline **a1-4** (3.5 g, 17.2 mmol) from the previous step was dissolved in 50 mL of ethyl acetate, and platinum dioxide (0.78 g, 3.4 mmol) was added. The mixture was reacted at room temperature for 6 hours under hydrogen atmosphere. The reaction solution was filtered. The filtrate was concentrated, and separated by column chromatography to obtain an oily intermediate **a1-5** (2.1 g), yield: 54%, LC-MS: ESI-MS (m/z): 206.1 $[M + H]^+$.

1H NMR (400 MHz, DMSO-$d_6$) $\delta$ 9.21 (s, 1H), 8.12 (s, 1H), 8.03 (d, $J$ = 8.1 Hz, 1H), 7.76 (d, $J$ = 6.7 Hz, 1H), 7.69 (d, $J$ = 7.9 Hz, 1H), 3.76 - 3.65 (m, 1H), 1.32 (d, $J$ = 6.8 Hz, 6H).

Step 4: The intermediate **a1-5** (1.8 g, 8.75 mmol) from the previous step was dissolved in 24 mL of concentrated sulfuric acid at -10°C under nitrogen protection. N-bromosuccinimide NBS (2.02 g, 11.4 mmol) was added in batches and the mixture was stirred for 8 hours. The reaction was completed by LC-MS monitoring. 200 mL of ice water was slowly added to the reaction solution to quench the reaction, and the mixture was adjusted to pH of about 8 with ammonia water. The mixture was extracted with ethyl acetate, washed with saturated brine, dried over anhydrous sodium sulfate, and filtered. The filtrate was concentrated. The residue was separated by column chromatography (PE/EA=50/1) to obtain a white solid **a1** (1.0 g), yield: 36%, LCMS: ESI-MS (m/z): 283.9 [M + H]$^+$.

1H NMR (400 MHz, DMSO-$d_6$) $\delta$ 9.34 (s, 1H), 8.22 (s, 1H), 7.99 (d, $J$ = 7.9 Hz, 1H), 7.66 (d, $J$ = 7.9 Hz, 1H), 3.72 (dt, $J$ = 13.7, 6.8 Hz, 1H), 1.31 (d, $J$ = 6.8 Hz, 6H).

Synthesis of intermediate **a2**

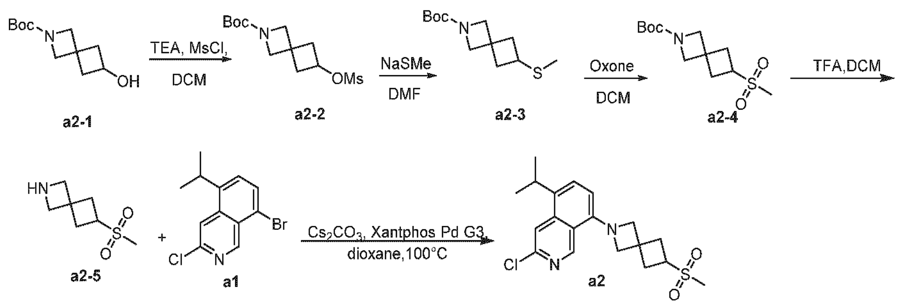


Step 1: Tert-butyl 6-hydroxy-2-azaspiro[3.3]heptane-2-carboxylate **a2-1** (4.8 g, 22 mmol) and triethylamine (4.53 g, 44 mmol) were dissolved in 50 mL of dichloromethane in an ice bath and under nitrogen protection, and methylsulfonyl chloride (3.08 g, 26 mmol) was added dropwise. After the addition was completed, the mixture was stirred for another 2 hours. 10 mL of aqueous saturated sodium bicarbonate solution was added to the reaction solution to quench the reaction, and the mixture was extracted with dichloromethane. The combined organic layer was dried over anhydrous sodium sulfate and concentrated to obtain a crude compound **a2-2** (6.24 g), yield: 86%.

Step 2: Under nitrogen protection, the intermediate **a2-2** (6.24 g, 21 mmol) from the previous step and sodium thiomethoxide (20% aqueous solution) (15.0 g, 42 mmol) were dissolved in 50 mL of DMF, and the solution was heated to 60°C and stirred for 12 hours. The reaction was completed by LC-MS monitoring. 200 mL of water was added to the system, and the mixture was extracted with ethyl acetate, and washed with saturated brine. The combined organic layer was dried over anhydrous sodium sulfate, and filtered. The filtrate was concentrated to obtain a colorless oil **a2-3** (3.98 g), yield: 69%.

Step 3: The intermediate **a2-3** (3.98 g, 16 mmol) from the previous step was dissolved in 200 mL of dichloromethane, and the oxidant Oxone (50.1 g, 81 mmol) was added. The mixture was reacted at room temperature for 48 hours. The reaction was stopped, and the mixture was filtered. The filtrate was concentrated to obtain compound **a2-4** (4.18 g), yield: 83%.

1H NMR (400 MHz, $CDCl_3$) $\delta$ 3.95 (d, $J$ = 5.7 Hz, 4H), 3.72 - 3.52 (m, 1H), 2.80 (s, 3H), 2.76 - 2.65 (m, 2H), 2.59 - 2.49 (m, 2H), 1.43 (s, 9H).

Step 4: Compound **a2-4** (3.72 g, 13 mmol) was dissolved in 40 mL of dichloromethane, and trifluoroacetic acid (4.62 g, 41 mmol) was added dropwise. The mixture was stirred at room temperature for 2 hours. The solvent was removed by evaporation under reduced pressure to obtain crude product **a2-5** (1.95 g). LCMS: ESI-MS (m/z): 176.0 [M + H]$^+$.

Step 5: Under nitrogen protection, the intermediate **a2-5** (1.6 g, 5.5 mmol) from the previous step and $Cs_2CO_3$ (5.38 g, 16.5 mmol) were mixed in 25 mL of 1,4-dioxane, and Xantphos Pd G3 (520 mg, 0.5 mmol) was added. The mixture was heated to 100°C. The intermediate **a1** (1.57 g, 5.5 mmol) was added to the reaction solution, and the reaction solution was reacted at 100°C for another 3 hours. The reaction was stopped, and the mixture was filtered. The filtrate was concentrated, and separated by flash column chromatography to obtain a yellow solid a2 (820 mg), yield: 35%, LCMS: ESI-MS (m/z): 379.1 [M + H]$^+$.

Synthesis of intermediates **a3, a5, a9**

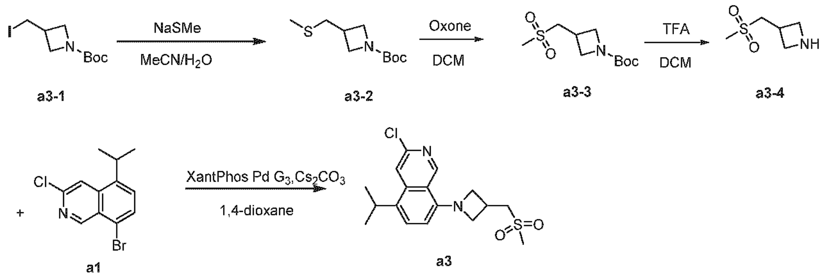


a3-1 a3-2 a3-3 a3-4

+ a1 a3

Step 1: Under nitrogen protection, tert-butyl [3-(iodomethyl)azetidin-1-yl]carboxylate **a3-1** (6.0 g, 16 mmol) and sodium thiomethoxide (20% aqueous solution) (6.0 g, 16 mmol) were dissolved in 50 mL of a mixture of acetonitrile and water (v/v=3/1). The mixture was heated to 60°C and stirred for 12 hours. The reaction was completed by LC-MS monitoring. 100 mL of water was added to the reaction solution, and the mixture was extracted with ethyl acetate, washed with saturated brine, dried over anhydrous sodium sulfate, and filtered. The filtrate was concentrated to obtain a yellow oil **a3-2** (2.43 g), yield: 81%, LC-MS: $[M+H]^+$ = 162.0.

Step 2: Under nitrogen protection, the oil **a3-2** (2.43 g, 10 mmol) from the previous step was dissolved in 50 mL of dichloromethane, and the oxidant Oxone (34.4 g, 56 mmol) was added. The mixture was reacted at room temperature for 48 hours. The reaction was completed by LC-MS monitoring. The reaction solution was filtered and the filtrate was concentrated to obtain a white solid **a3-3** (2.99 g), yield: 95%, LC-MS: $[M+H]^+$ = 250.0.

Step 3: The intermediate **a3-3** (2.99 g, 12 mmol) from the previous step was dissolved in 20 mL of dichloromethane, and 5 mL of trifluoroacetic acid was added. The mixture was stirred at room temperature for 2 hours. The reaction solution was concentrated under reduced pressure to obtain a crude product **a3-4**, LC-MS: $[M+H]^+$ = 150.0.

Step 4: Under nitrogen protection, the crude product **a3-4** (1.39 g, 5.27 mmol) from the previous step, intermediate **a1** (500 mg, 1.76 mmol) and $Cs_2CO_3$ (2.86 g, 8.79 mmol) were mixed in 10 mL of 1,4-dioxane, and XantPhos Pd G3 (167 mg, 0.18 mmol) was added. The mixture was heated to 100°C and reacted for 3 hours. The reaction was stopped, and filtered. The filtrate was concentrated, and separated by flash column chromatography to obtain a green solid **a3** (410 mg), yield: 60%, LC-MS: $M+H^+$= 353.0.

1H NMR (400 MHz, $CDCl_3$) $\delta$ 9.11 (d, $J$ = 0.5 Hz, 1H), 7.84 (s, 1H), 7.46 (d, $J$ = 8.0 Hz, 1H), 6.52 (d, $J$ = 8.0 Hz, 1H), 4.45 (t, $J$ = 7.5 Hz, 2H), 4.02 (dd, $J$ = 7.6, 5.3 Hz, 2H), 3.50 - 3.40 (m, 4H), 2.98 (s, 3H), 1.33 (d, $J$ = 6.8 Hz, 6H).

Referring to the synthetic route of intermediate **a3,** the following intermediates were synthesized using a similar skeleton structure.

| Replacement of structure | Target intermediate | LC-MS $[M+H]^+$ |
|---|---|---|
| Replace **a3-4** with 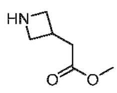 a5-1 | 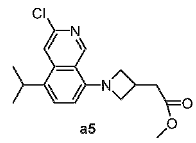 a5 | 333 |
| Replace **a3-4** with 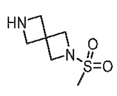 a9-1 | 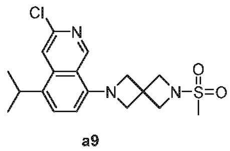 a9 | 380 |

Synthesis of intermediate **a4**

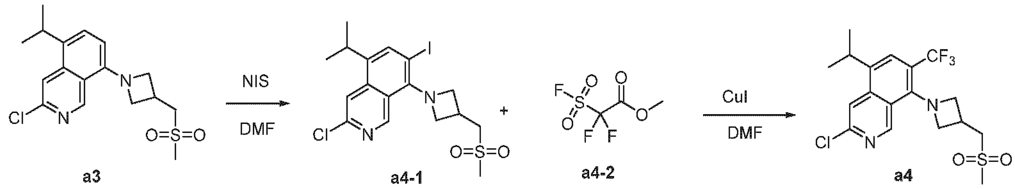


a3 a4-1 a4-2 a4

Step 1: The intermediate **a3** (200 mg, 0.57 mmol) was dissolved in 20 mL of DMF, and N-iodosuccinimide NIS (380 mg, 1.7 mmol) was added. The mixture was reacted at room temperature for 2 hours. The reaction was completed by LC-MS monitoring. 100 mL of water was added to the reaction solution. The mixture was extracted with ethyl acetate, washed with saturated sodium thiosulfate solution, dried with anhydrous sodium sulfate, and filtered. The filtrate was concentrated to obtain a yellow solid **a4-1** (200 mg), yield: 59%, LC-MS: $M+H^+$= 478.9.

Step 2: Under nitrogen protection, the intermediate **a4-1** (20 mg, 0.042 mmol), methyl 2,2-difluoro-2-(fluorosulfonyl)acetate **a4-2** (40 mg, 0.21 mmol) and CuI (24 mg, 0.12 mmol) were mixed in 5 mL of DMF. The mixture was heated to 90°C and reacted for 1 hour. The reaction was completed by LC-MS monitoring. 50 mL of water was added to the reaction solution. The mixture was extracted with ethyl acetate, dried over anhydrous sodium sulfate, and filtered. The filtrate was concentrated, and separated by column chromatography (DCM/MeOH=25/1) to obtain a yellow solid **a4** (15 mg), yield: 77%, LC-MS: $M+H^+$= 421.0.

Synthesis of intermediate **a6**

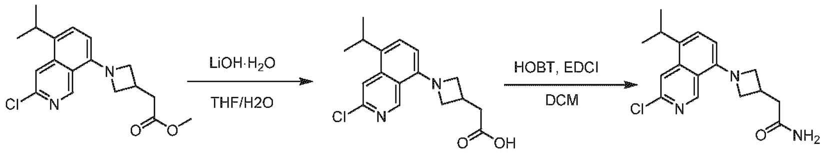


a5 a6-1 a6

Step 1: Methyl 2-(1-(3-chloro-5-isopropylisoquinolin-8-yl)azetidin-3-yl)acetate **a5** (120 mg, 0.36 mmol) was dissolved in 6 mL of a mixture of tetrahydrofuran and water (v/v=5/1), and lithium hydroxide monohydrate (23 mg, 0.54 mmol) was added. The mixture was stirred at room temperature for 12 hours. The reaction was stopped, and acetic acid was slowly added to the reaction solution to adjust the pH value to about 6. The mixture was extracted with ethyl acetate. The organic phases were combined, dried over anhydrous sodium sulfate, and filtered. The filtrate was concentrated under reduced pressure to obtain a crude intermediate **a6-1** (100 mg), yield: 18%, LC-MS:ESI-MS (m/z): 319.1 $[M + H]^+$.

Step 2: The crude product **a6-1** (50 mg, 0.16 mmol) from the previous step was dissolved in 3 mL of dichloromethane. HOBT (6 mg, 0.047 mmol), EDCI (36 mg, 0.19 mmol) and DIPEA (40 mg, 0.31 mmol) were added. The mixture was stirred at room temperature for 1 hour. 0.2 mL of ammonia water was added to the reaction solution, and the mixture was reacted at room temperature for another 2 hours. The reaction was stopped, and 20 mL of water was added to the reaction solution. The mixture was extracted with ethyl acetate. The organic phases were combined, dried over anhydrous sodium sulfate, filtered, and separated by thin layer chromatography to obtain intermediate **a6** (20 mg), yield: 36%, LC-MS:ESI-MS (m/z): 318.1 $[M + H]^+$.

Synthesis of intermediate **a7**

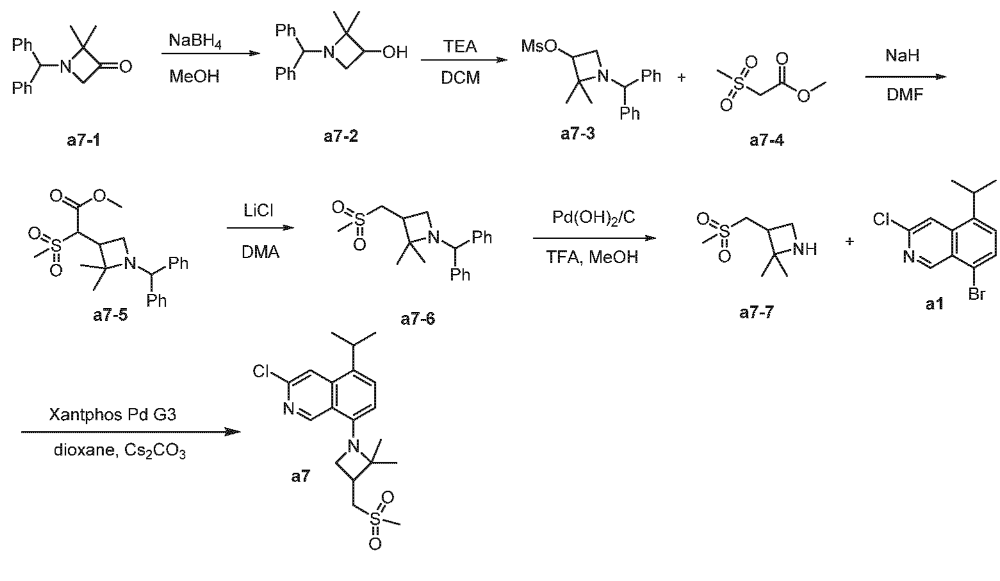


a7-1 a7-2 a7-3 a7-4 a7-5 a7-6 a7-7 a1 a7

Step 1: In an ice bath, the raw material 1-(diphenylmethyl)-2,2-dimethylazetidin-3-one **a7-1** (3.2 g, 12.1 mmol) was dissolved in 50 mL of methanol, and sodium borohydride (912 mg, 24.1 mmol) was added. The mixture was heated to 70°C and reacted for 16 hours. The reaction was stopped, and the solvent was removed by evaporation under reduced pressure. 100 mL of water was added to the residue, and the mixture was extracted with ethyl acetate. The organic phase was washed with brine, dried over anhydrous sodium sulfate, and filtered. The filtrate was concentrated, and separated by flash column chromatography (PE/EA=3/1) to obtain a white solid **a7-2** (2.0 g), yield: 59%, LC-MS: ESI-MS (m/z): 268.1 $[M + H]^+$. 1H NMR (400 MHz, $CDCl_3$) $\delta$ 7.46 (dd, J = 18.3, 7.4 Hz, 4H), 7.24 - 7.08 (m, 6H), 4.58 (s, 1H), 4.05 - 3.95 (m, 1H), 3.40 - 3.28 (m, 1H), 2.72 (dd, *J* = 7.7, 5.9 Hz, 1H), 1.69 (d, *J* = 7.5 Hz, 1H), 1.08 (s, 3H), 1.01 (s, 3H).

Step 2: In an ice bath, the intermediate **a7-2** (1.0 g, 3.74 mmol) from the previous step and triethylamine (605 mg, 5.98 mmol) were dissolved in 20 mL of dichloromethane, and methylsulfonyl chloride (554 mg, 4.86 mmol) was added. The mixture was stirred at room temperature for 1 hour. The reaction was stopped. 50 mL of water was added to the system. The mixture was extracted with dichloromethane and concentrated to obtain a crude product **a7-3** (1.1 g), which was directly used in the next step.

Step 3: In an ice bath, methyl 2-methanesulfonylacetate **a7-4** (745 mg, 4.89 mmol) was dissolved in 20 mL of DMF, and NaH (181 mg, 4.52 mmol, 60%) was added. The reaction solution was stirred at 0°C for 15 minutes, and the crude product **a7-3** (1.1 g) from the previous step was added to the system. The mixture was heated to 80°C and reacted for 16 hours. 50 mL of saturated aqueous ammonium chloride solution was added to the system to quench the reaction. The mixture was extracted with ethyl acetate. The organic phase was washed with brine, dried over anhydrous sodium sulfate, and filtered. The filtrate was concentrated, and separated by flash column chromatography (DCM/MeOH=20/1) to obtain a white solid **a7-5** (900 mg), yield: 54%, LC-MS: ESI-MS (m/z): 402.0 $[M + H]^+$.

Step 4: The intermediate **a7-5** (900 mg, 2.24 mmol) from the previous step was dissolved in 20 mL of DMA, and LiCl (950 mg, 22.42 mmol) was added. The mixture was heated to 150°C and reacted for 1 hour. The reaction was stopped, and 80 mL of water was added to the system. The mixture was extracted with ethyl acetate. The organic phase was washed with brine, dried over anhydrous sodium sulfate, and filtered. The filtrate was concentrated, and separated by flash column chromatography (DCM/MeOH=20/1) to obtain a white solid **a7-6** (200 mg), yield: 27%, LC-MS: ESI-MS (m/z): 344.0 $[M + H]^+$.

Step 5: Under a hydrogen balloon atmosphere, the intermediate **a7-6** (2200 mg, 0.64 mmol) from the previous step was dissolved in 12 mL of methanol. 0.5 mL of trifluoroacetic acid and $Pd(OH)_2/C$ (20 mg) were added. The mixture was reacted at room temperature for 16 hours. The reaction was stopped, and filtered. The filtrate was concentrated under reduced pressure to obtain a yellow trifluoroacetate **a7-7** (150 mg), yield: 92%, LC-MS: ESI-MS (m/z): 178.2 $[M + H]^+$.

Step 6: Under nitrogen protection, the trifluoroacetate salt **a7-7** (92 mg, 0.31 mmol) from the previous step, intermediate **a1** (60 mg, 0.21 mmol) and $Cs_2CO_3$ (171 mg, 0.52 mmol) were mixed in 3 mL of 1,4-dioxane. XantPhos Pd G3 (65 mg, 0.063 mmol) was added. The mixture was heated to 100°C and reacted for 6 hours. The reaction was stopped, and filtered. The filtrate was concentrated, and separated by flash column chromatography to obtain a yellow solid a7 (35 mg), yield: 39%, LC-MS: ESI-MS (m/z): 381.1 $[M + H]^+$.

Synthesis of intermediate **a8**

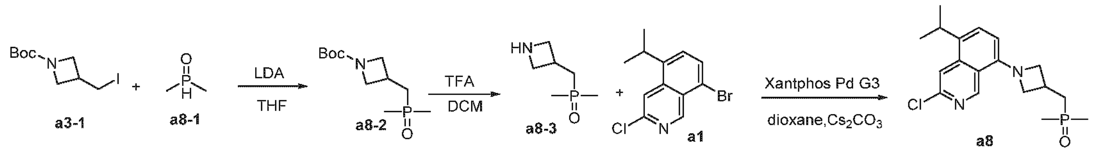


Step 1: Dimethylphosphine oxide **a8-1** (1.0 g, 12.8 mmol) was dissolved in 20 mL of anhydrous tetrahydrofuran at -20°C under nitrogen protection, and LDA (1M, 38.4 mL) was added dropwise. After stirring for 20 minutes, a solution of intermediate **a3-1** (5.72 g, 19.2 mmol) in tetrahydrofuran (10 mL) was added dropwise. After the addition was completed, the mixture was slowly warmed to room temperature, and the mixture was reacted at room temperature for 5 hours. The reaction was stopped. 50 mL of saturated aqueous ammonium chloride solution was added to the system to quench the reaction. The mixture was extracted with ethyl acetate, dried over anhydrous sodium sulfate, and filtered. The filtrate was concentrated, and separated by flash column chromatography to obtain a brown solid **a8-2** (0.8 g), yield: 24%, LC-MS: ESI-MS (m/z): 248.1 $[M + H]^+$.

Step 2: The intermediate **a8-2** (800 mg, 3.23 mmol) was dissolved in 6 mL of dichloromethane, and 3 mL of trifluoroacetic acid was added. The mixture was stirred at room temperature overnight. The reaction solution was concentrated under reduced pressure to obtain a crude product **a8-3**, which was directly used in the next step. LC-MS: ESI-MS (m/z): 148.1 $[M + H]^+$.

Step 3: Under nitrogen protection, the trifluoroacetate salt **a8-3**, intermediate **a1** (774 mg, 2.7 mmol) and $Cs_2CO_3$ (2.66 g, 8.2 mmol) were mixed in 15 mL of 1,4-dioxane. XantPhos Pd G3 (258 mg, 0.27 mmol) was added. The mixture was heated to 100°C and reacted for 16 hours. The reaction was stopped, and filtered. The filtrate was concentrated, and separated by flash column chromatography to obtain a yellow solid **a8** (200 mg), LC-MS: ESI-MS (m/z): 351.0 $[M + H]^+$.

Synthesis of intermediates **a10, a17**

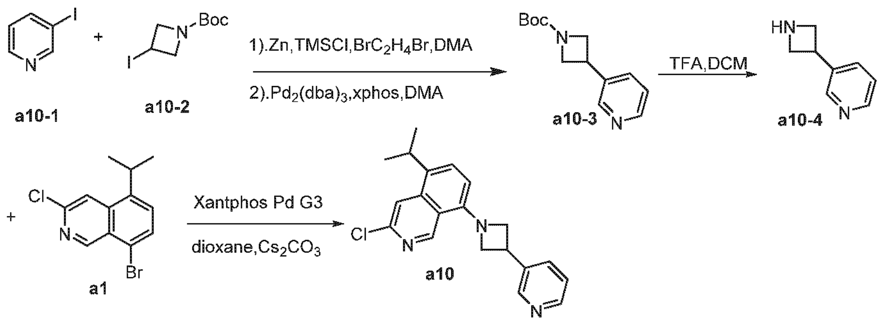


Step 1: Activated zinc powder (0.6 g, 9.16 mmol) was dissolved in 4 mL of DMA, and the mixture was heated to 65 °C. Trimethylsilyl chloride (0.13 g, 0.13 mL) and 1,2-dibromoethane (0.1 mL, 1.3 mmol) were added dropwise to the system. After the addition was completed, the mixture was stirred for another 40 minutes. A solution of **a10-2** (2.0 g, 7.06 mmol) in DMF (4 mL) was added to the reaction solution, and the mixture was reacted for another 30 minutes. The mixture was allowed to cool down to room temperature for use.

Under nitrogen protection, 3-iodopyridine **a10-1** (1.45 g, 7.1 mmol), $Pd_2(dba)_3$ (647 mg, 0.71 mmol) and Xphos (673 mg, 1.41 mmol) were dissolved in 4 mL of DMA in another reaction bottle. The solution prepared from the previous step was added dropwise. The mixture was heated to 85°C and reacted for 16 hours. The reaction was stopped, and the mixture was cooled to room temperature. The reaction solution was poured into 30 mL of ice water, and the mixture was filtered by suction. The filter cake was dissolved in ethyl acetate, concentrated, and separated by flash column chromatography (PE/EA=1/1) to obtain compound **a10-3** (200 mg), yield 11%, LC-MS: ESI-MS (m/z): 235.2 $[M + H]^+$.

Step 2: The intermediate **a10-3** (200 mg, 0.85 mmol) was dissolved in 4 mL of dichloromethane, and 1.5 mL of trifluoroacetic acid was added dropwise. The mixture was reacted at room temperature for 3 hours. The reaction was stopped. The solvent was removed by evaporation under reduced pressure to obtain trifluoroacetate **a10-4** (160 mg) with a yield of 69%, LC-MS: ESI-MS (m/z): 135.1 $[M + H]^+$.

Step 3: Under nitrogen protection, the trifluoroacetate salt **a10-4** (31 mg, 0.23 mmol) from the previous step, intermediate **a1** (65 mg, 0.23 mmol) and $Cs_2CO_3$ (223 mg, 0.69 mmol) were mixed in 3 mL of 1,4-dioxane. XantPhos Pd G3 (22 mg, 0.023 mmol) was added. The mixture was heated to 100°C and reacted for 16 hours. The reaction was stopped, and filtered. The filtrate was concentrated, and separated by flash column chromatography to obtain a yellow solid **a10** (50 mg), LC-MS: ESI-MS (m/z): 338.0 $[M + H]^+$.

Referring to the synthetic route of compound **a10,** the following intermediates were synthesized using a similar skeleton structure.

| Replacement of structure | Target intermediate | LC-MS $[M+H]^+$ |
|---|---|---|
| Replace **a10-1** with : 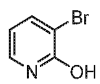 a17-1 | 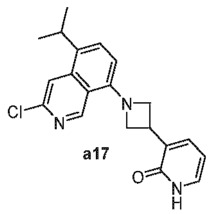 a17 | 354 |

Synthesis of intermediate **a11**

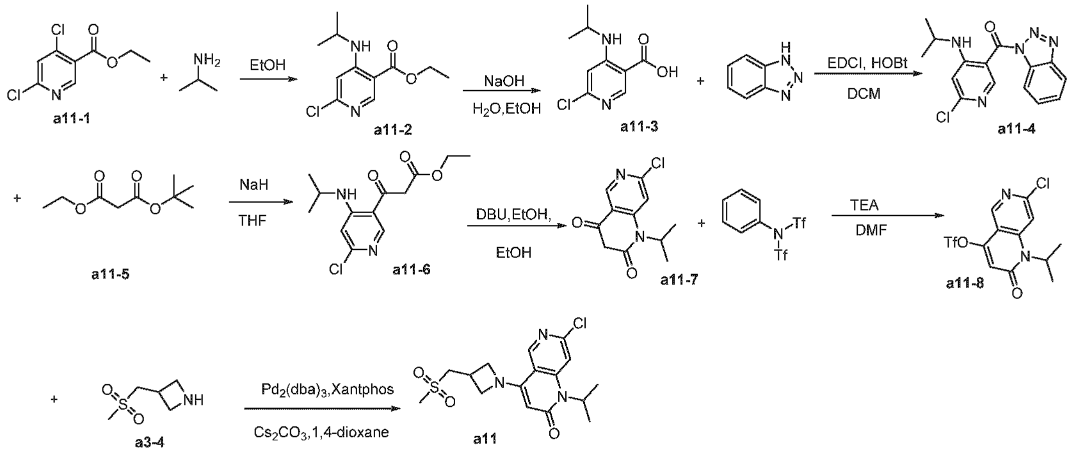

a11-1 a11-2 a11-3 a11-4 a11-5 a11-6 a11-7 a11-8 a3-4 a11

Step 1: Ethyl 4,6-dichloronicotinate **a11-1** (3.0 g, 0.013 mol) was dissolved in 50 mL of ethanol, and isopropylamine (2.41 g, 0.040 mol) was added. The reaction solution was stirred at 80 °C for 12 hours. The reaction was completed by LC-MS monitoring. The solvent was removed by evaporation under reduced pressure, and the residue was separated by column chromatography (PE/EA=10/1) to obtain a white solid **a11-2** (3.31 g), yield: 91%, LCMS: ESI-MS (m/z): 243.1$[M + H]^+$.

Step 2: The intermediate **a11-2** (3.3 g, 13 mmol) was dissolved in 40 mL of ethanol, and 20 mL of 1N aqueous NaOH solution was added. The mixture was heated to 80 °C and reacted for 2 hours. The reaction was stopped. The solvent ethanol was removed by distillation in vacuum, and the residue was adjusted to pH of 3-4 with 1M hydrochloric acid, with a large amount of white solid precipitated. The mixture was filtered by suction. The filter cake was washed with water, and dried to obtain a white solid **a11-3** (1.6 g), yield: 50%, LC-MS: ESI-MS (m/z): 215.0 $[M + H]^+$.

Step 3: The intermediate **a11-3** (1.6 g, 7.48 mmol) from the previous step was dissolved in 30 mL of dichloromethane. Benzotriazole (1.07 g, 8.99 mmol), EDCI (1.73 g, 8.99 mmol) and HOBT (0.3 g, 2.2 mmol) were added. The mixture was reacted at room temperature for 2 hours. The reaction was stopped. 50 mL of water was added to the reaction solution. The mixture was extracted with dichloromethane, dried over anhydrous sodium sulfate, and filtered. The filtrate was concentrated, and separated by column chromatography (PE/EA=10/1) to obtain an oil **a11-4** (1.65 g), yield: 63%, LC-MS: ESI-MS (m/z): 315.9 $[M + H]^+$.

Step 4: In an ice bath, NaH (420 mg, 10.g mmol, 60%) was dissolved in 10 mL of anhydrous tetrahydrofuran under nitrogen protection, and tert-butyl ethyl malonate **a11-5** (1.96 g, 10.5 mmol) was slowly added. The mixture was stirred for 30 minutes, and a solution of intermediate **a11-4** (1.65 g, 5.24 mmol) in tetrahydrofuran (10 mL) was added.

The mixture was stirred for another 2 hours in an ice bath. 30 mL of saturated aqueous ammonium chloride solution was added to the reaction solution to quench the reaction. Tetrahydrofuran was removed by distillation under reduced pressure, and the mixture was extracted with ethyl acetate. The organic phase was washed with saturated brine, dried over anhydrous sodium sulfate, and filtered. The filtrate was concentrated to obtain a crude product. The crude product was dissolved in dichloromethane, and 10 mL of trifluoroacetic acid was added. The mixture was stirred at room temperature for 2 hours. After the reaction was completed, 20 mL of toluene was added and the mixture was separated by column chromatography (PE/EA=10/1) to obtain a colorless oil **a11-6** (780 mg), yield: 48%, LCMS: ESI-MS (m/z): 285.1[M + H]$^+$.

Step 5: The intermediate **a11-6** (780 mg, 2.75 mmol) from the previous step was dissolved in 10 mL of ethanol, and DBU (1.23 g, 8.22 mmol) was added. The mixture was heated to 80°C and reacted for 2 hours. The reaction was stopped. Ethanol was removed by concentration under reduced pressure. 1M hydrochloric acid was added to the reaction solution to adjust the pH to about 3, with a white solid precipitated. The mixture was filtered by suction. The filter cake was washed with water, and dried to obtain intermediate **a11-7** (440 mg), yield: 61%, LC-MS: ESI-MS (m/z): 239.0 [M + H]$^+$.

Step 6: The intermediate **a11-7** (440 mg, 1.67 mmol) from the previous step was dissolved in 10 mL of DMF. Triethylamine (507 mg, 5.02 mmol) and N-phenylbis(trifluoromethanesulfonyl)imide (718 mg, 2.0 mmol) were added, and the mixture was reacted at room temperature for 2 hours. The reaction was stopped. 50 mL of water was added to the reaction solution, and the mixture was extracted with ethyl acetate. The organic phases were combined, washed with saturated brine, dried over anhydrous sodium sulfate, and filtered. The filtrate was concentrated, and separated by thin layer chromatography (PE/EA=3/1) to obtain a white solid **a11-8** (620 mg), yield: 90%, LCMS: ESI-MS (m/z): 371.0 [M + H]$^+$.

Step 7: Under nitrogen protection, the intermediate **a11-8** (210 mg, 0.57 mmol) from the previous step, intermediate **a3-4** (140 mg, 0.53 mmol) and $Cs_2CO_3$ (556 mg, 1.71 mmol) were mixed in 10 mL of 1,4-dioxane. $Pd_2(dba)_3$ (52 mg, 0.057 mmol) and Xantphos (65 mg, 0.113 mmol) were added. The mixture was heated to 60°C and reacted for 2 hours. The reaction was stopped, and filtered. The filtrate was concentrated, and separated by flash column chromatography to obtain a white solid **a11** (30 mg), yield: 15%, LC-MS: ESI-MS (m/z): 370.0 [M + H]$^+$.

Synthesis of intermediate **a12**

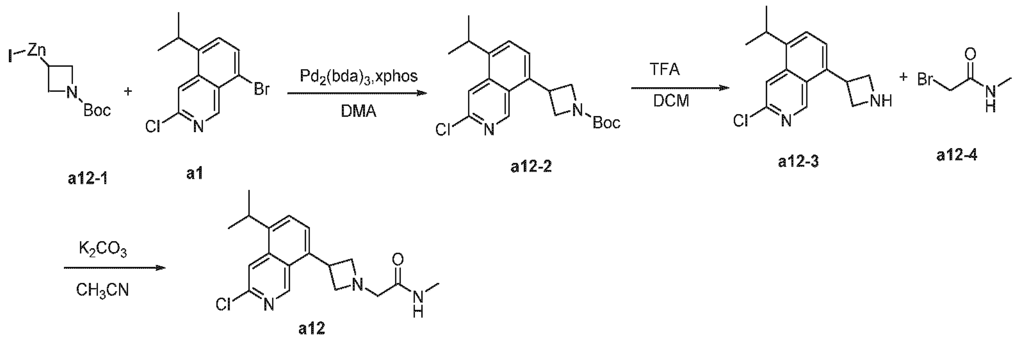


a12-1 a1 a12-2 a12-3 a12-4 a12

Step 1: Under nitrogen protection, the intermediate **a1** (700 mg, 2.46 mmol), $Pd_2(dba)_3$ (225 mg, 0.25 mmol), Xphos (234 mg, 0.49 mmol) and the newly prepared raw material **a12-1** (4 mL, 5.0 mmol) were dissolved in 4 mL of DMA. The mixture was heated to 85°C and reacted for 15 hours. After cooling to room temperature, the reaction solution was poured into 15 mL of ice water, and a solid precipitated. The mixture was filtered by suction. The filter cake was dissolved in ethyl acetate and filtered. The filtrate was concentrated, and separated by flash column chromatography (PE/EA=1/1) to obtain a yellow solid **a12-2** (280 mg), yield: 29%, LC-MS: ESI-MS (m/z): 361.1 [M + H]$^+$.

Step 2: The intermediate **a12-2** (85 mg, 0.24 mmol) from the previous step was dissolved in 6 mL of dichloromethane, and 2 mL of trifluoroacetic acid was added. The mixture was reacted at room temperature for 12 hours. The reaction was stopped. The reaction solution was concentrated to obtain trifluoroacetate **a12-3** (20 mg), yield: 30%, LC-MS: ESI-MS (m/z): 261.1 [M + H]$^+$.

Step 3: The intermediate **a12-3** (20 mg, 0.08 mmol) from the previous step and $K_2CO_3$ (43 mg, 0.31 mmol) were mixed in 5 mL of acetonitrile, and 2-bromo-N-methylacetamide **a12-4** (9 mg, 0.061 mmol) was added. The mixture was reacted at room temperature for 3 hours. The reaction was stopped. 20 mL of water was added to the reaction solution. The mixture was extracted with ethyl acetate, washed with saturated brine, dried over anhydrous sodium sulfate, and filtered. The filtrate was concentrated, and separated by flash column chromatography (DCM/MeOH=10/1) to obtain a white solid **a12** (35 mg), yield: 96%, LC-MS: ESI-MS (m/z): 332.2 [M + H]$^+$.

Synthesis of intermediate **a13**

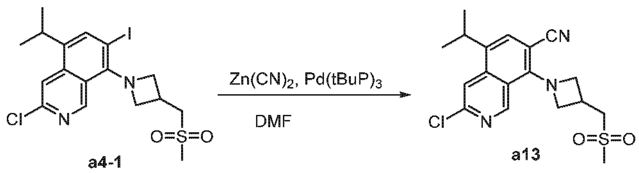


Under nitrogen protection, the intermediate **a4-1** (50 mg, 0.10 mmol) was dissolved in 5 mL of DMF, Pd($^t$BuP)$_3$ (16 mg, 0.03 mmol) and zinc cyanide (18 mg, 0.16 mmol) were added. The mixture was heated to 100°C and reacted for 1 hour. The reaction was completed by LC-MS monitoring. The reaction was stopped. 30 mL of aqueous saturated sodium bicarbonate solution was added to the reaction solution. The mixture was extracted with ethyl acetate, dried over anhydrous sodium sulfate, and filtered. The filtrate was concentrated, and separated by thin layer chromatography to obtain a yellow solid **a13** (30 mg), yield: 69%, LCMS: ESI-MS (m/z): 378.0 [M + H]$^+$.

Synthesis of intermediate **a14**

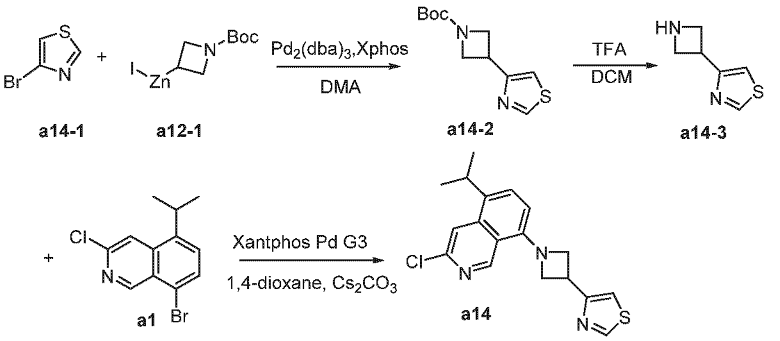


Step 1: Under nitrogen protection, the intermediate **a14-1** (1.0 g, 6.11 mmol), Pd$_2$(dba)$_3$ (558 mg, 0.61 mmol), Xphos (581 mg, 1.22 mmol) and the newly prepared raw material **a12-1** (8 mL, 12.0 mmol) were dissolved in 6 mL of DMA. The mixture was heated to 85°C and reacted for 12 hours. After cooling to room temperature, the reaction solution was poured into 35 mL of ice water. The mixture was extracted with ethyl acetate, concentrated, and separated by flash column chromatography (PE/EA=2/1) to obtain a yellow solid **a14-2** (120 mg), yield: 8%, LC-MS: ESI-MS (m/z): 185.1 [M - 56]$^+$.

Step 2: The intermediate **a14-2** (120 mg, 0.42 mmol) from the previous step was dissolved in 6 mL of dichloromethane, and 2 mL of trifluoroacetic acid was added. The mixture was reacted at room temperature for 0.5 hours. The reaction was stopped. The reaction solution was concentrated to obtain trifluoroacetate **a14-3** (70 mg), yield: 91%, LC-MS: ESI-MS (m/z): 141.1 [M + H]$^+$.

Step 3: Under nitrogen protection, the trifluoroacetate salt **a14-3** (100 mg, 0.71 mmol) from the previous step, intermediate **a1** (203 mg, 0.71 mmol) and Cs$_2$CO$_3$ (1162 mg, 3.57 mmol) were mixed in 10 mL of 1,4-dioxane. XantPhos Pd G3 (68 mg, 0.071 mmol) was added. The mixture was heated to 100°C and reacted for 3 hours. The reaction was stopped, and filtered. The filtrate was concentrated, and separated by flash column chromatography to obtain a yellow solid **a14** (70 mg), yield: 26%, LC-MS: ESI-MS (m/z): 344.1 [M + H]$^+$.

Synthesis of intermediate **a15**

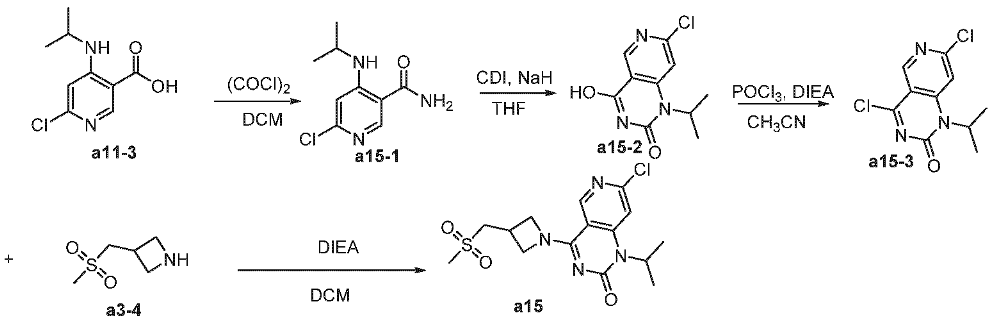


Step 1: In an ice bath, the intermediate **a11-3** (1.3 g, 6.10 mmol) was dissolved in 13 mL of dichloromethane, and oxalyl chloride (1.54 g, 12.1 mmol) and 3 drops of anhydrous DMF were added dropwise. The reaction solution was stirred for 1 hour. 12 mL of ammonia water was added dropwise to the reaction solution. The mixture was heated to room temperature and reacted for another 1 hour. 30 mL of ice water was added to the reaction solution to quench the reaction. The mixture was extracted with dichloromethane, concentrated, and separated by column chromatography (PE/EA=1/1) to obtain a white solid **a15-1** (1.1 g), yield: 85%.

1H NMR (400 MHz, DMSO-$d_6$) $\delta$ 8.72 (d, J = 7.8 Hz, 1H), 8.39 (s, 1H), 8.07 (s, 1H), 7.47 (s, 1H), 6.69 (s, 1H), 3.72-3.77 (m, 1H), 1.16 (d, *J* = 6.3 Hz, 6H).

Step 2: In an ice bath, the intermediate **a15-1** (1.1 g, 5.16 mmol) from the previous step was dissolved in 13 mL of anhydrous tetrahydrofuran, and NaH (1.2 g, 30.1 mmol, 60%) was added. The reaction solution was stirred for 30 minutes. CDI (2.5 g, 15.4 mmol) was added to the reaction solution. The mixture was heated to room temperature and reacted for another 1 hour. The reaction was completed by LC-MS monitoring. 50 mL of ice water was added to the reaction solution. The mixture was extracted with dichloromethane, concentrated, and separated by column chromatography (PE/EA=1/1) to obtain a white solid **a15-2** (1.0 g), yield: 81%, LC-MS: ESI-MS (m/z): 240.0 $[M + H]^+$.

Step 3: The intermediate **a15-2** (1.0 g, 4.18 mmol) from the previous step and DIEA (530 mg, 4.18 mmol) was dissolved in 15 mL of acetonitrile, and $POCl_3$ (640 mg, 4.18 mmol) was added. The reaction solution was heated to 90°C and reacted for 1 hour. The reaction was stopped, and the mixture was cooled to room temperature. The reaction solution was poured into 40 mL of ice water. The mixture was extracted with ethyl acetate, dried with anhydrous sodium sulfate, and filtered. The filtrate was concentrated, and separated by column chromatography (PE/EA=5/1) to obtain a white solid **a15-3** (700 mg), yield: 65%, LC-MS: ESI-MS (m/z): 258.0 $[M + H]^+$.

Step 4: In an ice bath, intermediate **a3-4** (408 mg, 1.55 mmol), intermediate **a15-3** (400 mg, 1.55 mmol) and DIEA (802 mg, 6.19 mmol) were dissolved in 10 mL of dichloromethane. The mixture was reacted in an ice bath for 1 hour. The reaction was stopped. 30 mL of water was added to the reaction solution. The mixture was extracted with dichloromethane, dried over anhydrous sodium sulfate, and filtered. The filtrate was concentrated, and separated by flash column chromatography to obtain a white solid **a15** (300 mg), yield: 47%, LC-MS: ESI-MS (m/z): 371.0 $[M + H]^+$.

Synthesis of intermediates **a16, a18-a22, a24, a27, a29, a31, a34, and a36-a38**

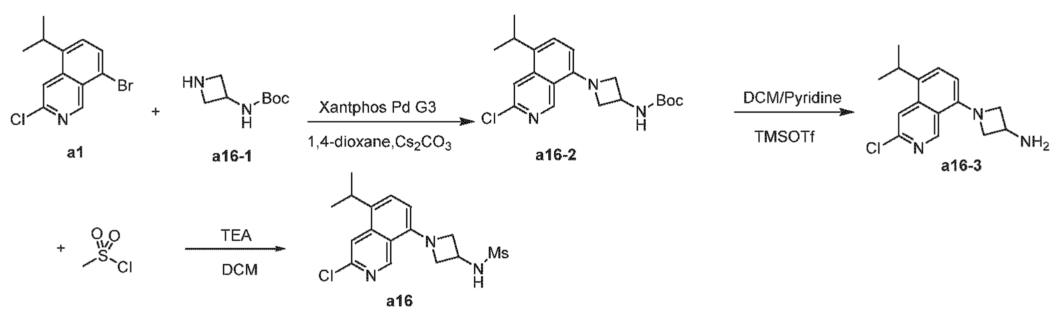


Step 1: Under nitrogen protection, compound **a16-1** (304 mg, 1.76 mmol), intermediate **a1** (500 mg, 1.76 mmol) and $Cs_2CO_3$ (2.86 g, 8.78 mmol) were mixed in 10 mL of 1,4-dioxane. XantPhos Pd G3 (166 mg, 0.17 mmol) was added. The mixture was heated to 100°C for 2 hours. The reaction was stopped. The mixture was filtered. The filtrate was concentrated, and separated by flash column chromatography to obtain a yellow solid **a16-2** (400 mg), yield: 55%, LC-MS: ESI-MS (m/z): 376.2 $[M + H]^+$.

Step 2: In an ice bath, the intermediate **a16-2** (120 mg, 0.32 mmol) from the previous step was dissolved in 2 mL of dichloromethane. 0.5 mL of pyridine was added, and 0.5 mL of trimethylsilyl trifluoromethanesulfonate was slowly added. The mixture was reacted at room temperature for 16 hours. The reaction was completed by LC-MS monitoring. 30 mL of aqueous saturated sodium bicarbonate solution was added to the system to quench the reaction. The mixture was extracted with dichloromethane. The organic phases were combined, washed with saturated brine, dried over anhydrous sodium sulfate, and filtered. The filtrate was concentrated, and separated by thin layer chromatography to obtain a yellow solid **a16-3** (75 mg), yield: 77%, LC-MS: ESI-MS (m/z): 276.2 $[M + H]^+$.

Step 3: The intermediate **a16-3** (21 mg, 0.077 mmol) from the previous step and triethylamine (21 mg, 0.21 mmol) were dissolved in 2 mL of dichloromethane. Methylsulfonyl chloride (139 μL, 0.18 mmol) was added. The mixture was stirred at room temperature for 2 hours. The reaction was stopped. 10 mL of ice water was added to the reaction solution. The mixture was extracted with dichloromethane, dried with anhydrous sodium sulfate, and filtered. The filtrate was concentrated, and separated by thin layer chromatography to obtain a white solid a16 (20 mg), yield: 65%, LC-MS: ESI-MS (m/z): 354.0 $[M + H]^+$.

Referring to the synthetic route of intermediate **a16,** the following intermediates were synthesized using a similar skeleton structure.

| Replacement of structure | Target intermediate | LC-MS $[M+H]^+$ |
|---|---|---|
| Replace **MsCl** in step 3 with 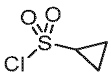 | 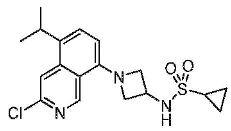 a18 | 380 |
| Replace intermediate **a16-1** with 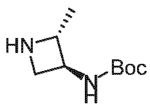 a19-1 | 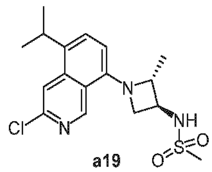 a19 | 368 |
| Replace intermediate **a16-1** with 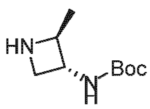 a20-1 | 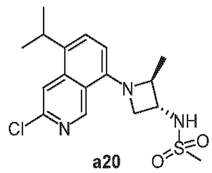 a20 | 368 |
| Replace **MsCl** in step 3 with 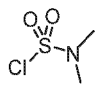 | 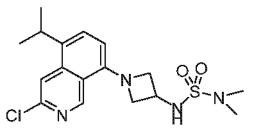 a21 | 383 |
| Replace **MsCl** in step 3 with 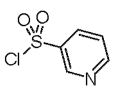 | 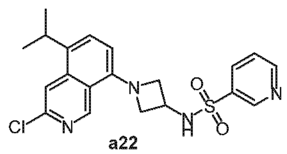 a22 | 417 |
| Replace intermediate **a16-1** with 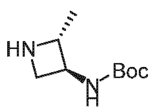 a19-1 ; Replace **MsCl** in step 3 with 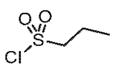 | 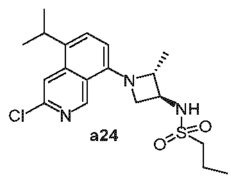 a24 | 396 |

(continued)

| Replacement of structure | Target intermediate | LC-MS [M+H]+ |
|---|---|---|
| Replace **MsCl** in step 3 with 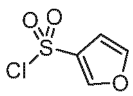 | 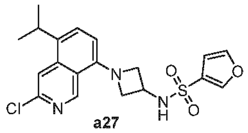 a27 | 406 |
| Replace **MsCl** in step 3 with acetyl chloride | 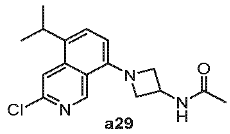 a29 | 318 |
| Replace **MsCl** in step 3 with 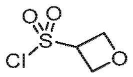 | 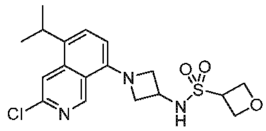 a31 | 396 |
| Replace intermediate **a16-1** with 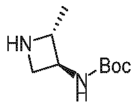 **a19-1** ; Replace **MsCl** in step 3 with 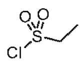 | 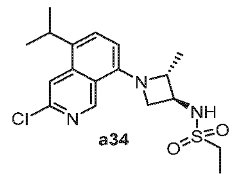 a34 | 382 |
| Replace **MsCl** in step 3 with 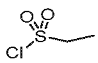 | 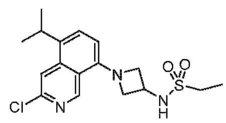 a36 | 368 |
| Replace **MsCl** in step 3 with 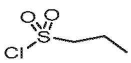 | 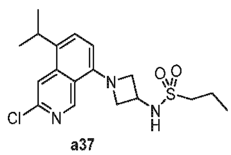 a37 | 382 |
| Replace **MsCl** in step 3 with 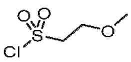 | 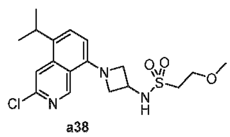 a38 | 399 |

Synthesis of intermediates **a23, a30, a32, a33, a35, a39, and a41**

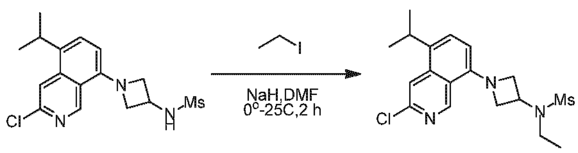


**a16** **a23**

In an ice bath, intermediate **a16** (40 mg, 0.11 mmol) was dissolved in 3 mL of DMF. NaH (6 mg, 0.15 mmol) and iodoethane (20 mg, 0.13 mmol) were added. The mixture was warmed to room temperature and reacted for 2 hours. The reaction was completed by LC-MS monitoring. 30 mL of aqueous saturated sodium bicarbonate solution was added

to the system to quench the reaction. The mixture was extracted with dichloromethane. The organic phases were combined, washed with saturated brine, dried over anhydrous sodium sulfate, and filtered. The filtrate was concentrated, and separated by column chromatography to obtain a yellow solid **a23** (40 mg), yield: 84%, LC-MS: ESI-MS (m/z): 382.1 [M + H]$^+$.

Referring to the synthetic route of intermediate **a23,** the following intermediates were synthesized using a similar skeleton structure.

| Replacement of structure | Target intermediate | LC-MS [M+H]$^+$ |
|---|---|---|
| Replace ethyl iodide **EtI** with methyl iodide **MeI** | 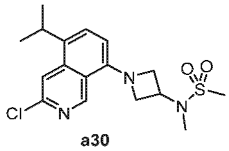 a30 | 368 |
| Replace intermediate **a16** with: 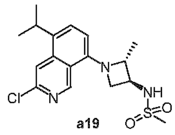 a19<br>Replace ethyl iodide **EtI** with methyl iodide **MeI** | 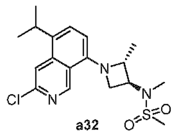 a32 | 382 |
| Replace intermediate **a16** with: 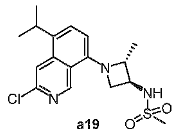 a19<br>Replace ethyl iodide **EtI** with deuterated methyl iodide **$CD_3I$** | 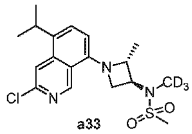 a33 | 385 |
| Replace intermediate **a16** with: 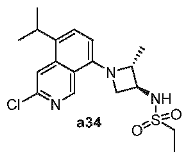 a34<br>Replace ethyl iodide **EtI** with methyl iodide **MeI** | 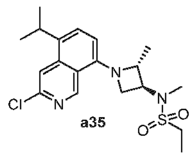 a35 | 396 |
| Replace intermediate **a16** with: 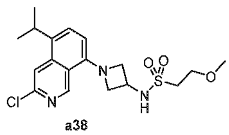 a38<br>Replace ethyl iodide **EtI** with methyl iodide **MeI** | 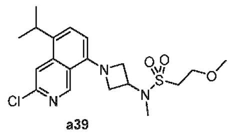 a39 | 412 |
| Replace intermediate **a16** with: 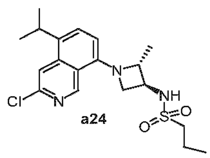 a24<br>Replace ethyl iodide **EtI** with methyl iodide **MeI** | 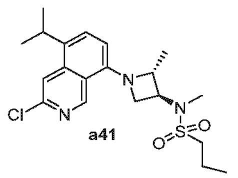 a41 | 410 |

Synthesis of intermediates **a25 and a42**

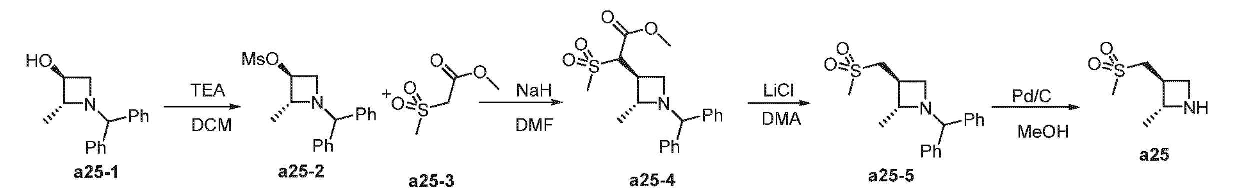


a25-1 a25-2 a25-3 a25-4 a25-5 a25

Step 1: Under nitrogen protection, the raw material **a25-1** (7.0 g, 27.7 mmol) and triethylamine (4.2 g, 41.5 mmol) were dissolved in 40 mL of dichloromethane. Methylsulfonyl chloride (3.21 g, 28.0 mmol) was added. The mixture was reacted at room temperature for 4 hours. The reaction was stopped, and the solvent was removed under reduced pressure to obtain a crude product **a25-2** (10.8 g).

Step 2: The crude product **a25-2** (10.8 g) from the previous step and the raw material **a25-3** (4.21 g, 27.7 mmol) were dissolved in 30 mL of DMF. NaH (1.11 g, 27.7 mmol) was added. The mixture was slowly heated to 80 °C and reacted for 12 hours. The reaction was completed by LC-MS monitoring. 100 mL of saturated saline solution was added to the system to quench the reaction. The mixture was extracted with dichloromethane. The organic phases were combined, washed with saturated brine, dried with anhydrous sodium sulfate, and filtered. The filtrate was concentrated to obtain a crude product **a25-4** (15 g). LC-MS: ESI-MS (m/z): 388.3 $[M + H]^+$.

Step 3: The crude product **a25-4** (15 g) from the previous step and LiCl (2.52 g, 60.0 mmol) were dissolved in 40 mL of N,N-dimethylacetamide DMA. The mixture was heated to 150°C and reacted for 2 hours. The reaction was stopped. 100 mL of ice water was added to the reaction solution. The mixture was extracted with dichloromethane, dried over anhydrous sodium sulfate, and filtered. The filtrate was concentrated, and separated by flash column chromatography to obtain a yellow solid **a25-5** (6.42 g), with a total yield of 71% for three steps, LC-MS: ESI-MS (m/z): 330.1 $[M + H]^+$.

Step 4: Under hydrogen atmosphere (2 atm), the intermediate **a25-5** (6.42 g, 19.5 mmol) from the previous step and Pd/C (1.28 g, 20%) were mixed in 40 mL of methanol. 2 mL of trifluoroacetic acid was added. The reaction was allowed to react at room temperature for 16 hours. The reaction was stopped. The mixture was filtered and concentrated to obtain a yellow oil **a25** (4.5 g), which was directly used in the next reaction. LC-MS: ESI-MS (m/z): 164.2 $[M + H]^+$.

Referring to the synthetic route of intermediate a25, the following intermediate was synthesized using a similar skeleton structure.

| Replacement of structure | Target intermediate | LC-MS $[M+H]^+$ |
|---|---|---|
| Replace **a25-3** with: 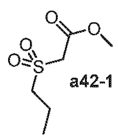 a42-1 | 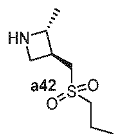 a42 | 192 |

Synthesis of intermediates **a26 and a43**

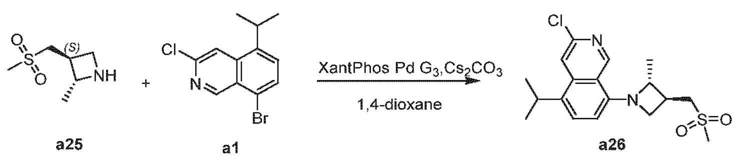


a25 a1 a26

Under nitrogen protection, intermediate **a25** (2.0 g, 12.3 mmol), intermediate **a1** (3.47 g, 12.3 mmol) and $Cs_2CO_3$ (8.01 g, 24.6 mmol) were mixed in 80 mL of 1,4-dioxane. XantPhos Pd G3 (1.14 g, 1.2 mmol) was added. The mixture was heated to 100°C and reacted for 6 hours. The reaction was stopped. The mixture was filtered. The filtrate was concentrated, and separated by flash column chromatography to obtain a brown solid **a26** (970 mg), yield: 23%, LC-MS: $M+H^+$= 367.3.

Referring to the synthetic route of intermediate **a26,** the following intermediate was synthesized using a similar skeleton structure.

| Replacement of structure | Target intermediate | LC-MS $[M+H]^+$ |
|---|---|---|
| Replace **a25** with **a42** | 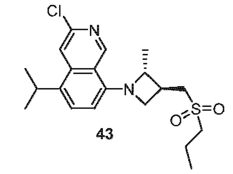 43 | 395 |

Synthesis of intermediate **a28**

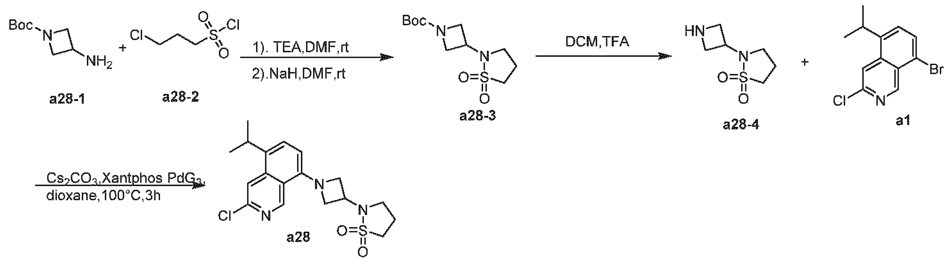


a28-1 a28-2 a28-3 a28-4 a1 a28

Step 1: In an ice bath, under nitrogen protection, the raw material **a28-1** (250 mg, 1.45 mmol) and triethylamine (294 mg, 2.9 mmol) were dissolved in 5 mL of DMF. 3-Chloropropylsulfonyl chloride **a28-2** (282 mg, 1.59 mmol) was added. The mixture was stirred for 30 minutes, and NaH (174 mg, 4.35 mmol) was added. The mixture was warmed to room temperature and reacted for 12 hours. The reaction was stopped. 30 mL of aqueous saturated $NH_4Cl$ solution was added to the system. The mixture was extracted with ethyl acetate, dried over anhydrous sodium sulfate, and filtered. The filtrate was concentrated to obtain a crude product **a28-3** (380 mg), yield: 83%, LC-MS: $M+Na^+$= 299.0.

Step 2: The intermediate **a28-3** (220 mg, 0.72 mmol) from the previous step was dissolved in 7 mL of dichloromethane, and 3 mL of trifluoroacetic acid was added. The mixture was reacted at room temperature for 2 hours. The reaction was stopped. The solvent was removed by evaporation under reduced pressure. 10 mL of water was added, and the mixture was freeze-dried to obtain a white solid **a28-4** (150 mg), yield: 65%, LC-MS: $M+H^+$= 177.1.

Step 3: Under nitrogen protection, the intermediate trifluoroacetate **a28-4** (100 mg, 0.34 mmol), intermediate **a1** (212 mg, 0.75 mmol) and $Cs_2CO_3$ (332 mg, 1.02 mmol) were mixed in 10 mL of 1,4-dioxane. XantPhos Pd G3 (35 mg, 0.034 mmol) was added. The mixture was heated to 100°C and reacted for 3 hours. The reaction was stopped, and the mixture was filtered. The filtrate was concentrated, and separated by flash column chromatography to obtain a yellow solid **a28** (50 mg), yield: 35%, LC-MS: $M+H^+$= 380.1.

Synthesis of intermediate **a40**

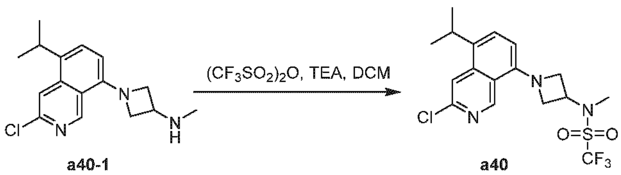


a40-1 a40

Referring to the synthetic route of intermediate **a16**, intermediate **a40-1** was obtained. Operation steps: The intermediate 1-(3-chloro-5-isopropylisoquinolin-8-yl)-N-methylazetidin-3-amine **a40-1** (10 mg, 0.035 mmol) and triethylamine (11 mg, 0.11 mmol) were dissolved in 3 mL of dichloromethane. 1.0 mL of a solution of trifluoromethanesulfonic anhydride (20 mg, 0.07 mmol) in dichloromethane was added dropwise. The mixture was reacted at room temperature for 2 hours. 30 mL of aqueous saturated sodium bicarbonate solution was added to the system to quench the reaction. The mixture was extracted with ethyl acetate, dried over anhydrous sodium sulfate, and concentrated. The crude product

was separated by thin layer chromatography (DCM/MeOH, 10/1) to obtain a light yellow solid **a40** (15 mg), yield: 93%, LC-MS: M+H$^+$= 422.1.

Synthesis of intermediates **b1 and b2**

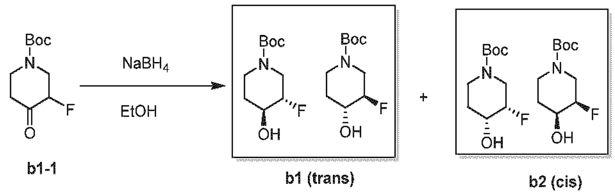


b1-1 b1 (trans) b2 (cis)

In an ice bath, under nitrogen protection, the raw material tert-butyl 3-fluoro-4-oxopiperidin-1-carboxylate intermediate **b1-1** (3.0 g, 14.0 mmol) was dissolved in 50 mL of ethanol. Sodium borohydride (0.78 g, 21.0 mmol) was added. The mixture was heated to room temperature and reacted for 4 hours. The reaction was completed by LC-MS monitoring. 50 mL of aqueous saturated ammonium chloride solution was added to the reaction solution to quench the reaction. The mixture was extracted with ethyl acetate, washed with saturated brine, dried over anhydrous sodium sulfate, concentrated, and separated by flash column chromatography (PE/EA=2/1) to obtain trans intermediate **b1** and cis intermediate **b2** (2.52 g), yield: 90%, LCMS: ESI-MS (m/z): 164.0 [M -55]$^+$.

Synthesis of intermediates **b3-b5, b10-b12, x1 and x2**

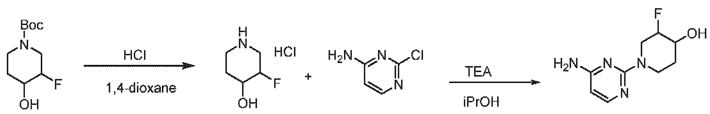


b2 cis b3-1 (cis) b3-2 b3 (cis)

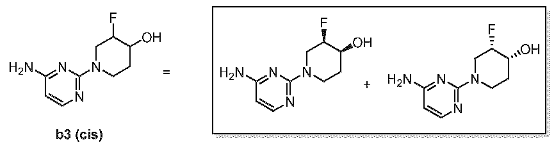

b3 (cis)

Under nitrogen protection, cis-tert-butyl 3-fluoro-4-hydroxypiperidin-1-carboxylate **b2** (800 mg, 3.63 mmol) was dissolved in 5 mL of 1,4-dioxane. A 4M solution of hydrogen chloride in dioxane (4.5 mL, 18.16 mmol) was slowly added dropwise. The mixture was reacted at room temperature for 2 hours, and the solvent was removed by evaporation under reduced pressure to obtain compound **b3-1** (210 mg), which was directly used in the next step.

cis-3-Fluoropiperidin-4-ol hydrochloride **b3-1** (840 mg, 5.4 mmol), 2-chloropyrimidin-4-amine **b3-2** (700 mg, 5.4 mmol) and triethylamine (1.63 g, 16.2 mmol) was dissolved in 20 mL of isopropanol. The reaction system was sealed and reacted at 110°C for 18 hours. After cooling to room temperature, the reaction solution was concentrated under reduced pressure and separated by column chromatography (DCM/MeOH=21/1) to obtain a yellow solid cis-**b3** (200 mg), yield: 16%, LC-MS: ESI-MS (m/z): 283.9 [M + H]$^+$.

Referring to the synthetic route of compound **b3,** the following intermediates were synthesized using a similar skeleton structure.

| Replacement of structure | Target intermediate | LC-MS [M+H]$^+$ |
|---|---|---|
| Replace **b2 (cis)** with **b1 (trans)** | 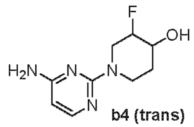 b4 (trans) | 213 |
| Replace **b2 (cis)** with the following structure: 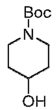 **b5-1** | 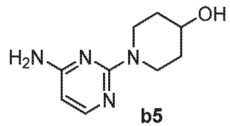 **b5** | 195 |
| Replace **b2 (cis)** with the following structure: 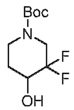 **b10-1** | 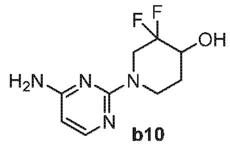 **b10** | 231 |
| Replace **b2 (cis)** with the following structure: 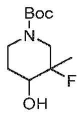 **b11-1 (cis)** = 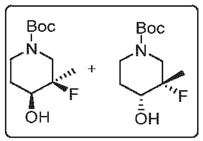 | 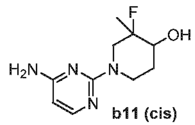 **b11 (cis)** = 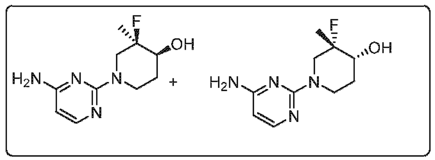 | 227 |

(continued)

| Replacement of structure | Target intermediate | LC-MS [M+H]+ |
|---|---|---|
| Replace **b2 (cis)** with the following<br>structure: 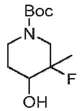<br>**b12-1 (trans)**<br>= 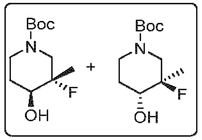 | 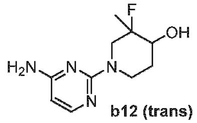<br>**b12 (trans)**<br>=<br>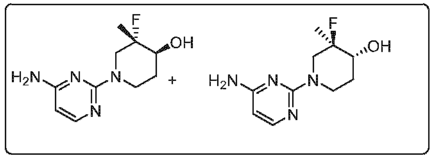 | 227 |
| Replace **b2 (cis)** with the following<br>structure: 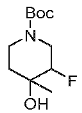<br>**b13-1 (cis)**<br>= 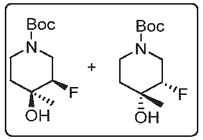 | 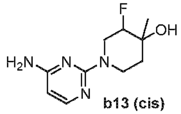<br>**b13 (cis)**<br>=<br>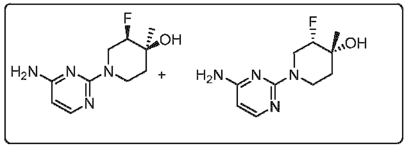 | 227 |
| Replace **b2 (cis)** with **b2** chiral **(3S, 4R)** | 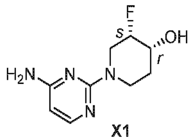<br>**X1** | 213 |
| Replace **b2 (cis)** with **b2** chiral **(3R, 4S)** | 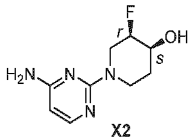<br>**X2** | 213 |

Synthesis of intermediates **b6-b8**

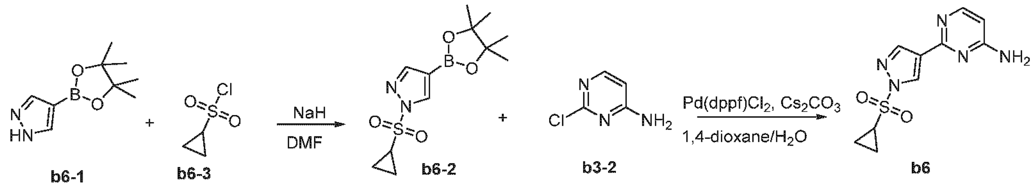


b6-1 + b6-3 → b6-2 + b3-2 → b6

In an ice bath, the raw material 4-(4,4,5,5-tetramethyl-1,3,2-dioxaborolan-2-yl)-1H-pyrazole **b6-1** (200 mg, 1.03 mmol) was dissolved in 2 mL of DMF. 60% NaH (82 mg, 2.06 mmol) was added. The mixture was stirred for 1 hour, and cyclopropanesulfonyl chloride (217 mg, 1.54 mmol) was added. The mixture was warmed to room temperature and stirred for 5 hours. 30 mL of ice water was added to the reaction solution to quench the reaction. The mixture was extracted with ethyl acetate, washed with saturated brine, dried over anhydrous sodium sulfate, and filtered. The filtrate was concentrated to obtain crude **b6-2,** which was directly used in the next step. LCMS: ESI-MS (m/z): 299.1 $[M + H]^+$.

Under nitrogen protection, the crude product **b6-2** (288 mg, 0.58 mmol) from the previous step, 2-chloropyrimidin-4-amine **b3-2** (50 mg, 0.39 mmol) and $Cs_2CO_3$ (252 mg, 0.77 mmol) were mixed in 6 mL of a mixture of 1,4-dioxane and water (v/v=5/1). $Pd(dppf)Cl_2$ (57 mg, 0.077 mmol) was added. The mixture was heated to 100°C and reacted for 6 hours. The reaction was completed by LC-MS monitoring. The mixture was filtered, and 20 mL of water was added to the filtrate. The mixture was extracted with ethyl acetate, dried over anhydrous sodium sulfate, and filtered. The filtrate was concentrated, and separated by flash column chromatography (PE/EA=2/1) to obtain compound **b6** (30 mg), yield: 27%, LCMS: ESI-MS (m/z): 266.0 $[M + H]^+$.

Referring to the synthetic route of compound b6, the following intermediates were synthesized using a similar skeleton structure.

| Replacement of structure | Target intermediate | LC-MS $[M+H]^+$ |
|---|---|---|
| Replace **b6-2** with 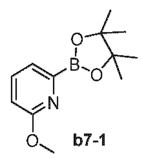 b7-1 | 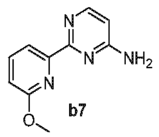 b7 | 203 |
| Replace **b6-3** with 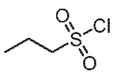 b8-1 | 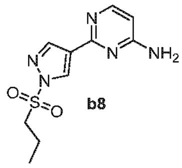 b8 | 268 |

Synthesis of intermediates **b9, x3 and x4**

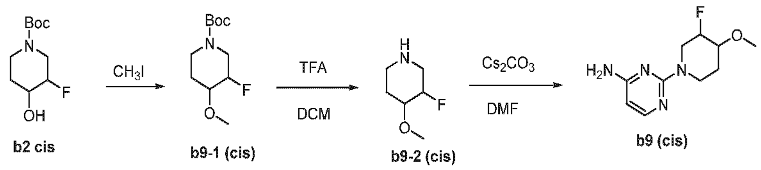


b2 cis → b9-1 (cis) → b9-2 (cis) → b9 (cis)

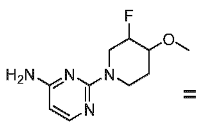
b9 (cis)

=

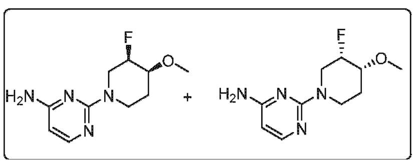

In an ice bath, cis intermediate **b2** (600 mg, 2.72 mmol) was dissolved in 6 mL of anhydrous tetrahydrofuran. NaH (131 mg, 3.3 mmol) was added, and iodomethane (426 mg, 3.0 mmol) was added dropwise. The mixture was heated to room temperature and reacted for 3 hours. 20 mL of ice water was added to the system to quench the reaction. The mixture was extracted with ethyl acetate, dried over anhydrous sodium sulfate, and filtered. The filtrate was concentrated, and separated by flash column chromatography (PE/EA=5/1) to obtain a light yellow solid **b9-1** (600 mg), yield: 94%, LCMS: ESI-MS (m/z): 134.0 $[M-55]^+$.

Under nitrogen protection, the cis intermediate **b9-1** (600 mg, 2.56 mmol) was dissolved in 5 mL of dichloromethane. 2 mL of trifluoroacetic acid was slowly added dropwise. The mixture was reacted at room temperature for 2 hours. The solvent was removed by evaporation under reduced pressure to obtain compound **b9-2** (340 mg), which was directly used in the next step.

The cis trifluoroacetate **b9-2** (340 mg, 1.37 mmol), 2-chloropyrimidin-4-amine **b3-2** (125 mg, 0.96 mmol) and cesium carbonate (896 mg, 2.72 mmol) were mixed in 8 mL of DMF. The reaction system was reacted at 100°C for 1 hour. After cooling to room temperature, 30 mL of water was added to the system. The mixture was extracted with ethyl acetate, dried over anhydrous sodium sulfate, and filtered. The filtrate was concentrated, and separated by column chromatography (EtOAc) to obtain a white solid cis **b9** (200 mg), yield: 65%, LC-MS: ESI-MS (m/z): 227.1 $[M + H]^+$.

Referring to the synthetic route of compound **b9,** the following intermediates were synthesized using a similar skeleton structure.

| Replacement of structure | Target intermediate | LC-MS $[M+H]^+$ |
|---|---|---|
| Replace **b2 (cis)** with **b2** chiral **(3S, 4R)** | 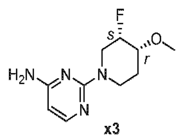 **x3** | 227 |
| Replace **b2 (cis)** with **b2** chiral **(3R, 4S)** | 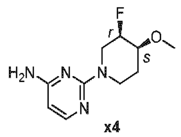 **x4** | 227 |

**Example 2**

**Preparation of target molecules P1-P5, P7-P9, P11-P19, P21-P51**

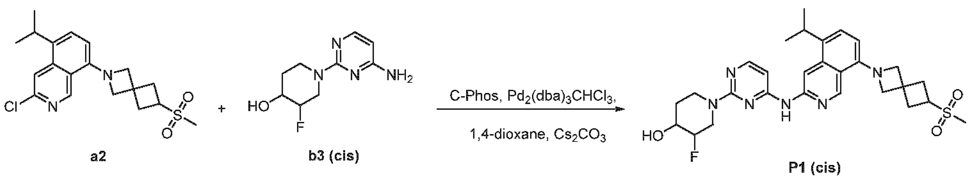

**a2** **b3 (cis)** **P1 (cis)**

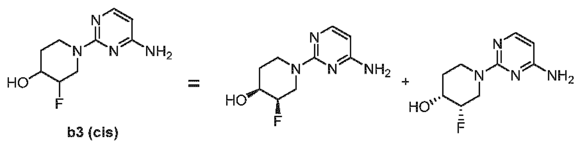
**b3 (cis)**

Under nitrogen protection, intermediate **a2** (70 mg, 0.185 mmol), $Cs_2CO_3$ (180 mg, 0.55 mmol) and intermediate **b3** (39 mg, 0.185 mmol) were mixed in 2 mL of 1,4-dioxane. $Pd_2(dba)_3CHCl_3$ (56 mg, 0.055 mmol) and C-phos (24 mg, 0.055 mmol) were added. The mixture was heated to 100°C and reacted for 12 hours. The reaction was completed by LC-MS monitoring. The mixture was filtered. The filtrate was concentrated, and separated by HPLC preparative chromatography to obtain a yellow solid **P1** (4.0 mg), yield: 4%, LCMS: ESI-MS (m/z): 555.0 $[M + H]^+$.

$^1$HNMR (400 MHz, DMSO-$d_6$ ) $\delta$ 9.87 (s, 1H), 9.00 (s, 1H), 8.60 (s, 1H), 7.95 (d, *J* = 5.7 Hz, 1H), 7.36 (d, *J* = 8.0 Hz, 1H), 6.41 (d, *J* = 5.7 Hz, 1H), 6.34 (d, *J* = 8.1 Hz, 1H), 5.11 (d, *J* = 5.1 Hz, 1H), 4.74 - 4.58 (m, 1H), 4.58 - 4.52 (m, 1H), 4.38-4.30 (m, 1H), 4.17 (s, 2H), 4.08 *(s, J* = 6.7 Hz, 2H), 3.93 - 3.76 (m, 2H), 3.59 - 3.40 (m, 2H), 2.87 (s, 3H),

2.64 - 2.53 (m, 4H), 1.72-1.64 (m, 2H), 1.25 (dd, $J$ = 6.7, 4.6 Hz, 6H).

Referring to the synthetic route of compound **P1,** the following target molecules were synthesized using a similar skeleton structure (unless otherwise stated, the compounds are in cis configuration).

| Replacement of structure | Target molecules | $^1$H NMR | LC-MS $[M+H]^+$ |
|---|---|---|---|
| Replace **b3 (cis)** with: **b4 (trans)** 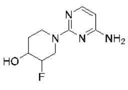 = b4 (trans) 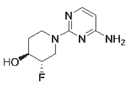 + 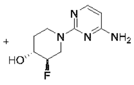 | 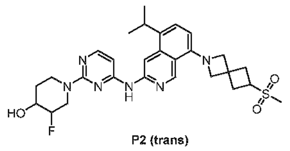 P2 (trans) | $^1$HNMR (400 MHz, DMSO-$d_6$) $\delta$ 9.90 (s, 1H), 9.00 (s, 1H), 8.59 (s, 1H), 7.97 (d, $J$ = 5.6 Hz, 1H), 7.35 (d, $J$ = 8.0 Hz, 1H), 6.44 (d, $J$ = 5.7 Hz, 1H), 6.33 (d, $J$ = 8.1 Hz, 1H), 5.31 (d, $J$ = 4.7 Hz, 1H), 4.48 - 4.21 (m, 2H), 4.16 (s, 3H), 4.07 (s, 2H), 3.93 - 3.83 (m, 1H), 3.80 - 3.71 (m, 1H), 3.54 -3.48 (m, 1H), 3.47 - 3.38 (m, 2H), 2.87 (s, 3H), 2.59 - 2.56 (m, 4H), 1.92 - 1.81 (m, 1H), 1.50 - 1.39 (m, 1H), 1.24 (dd, $J$ = 6.6, 5.4 Hz, 6H). | 555.0 |
| Replace **b3 (cis)** with: 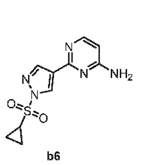 b6 | 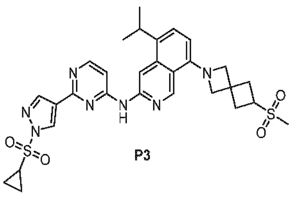 P3 | $^1$H NMR (400 MHz, DMSO-$d_6$) $\delta$ 10.35 (s, 1H), 9.06 (s, 1H), 8.73 (s, 1H), 8.66 (s, 1H), 8.48 (s, 1H), 8.39 (d, $J$ = 5.9 Hz, 1H), 7.39 (d, $J$ = 8.0 Hz, 1H), 7.19 (s, 1H), 6.37 (d, $J$ = 8.1 Hz, 1H), 4.19 (s, 2H), 4.11 (s, 2H), 3.94 - 3.85 (m, 1H), 3.58 - 3.48 (m, 1H), 3.24 (dd, $J$ = 8.5, 3.8 Hz, 1H), 2.88 (s, 3H), 2.63 - 2.54 (m, 4H), 1.33 (d, $J$ = 6.8 Hz, 6H), 1.31 - 1.21 (m, 4H). | 608.7 |
| Replace **b3 (cis)** with: 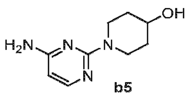 b5 | 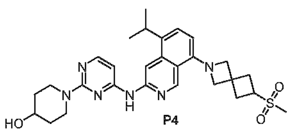 P4 | $^1$H NMR (400 MHz, DMSO-$d_6$) $\delta$ 9.87 (s, 1H), 9.03 (s, 1H), 8.67 (s, 1H), 7.98 (d, $J$ = 5.6 Hz, 1H), 7.39 (d, $J$ = 8.0 Hz, 1H), 6.39 (dd, $J$ = 14.6, 6.9 Hz, 2H), 4.75 (d, $J$ = 4.2 Hz, 1H), 4.40 - 4.32 (m, 2H), 4.21 (s, 2H), 4.12 (s, 2H), 3.97 - 3.88 (m, 1H), 3.80 - 3.72 (m, 1H), 3.46-3.42(m, 1H), 3.33 (s, 1H), 3.29 (s, 1H), 2.91 (s, 3H), 2.61 (dd, $J$ = 7.9, 3.2 Hz, 4H), 1.86 - 1.77 (m, 2H), 1.39 (td, $J$ = 12.9, 4.1 Hz, 2H), 1.28 (d, $J$ = 6.8 Hz, 6H). | 661.0 |
| Replace **a2** with: 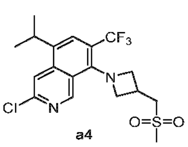 a4 | 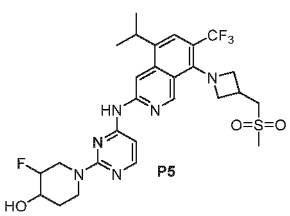 P5 | $^1$H NMR (400 MHz, DMSO-$d_6$) $\delta$ 10.53 (s, 1H), 9.08 (s, 1H), 8.49 (s, 1H), 7.99 (d, $J$ = 6.2 Hz, 1H), 7.37 (s, 1H), 6.56 (s, 1H), 5.35 - 5.00 (m, 1H), 4.81 - 4.61 (m, 3H), 4.59 - 4.43 (m, 1H), 4.28 - 4.24 (m, 2H), 3.92-3.80 (m, 2H), 3.66 - 3.59 (m, 1H), 3.55 (d, $J$ = 7.5 Hz, 2H), 3.42 - 3.32 (m, 2H), 3.23 (dt, $J$ = 21.4, 7.4 Hz, 1H), 2.94 (s, 3H), 1.76 - 1.68 (m, 2H), 1.26 (dd, $J$ = 6.7, 4.9 Hz, 6H). | 597.2 |

(continued)

| Replacement of structure | Target molecules | $^1$H NMR | LC-MS [M+H]$^+$ |
|---|---|---|---|
| Replace **a2** with: 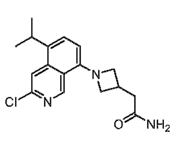 a6 | 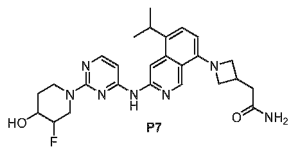 P7 | 1H NMR (400 MHz, MeOD) δ 9.07 (s, 1H), 8.64 (s, 1H), 7.92 (d, *J* = 5.7 Hz, 1H), 7.42 (d, *J* = 8.0 Hz, 1H), 6.47 (d, *J* = 8.2 Hz, 1H), 6.43 (d, *J* = 6.3 Hz, 1H), 4.76 (s, 1H), 4.62 (d, *J* = 8.2 Hz, 1H), 4.46 - 4.43 (m, 1H), 4.37-4.40 (m, 2H), 3.89 - 4.00 (m, 1H), 3.85-3.89 (m, 2H), 3.57 -3.65 (m, 2H), 3.45-3.50 (m, 1H), 3.08-3.13 (m, 1H), 2.62-2.65 (d, *J* = 8.2 Hz, 2H), 1.84-1.90 (m, 1H), 1.80-1.82 (m, 1H), 1.33 (dd, *J* = 6.7, 4.5 Hz, 6H). | 493.9 |
| Replace **a2** with: 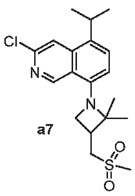 a7 | 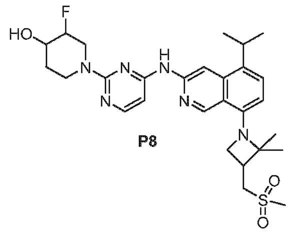 P8 | $^1$H NMR (400 MHz, DMSO-$d_6$) δ 9.93 (s, 1H), 9.30 (s, 1H), 8.64 (s, 1H), 8.20 (s, 0.3 H), 7.99 (d, *J* = 5.7 Hz, 1H), 7.47 (d, *J* = 8.0 Hz, 1H), 6.87 (d, *J* = 8.0 Hz, 1H), 6.42 *(d, J* = 5.4 Hz, 1H), 5.15 (d, *J* = 4.7 Hz, 1H), 4.78 - 4.56 (m, 2H), 4.43 - 4.34 (m, 1H), 3.98 - 3.90 (m, 2H), 3.89 - 3.80 (m, 1H), 3.61 - 3.54 (m, 1H), 3.51-3.46 (m, 2H), 3.05 (s, 3H), 3.01-2.93 (m, 1H), 1.83 - 1.65 (m, 3H), 1.56 (s, 2H), 1.35 - 1.25 (m, 6H), 0.99 (s, 3H). | 557.2 |
| Replace **a2** with: 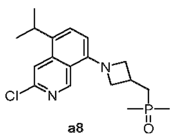 a8 | 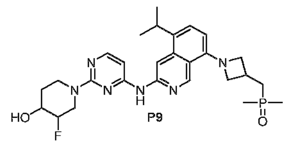 P9 | $^1$H NMR (400 MHz, DMSO-$d_6$) δ 11. 10 (s, 1H), 9.15 (s, 1H), 8.56 (s, 1H), 8.00 (d, *J* = 6.9 Hz, 1H), 7.51 (d, *J* = 8.1 Hz, 1H), 6.72 (s, 1H), 6.51 (d, *J* = 8.1 Hz, 1H), 4.88 (s, 1H), 4.76 (s, 1H), 4.50 (s, 1H), 4.44 - 4.40 (m, 2H), 3.97 (m, 1H), 3.91 - 3.87(m, 2H), 3.71 (dd, *J* = 30.2, 13.7 Hz, 2H), 3.50 - 3.40 (m, 2H), 3.15 - 3.10(m, 1H), 2.13 (dd, *J* = 12.0, 7.7 Hz, 2H), 1.81 (d, *J* = 4.8 Hz, 2H), 1.42 (d, *J* = 13.0 Hz, 6H), 1.30 (t, *J* = 6.3 Hz, 6H). | 527.3 |
| Replace **a2** with: 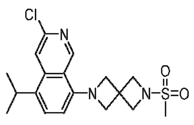 a9 | 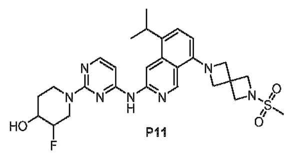 P11 | 1H NMR (400 MHz, DMSO-$d_6$) δ 9.92 (s, 1H), 9.05 (s, 1H), 8.65 (s, 1H), 8.07 (s, 1H), 8.00 (d, J = 5.6 Hz, 1H), 7.42 (d, J = 8.0 Hz, 1H), 6.45 (d, J = 5.6 Hz, 1H), 6.41 (d, J = 8.1 Hz, 1H), 5.15 (d, J = 4.9 Hz, 1H), 4.74 (m, 1H), 4.64(m, 2H), 4.39 (m, 1H), 4.31 (s, 4H), 4.13 (s, 4H), 3.87 (m, 1H), 3.48 (m, 1H), 3.03 (s, 3H), 1.72 (d, J = 4.8 Hz, 2H), 1.29 (dd, J = 6.6, 4.6 Hz, 6H). | 556.2 |

(continued)

| Replacement of structure | Target molecules | $^1H$ NMR | LC-MS $[M+H]^+$ |
|---|---|---|---|
| Replace **a2** with: 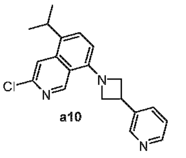 a10 | 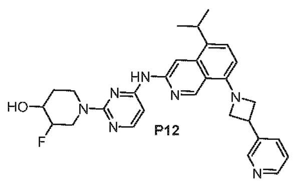 P12 | 1H NMR (400 MHz, DMSO-$d_6$) $\delta$ 9.89 (s, 1H), 9.13 (s, 1H), 8.66 (s, 2H), 8.49 (d, *J* = 3.7 Hz, 1H), 8.33 (s, 2H), 7.99 - 7.96 (m, 2H), 7.52 - 7.38 (m, 2H), 6.48 (dd, *J* = 19.8, 6.8 Hz, 2H), 4.75 (s, 1H), 4.63 - 4.55 (m, 3H), 4.39 (d, *J* = 13.0 Hz, 1H), 4.17-4.20 (m, 2H), 4.09 (d, *J* = 6.4 Hz, 1H), 3.90 - 3.83 (m, 1H), 3.48-3.60 (m, 4H), 1.73 (d, *J* = 4.7 Hz, 2H), 1.23-1.33 (m, 6H). | 514.2 |
| Replace **a2** with: 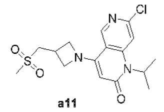 a11 Replace **b3 (cis)** with: 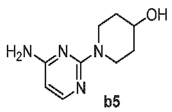 b5 | 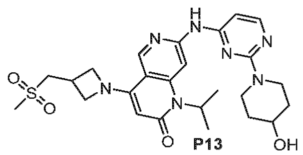 P13 | $^1H$ NMR (400 MHz, DMSO-$d_6$) $\delta$ 9.94 (s, 1H), 8.52 (s, 1H), 8.02 (d, *J* = 5.6 Hz, 2H), 6.70 (s, 1H), 5.05 (s, 1H), 4.75 - 4.65 (m, 1H), 4.42 (t, *J* = 8.2 Hz, 2H), 4.30 - 4.18 (m, 2H), 4.08 - 3.98 (m, 2H), 3.75 - 3.65 (m, 1H), 3.55 (d, *J* = 7.3 Hz, 2H), 3.28-3.16 (m, 4H), 2.97 (s, 3H), 1.80-1.70 (m, 2H), 1.48 (d, *J* = 6.8 Hz, 6H), 1.36 - 1.28 (m, 2H). | 528.2 |
| Replace **a2** with: 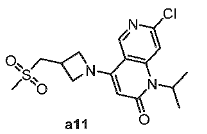 a11 | 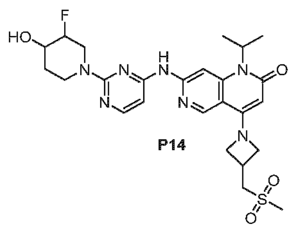 P14 | $^1H$ NMR (400 MHz, MeOD) $\delta$ 8.66 (s, 1H), 7.86 (d, *J* = 7.1 Hz, 1H), 7.76 (s, 1H), 7.11 (s, 1H), 5.18 (s, 1H), 4.69 - 4.37 (m, 4H), 4.27 - 4.17 (m, 1H), 4.16 - 4.09 (m, 2H), 3.98 - 3.77 (m, 2H), 3.61 (dd, *J* = 28.6, 14.2 Hz, 1H), 3.51 (d, *J* = 7.5 Hz, 2H), 3.46-3.28 (m, 3H), 2.92 (s, 3H), 2.56 (s, 1H), 1.87 - 1.80 (m, 2H), 1.52 (dd, *J* = 6.9, 3.7 Hz, 6H). | 546.8 |
| Replace **a2** with: 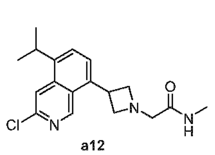 a12 | 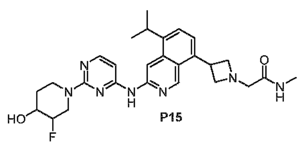 P15 | $^1H$ NMR (400 MHz, MeOD) $\delta$ 9.07 (s, 1H), 8.80 (brs, 1H), 7.86 (d, *J* = 7.2 Hz, 1H), 7.74 (d, *J* = 7.5 Hz, 1H), 7.55 (s, 1H), 6.76 (brs, 1H), 5.02-5.00 (m, 2H), 4.84 - 4.71 (m, 2H), 4.51 (brs, 3H), 4.32 (s, 1H), 4.21-3.94 (m, 2H), 3.69 - 3.65 (m, 2H), 3.57 - 3.39 (m, 1H), 2.77 (s, 3H), 2.04 - 1.89 (m, 2H), 1.40 (t, *J* = 6.5 Hz, 6H). | 508.3 |

(continued)

| Replacement of structure | Target molecules | $^1$H NMR | LC-MS $[M+H]^+$ |
|---|---|---|---|
| Replace **a2** with: 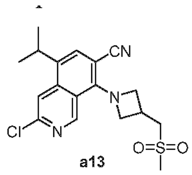 a13 | 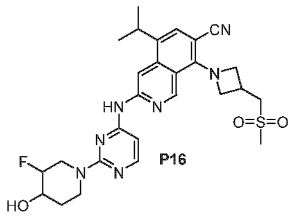 P16 | $^1$H NMR (400 MHz, DMSO-$d_6$) $\delta$ 10.09 (s, 1H), 9.07 (s, 1H), 8.56 (s, 1H), 8.04 (d, *J* = 5.6 Hz, 1H), 7.31 (s, 1H), 6.52 (d, *J* = 5.6 Hz, 1H), 5.13 (d, *J* = 5.0 Hz, 1H), 4.88 (t, *J* = 8.5 Hz, 2H), 4.74 (s, 1H), 4.61 - 4.55 (m, 1H), 4.50 - 4.44 (m, 2H), 4.39 - 4.33 (m, 1H), 3.91 - 3.83 (m, 1H), 3.626-3.61 (m, 2H), 3.58 - 3.49 (m, 1H), 3.41 - 3.34 (m, 2H), 3.01 (s, 3H), 1.75 - 1.68 (m,, 2H), 1.28 (dd, *J* = 6.5, 4.6 Hz, 6H). | 554.2 |
| Replace **a2** with: 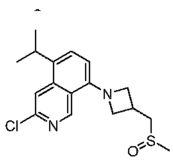 | 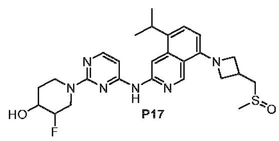 P17 | $^1$H NMR (400 MHz, DMSO-$d_6$) $\delta$ 9.90 (s, 1H), 9.03 (s, 1H), 8.60 (s, 1H), 7.96 (d, *J* = 5.7 Hz, 1H), 7.39 (d, *J* = 8.0 Hz, 1H), 6.57 - 6.29 (m, 2H), 5.12 (s, 1H), 4.77 - 4.59 (m, 1H), 4.59 - 4.52 (m, 1H), 4.35 (t, *J* = 7.0 Hz, 3H), 3.97 - 3.91 (m, 2H), 3.89 - 3.76 (m, 1H), 3.61 - 3.49 (m, 1H), 3.45 (dd, *J* = 13.5, 7.0 Hz, 1H), 3.22 - 3.16 (m, 2H), 3.06 (dd, *J* = 16.1, 10.3 Hz, 1H), 2.56 (s, 3H), 1.76 - 1.67 (m, 2H), 1.62 (dd, *J* = 14.9, 7.7 Hz, 1H), 1.26 (t, *J* = 6.0 Hz, 6H). | 513.2 |
| Replace **a2** with: 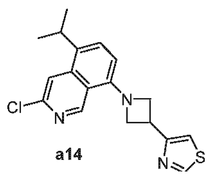 a14 | 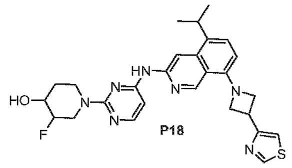 P18 | $^1$H NMR (400 MHz, DMSO-$d_6$) $\delta$ 9.90 (s, 1H), 9.14 - 9.07 (m, 2H), 8.65 (s, 1H), 7.99 (d, *J* = 5.6 Hz, 1H), 7.67 (d, *J* = 1.9 Hz, 1H), 7.43 (d, *J* = 7.9 Hz, 1H), 6.47 (dd, *J* = 11.7, 6.8 Hz, 2H), 5.16 (s, 1H), 4.75 (s, 1H), 4.61 (d, *J* = 10.2 Hz, 4H), 4.39 (m, *J* = 13.5 Hz, 1H), 4.30 - 4.21 (m, 3H), 3.87 (d, *J* = 25.6 Hz, 2H), 3.60 (m, *J* = 11.7 Hz, 1H), 3.53 - 3.49 (m, 1H), 1.72 (d, *J* = 4.8 Hz, 2H), 1.31 (dd, *J* = 6.6, 4.6 Hz, 6H). | 520.2 |
| Replace **a2** with: 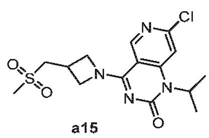 a15 | 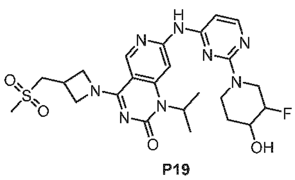 P19 | $^1$H NMR (400 MHz, DMSO-$d_6$) $\delta$ 10.37 (s, 1H), 8.57 (s, 1H), 8.07 (d, *J* = 5.8 Hz, 1H), 7.95 (s, 1H), 6.73 (s, 1H), 4.76 - 4.57 (m, 3H), 4.54 - 4.43 (m, 2H), 4.32 - 4.17 (m, 2H), 3.88-3.74 (m, 2H), 3.58 (d, *J* = 7.5 Hz, 3H), 3.52 - 3.41 (m, 1H), 3.33 - 3.22 (m, 2H), 2.98 (s, 3H), 1.70 - 1.63 (m, 2H), 1.47 (dd, *J* = 6.8, 4.6 Hz, 6H). | 547.0 |

(continued)

| Replacement of structure | Target molecules | $^1H$ NMR | LC-MS $[M+H]^+$ |
|---|---|---|---|
| Replace **a2** with:<br>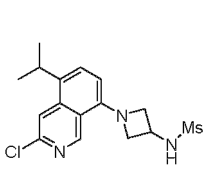<br>a16 | 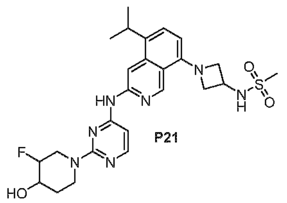<br>P21 | $^1H$ NMR (400 MHz, $CD_3OD$) $\delta$ 9.08 (s, 1H), 8.65 (s, 1H), 8.21 (s, 1H), 7.92 (d, *J* = 5.8 Hz, 1H), 7.45 (d, *J* = 7.9 Hz, 1H), 6.53 (d, *J* = 7.9 Hz, 1H), 6.37 (d, *J* = 5.8 Hz, 1H), 4.78 - 4.60 (m, 2H), 4.48 (dd, *J* = 10.2, 6.4 Hz, 3H), 4.39 (dd, *J* = 9.6, 4.1 Hz, 1H), 4.02 - 3.94 (m, 1H), 3.91 (d, *J* = 4.0 Hz, 2H), 3.64 (dd, *J* = 25.5, 10.8 Hz, 1H), 3.59 - 3.54 (m, 1H), 3.49 - 3.38 (m, 1H), 1.92 (dd, *J* = 19.9, 10.6 Hz, 1H), 1.83 (dd, *J* = 10.2, 6.1 Hz, 1H), 1.34 (t, *J* = 6.1 Hz, 6H). | 530.2 |
| Replace **a2** with:<br>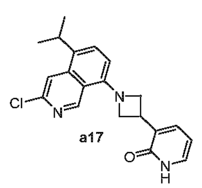<br>a17 | 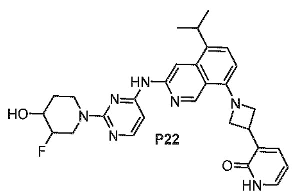<br>P22 | $^1H$ NMR (400 MHz, DMSO-$d_6$) $\delta$ 9.91 (s, 1H), 9.38 (s, 1H), 8.64 (s, 1H), 7.99 (d, *J* = 5.6 Hz, 1H), 7.96 (d, *J* = 5.1 Hz, 1H), 7.77 (d, *J* = *7.0* Hz, 1H), 7.37 (d, *J* = 8.0 Hz, 1H), 6.90 (dd, *J* = 7.2, 5.2 Hz, 2H), 6.58 (d, *J* = 8.2 Hz, 1H), 6.44 (d, *J* = 5.6 Hz, 1H), 5.15 (d, *J* = 4.9 Hz, 1H), 4.75 (s, 1H), 4.75- 4.63 (m, 3H), 4.49 (dd, *J* = 9.2, 5.1 Hz, 1H), 4.41- 4.38 (m, 1H), 4.01- 3.96 (m, 1H), 3.91-3.84 (m, 1H), 3.60 (d, *J* = 13.8 Hz, 1H), 3.49 - 3.45 (m, 2H), 3.61 - 3.35 (m, 2H), 1.72 (d, *J* = 5.2 Hz, 2H), 1.36 - 1.19 (m, 6H). | 529.8 |
| Replace **a2** with:<br>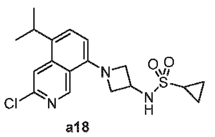<br>a18 | 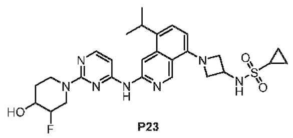<br>P23 | $^1H$ NMR (400 MHz, DMSO-$d_6$) $\delta$ 9.93 (s, 1H), 9.02 (s, 1H), 8.60 (s, 1H), 7.96 (d, *J* = 5.8 Hz, 1H), 7.83 (d, *J* = 9.1 Hz, 1H), 7.40 (d, *J* = 7.9 Hz, 1H), 6.43 (d, *J* = 8.0 Hz, 2H), 5.12 (s, 1H), 4.75 - 4.58 (m, 1H), 4.58 - 4.52 (m, 1H), 4.49 (t, *J* = 7.4 Hz, 2H), 4.37 - 4.27 (m, 2H), 3.90 (t, *J* = 6.8 Hz, 2H), 3.87 - 3.77 (m, 1H), 3.61 - 3.51 (m, 1H), 3.45 (dd, *J* = 13.6, 6.9 Hz, 2H), 2.55 - 2.52 (m, 1H), 1.75 - 1.66 (m, 2H), 1.26 (t, *J* = 5.6 Hz, 6H), 0.99 - 0.94 (m, 2H), 0.93 - 0.88 (m, 2H). | 556.2 |
| Replace **a2** with:<br>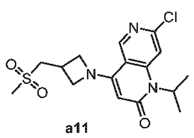<br>a11<br>Replace **b3(cis)** with:<br>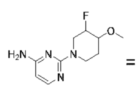 =<br>b9 (cis)<br>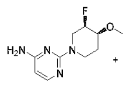 + 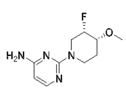 | 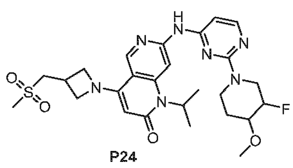<br>P24 | $^1H$ NMR (400 MHz, DMSO-$d_6$) $\delta$ 10.85 (s, 1H), 8.59 (s, 1H), 8.03 (d, *J* = 6.4 Hz, 1H), 7.86 (s, 1H), 7.71-7.57 (m, 1H), 5.13 (s, 1H), 5.01 (d, *J* = 48.9 Hz, 1H), 4.61 - 4.99 (m, 1H), 4.44 (t, *J* = 8.2 Hz, 2H), 4.33 - 4.23 (m, 1H), 4.18 (t, *J* = 6.5 Hz, 1H), 4.07 - 4.00 (m, 2H), 3.82 - 3.75 (m, 3H), 3.55 (d, *J* = 7.4 Hz, 2H), 3.50-3.43 (m, 1H), 3.29 - 3.13 (m, 2H), 2.97 (s, 3H), 1.90 - 1.80 (m, 1H), 1.76 - 1.66 (m, 1H), 1.64 - 1.54 (m, 1H), 1.48 (dd, *J* = 6.2, 4.0 Hz, 6H). | 560.2 |

(continued)

| Replacement of structure | Target molecules | $^1$H NMR | LC-MS [M+H]$^+$ |
|---|---|---|---|
| Replace **a2** with: 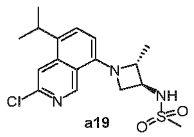 a19 | 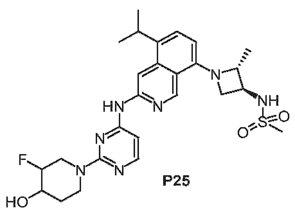 P25 | $^1$H NMR (400 MHz, DMSO-$d_6$) $\delta$ 9.95 (s, 1H), 9.05 (s, 1H), 8.67 (s, 1H), 8.00 (d, $J$ = 5.6 Hz, 1H), 7.78 (d, $J$ = 8.6 Hz, 1H), 7.44 (d, $J$ = 8.0 Hz, 1H), 6.62 (d, $J$ =8.2 Hz, 1H), 6.44 (d, $J$ = 5.6 Hz, 1H), 5.16 (s, 1H), 4.80-4.76 (m, 2H), 4.62-4.60 (m, 2H), 4.40-4.37(m, 1H), 4.17-4.11 (m, 1H), 3.97-3.84 (m, 2H), 3.58-3.47 (m, 3H), 2.96 (s, 3H), 1.77-1.67 (m, 2H), 1.42 (d, $J$ = 6.1 Hz, 3H), 1.35-1.18 (m, 6H). | 544.0 |
| Replace **a2** with: 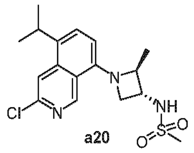 a20 | 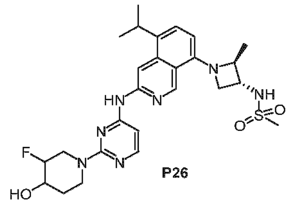 P26 | $^1$H NMR (400 MHz, DMSO-$d_6$) $\delta$ 9.92 (s, 1H), 9.06 (s, 1H), 8.67 (s, 1H), 8.20 (s, 1H), 8.00 (d, $J$ = 5.6 Hz, 1H), 7.76 (d, $J$ = 8.5 Hz, 1H), 7.44 (d, $J$ = 8.0 Hz, 1H), 6.62 (d, $J$ = 8.1 Hz, 1H), 6.45 (d, $J$ = 5.7 Hz, 1H), 5.14 (s, 1H), 4.80-4.74 (m, 1H), 4.59-4.52 (m, 1H), 4.35-4.41 (m, 1H), 4.17 - 4.11 (m, 1H), 3.96-3.88 (m, 2H), 3.64 - 3.57 (m, 1H), 3.57-3.48(m, 3H), 2.96 (s, 3H), 1.76 - 1.70 (m, 2H), 1.42 (d, $J$ = 6.1 Hz, 3H), 1.34 - 1.27 (m, 6H). | 544.0 |
| Replace **a2** with: 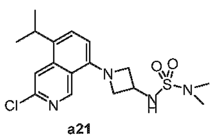 a21 | 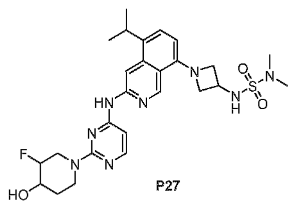 P27 | 1H NMR (400 MHz, DMSO-$d_6$) $\delta$ 9.91 (s, 1H), 9.05 (s, 1H), 8.65 (s, 1H), 8.17 (s, 1H), 8.00 (d, $J$ = 5.6 Hz, 1H), 7.94 (d, $J$ = 8.8 Hz, 1H), 7.42 (d, $J$ = 8.0 Hz, 1H), 6.46 (d, $J$ = 8.0 Hz, 2H), 5.16 (s, 1H), 4.75 (s, 1H), 4.62 (s, 1H), 4.49 (d, $J$= 7.5 Hz, 2H), 4.39 (t, $J$ = 13.9 Hz, 1H), 4.32 - 4.27 (m, 1H), 3.89 -3.84 (m, 1H), 3.59 (d, $J$ = 13.8 Hz, 1H), 3.45 - 3.53 (m, 2H), 2.69 (s, 6H), 1.72 (d, $J$ = 4.7 Hz, 2H), 1.30 (dd, $J$ = 6.7, 4.6 Hz, 6H). | 559.1 |
| Replace **a2** with: 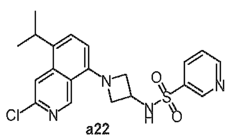 a22 | 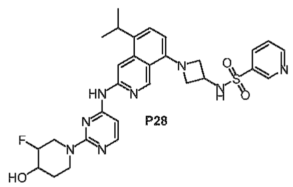 P28 | $^1$H NMR (400 MHz, DMSO-$d_6$) $\delta$ 9.91 (s, 1H), 8.97 (d, $J$= 1.9 Hz, 1H), 8.87 (s, 1H), 8.85 - 8.82 (m, 1H), 8.65 (d, $J$ = 6.6 Hz, 1H), 8.58 (s, 1H), 8.21 (d, $J$ = 8.1 Hz, 1H), 7.95 (d, $J$ = 5.7 Hz, 1H), 7.65 (dd, $J$ = 8.0, 4.9 Hz, 1H), 7.35 (d, $J$ = 8.0 Hz, 1H), 6.41 (d, $J$ = 4.5 Hz, 1H), 6.32 (d, $J$ = 8.0 Hz, 1H), 5.11 (s, 1H), 4.71 (s, 1H), 4.56-4.53 (m, 1H), 4.34-4.27 (m, 5H), 3.83 (d, $J$ = 24.4 Hz, 1H), 3.65 (s, 2H), 3.60 - 3.48 (m, 1H), 3.47 - 3.42 (m, 1H), 1.68 (d, $J$ = 4.5 Hz, 2H), 1.24 (dd, $J$ = 6.3, 5.0 Hz, 6H). | 593.0 |

(continued)

| Replacement of structure | Target molecules | $^1$H NMR | LC-MS $[M+H]^+$ |
|---|---|---|---|
| Replace **a2** with: 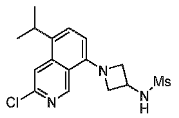 a16 Replace **b3(cis)** with: 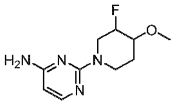 b9 (cis) | 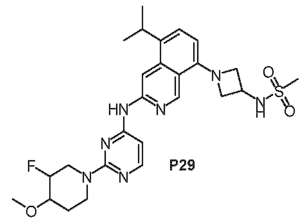 P29 | $^1$H NMR (400 MHz, DMSO-$d_6$) $\delta$ 9.93 (s, 1H), 9.05 (s, 1H), 8.63 (s, 1H), 8.00 (d, $J$ = 5.7 Hz, 1H), 7.84 (d, $J$ = 8.6 Hz, 1H), 7.43 (d, $J$ = 8.0 Hz, 1H), 6.49 -6.44(m, 2H), 5.01 (s, 1H), 4.89 (s, 1H), 4.76-4.70 (m, 1H), 4.57 - 4.47 (m, 3H), 4.39 - 4.34 (m, 1H), 3.93-3.89 (m, 2H), 3.67 - 3.44 (m, 4H), 3.37 (s, 3H), 2.97 (s, 3H), 1.80 - 1.72 (m, 2H), 1.30 (dd, $J$ = 6.7, 4.5 Hz, 6H). | 544.0 |
| Replace **a2** with: 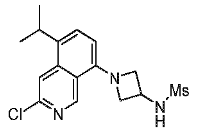 a16 Replace **b3(cis)** with: 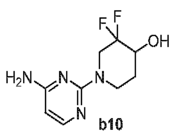 b10 | 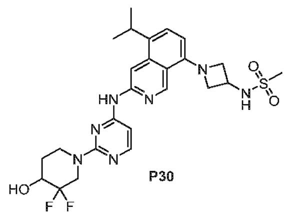 P30 | $^1$H NMR (400 MHz, DMSO-$d_6$) $\delta$ 9.92 (s, 1H), 9.01 (s, 1H), 8.55 (s, 1H), 8.21 (s, 1H), 7.99 (d, $J$ = 5.7 Hz, 1H), 7.79 (d, $J$ = 7.0 Hz, 1H), 7.39 (d, $J$ = 8.0 Hz, 1H), 6.50 (d, $J$ = 5.6 Hz, 1H), 6.42 (d, J = 8.1 Hz, 1H), 5.71 (s, 1H), 4.52-4.46 (m, 2H), 4.37 - 4.24 (m, 2H), 4.10-4.03 (m, 1H), 4.0-3.92 (m, 1H), 3.90-3.85 (m, 2H), 3.75-3.68 (m, 1H), 3.51 - 3.40 (m, 2H), 2.93 (s, 3H), 1.90-1.80 (m, 1H), 1.2-1.68 (m, 1H), 1.25 (d, $J$ = 6.7 Hz, 6H). | 547.9 |
| Replace a2 with: 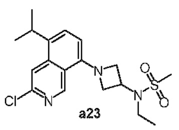 a23 Replace b3(cis) with: 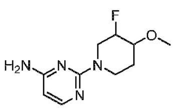 b9 (cis) | 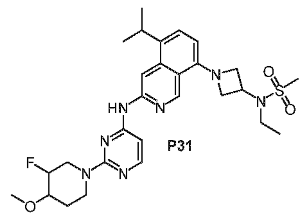 P31 | $^1$H NMR DMSO (400 MHz, DMSO-$d_6$) $\delta$ 10.00 (s, 1H), 9.05 (s, 1H), 8.58 (s, 1H), 7.96 (d, $J$ = 5.9 Hz, 1H), 7.41 (d, $J$ = 8.0 Hz, 1H), 6.47 (m, 2H), 4.93 (d, $J$ = 49.5 Hz, 1H), 4.64 (m, 2H), 4.43 (m, 3H), 4.08 (m, 2H), 3.64 - 3.49 (m, 2H), 3.44 -3.48 (m, 2H), 3.31 - 3.22 (m, 5H), 2.94 (s, 3H), 1.85 - 1.65 (m, 2H), 1.26 (dd, $J$ = 6.7, 4.8 Hz, 6H), 1.17 (t, $J$ = 7.1 Hz, 3H). | 572.1 |
| Replace a2 with: 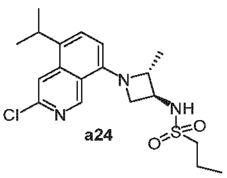 a24 Replace b3(cis) with: 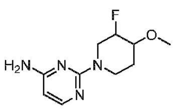 b9 (cis) | 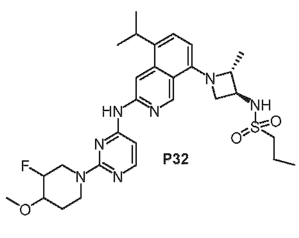 P32 | $^1$H NMR (400 MHz, DMSO-$d_6$) $\delta$ 9.96 (s, 1H), 9.06 (s, 1H), 8.65 (s, 1H), 8.00 (d, J = 5.7 Hz, 1H), 7.80 (d, $J$ = 8.7 Hz, 1H), 7.44 (d, J = 8.0 Hz, 1H), 6.62 (d, $J$ = 8.2 Hz, 1H), 6.47 (d, $J$ = 5.6 Hz, 1H), 5.01 (s, 1H), 4.88 (s, 1H), 4.74-4.79 (m, 2H), 4.49 (m, 1 H), 4.15 - 4.10 (m, 1H), 3.85-3.95 (m, 2 H), 3.60-3.65 (m, 1H), 3.47-3.55(m, 3H), 3.46-3.40 (m, 1H), 3.37 (s, 3H), 3.01 (dd, $J$ = 8.8, 5.6 Hz, 2H), 1.85-1.80 (m, 1H), 1.72-1.65(m, 3H), 1.41 (d, $J$ = 6.1 Hz, 3H), 1.33 - 1.28 (m, 6H), 0.99 (t, $J$ = 7.4 Hz, 3H). | 586.3 |

(continued)

| Replacement of structure | Target molecules | $^1$H NMR | LC-MS [M+H]$^+$ |
|---|---|---|---|
| Replace a2 with: 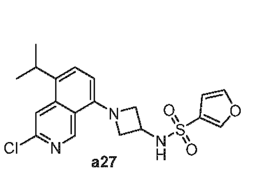 a27 | 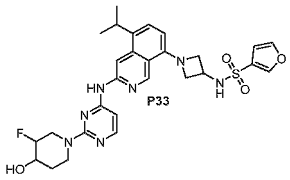 P33 | $^1$H NMR (400 MHz, DMSO-$d_6$) $\delta$ 9.91 (s, 1H), 8.97 (s, 1H), 8.63 (s, 1H), 8.42 (d, $J$ = 8.1 Hz, 1H), 7.99 (d, $J$ = 5.6 Hz, 2H), 7.91 (s, 1H), 7.75 - 7.63 (m, 1H), 7.41(d, $J$ = 7.8 Hz, 1H), 6.80 (s, 1H), 6.53-6.35 (m, 2H), 5.14 (s, 1H), 4.75 (s, 1H), 4.63 - 4.59 (m, 1H), 4.45-4.19 (m, 4H), 3.94 - 3.72 (m, 3H), 3.65 - 3.39 (m, 3H), 1.76 - 1.66 (m, 2H), 1.30 - 1.24 (m, 6H). | 582.0 |
| Replace **a2** with: 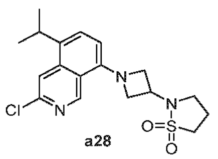 a28 | 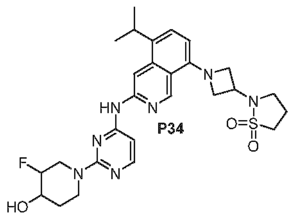 P34 | $^1$H NMR (400 MHz, DMSO-$d_6$) $\delta$ 9.92 (s, 1H), 9.06 (s, 1H), 8.65 (s, 1H), 8.00 (d, $J$ = 5.6 Hz, 1H), 7.42 (d, $J$ = 8.0 Hz, 1H), 6.50 - 6.37 (m, 2H), 5.16 (d, $J$ = 5.1Hz, 1H), 4.74 (s, 1H), 4.62 - 4.64 (m, 1H), 4.38 - 4.30(m, 4H), 4.23 -4.21 (m, 2H), 3.87 (d, $J$ = 19.1 Hz, 2H), 3.58 - 3.47 (m, 2H), 3.43 (t, $J$ = 6.6 Hz, 2H), 3.31 - 3.25 (m, 2H), 2.33 - 2.21 (m, 2H), 1.73 - 1.71 (m, $J$ = 4.7 Hz, 2H), 1.30 (dd, $J$ = 6.7, 4.6 Hz, 6H). | 556.0 |
| Replace **a2** with: 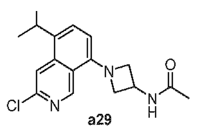 a29 | 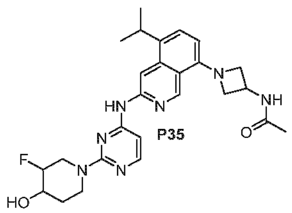 P35 | $^1$H NMR (400 MHz, DMSO-$d_6$) $\delta$ 9.92 (s, 1H), 9.05 (s, 1H), 8.65 (s, 1H), 8.56 (d, $J$ = 7.0 Hz, 1H), 7.99 (d, $J$ = 5.6 Hz, 1H), 7.42 (d, $J$ = 8.0 Hz, 1H), 6.50-6.38(m, 2H), 5.16 (s, 1H), 4.75 (s, 1H), 4.62 - 4.59 (m, 2H), 4.49 -4.33 (m, 3H), 4.07 - 4.01 (m, 1H), 3.93-3.78 (m, 3H), 3.59 (d, $J$ = 13.2 Hz, 1H), 3.54 - 3.39 (m, 3H), 1.85 (s, 3H), 1.75 - 1.65 (m, 2H), 1.30 (dd, $J$ = 6.6, 4.7 Hz, 6H). | 494.1 |
| Replace **a2** with: 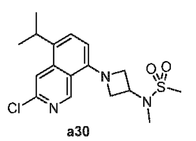 a30 Replace **b3(cis)** with: 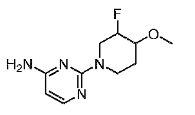 b9 (cis) | 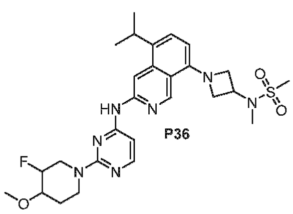 P36 | $^1$H NMR (400 MHz, DMSO-$d_6$) $\delta$ 9.90 (s, 1H), 9.08 (s, 1H), 8.62 (s, 1H), 8.00 (d, $J$ = 5.4 Hz, 1H), 7.43 (d, $J$ = 7.8 Hz, 1H), 6.46 (d, $J$ = 8.0 Hz, 2H), 5.01 (s, 1H), 4.88 (s, 1H), 4.67 - 4.63 (m, 2H), 4.48 - 4.44 (m, 1H), 4.40 - 4.37 (m, 2H), 4.22 - 4.17 (m, 3H), 3.62 - 3.58 (m, 1H), 3.50 - 3.46 (m, 2H), 3.37 (s, 3H), 2.95 (s, 3H), 2.93 (s, 3H), 1.81 - 1.76 (m, 2H), 1.30 - 1.23 (m, 6H). | 558.3 |

(continued)

| Replacement of structure | Target molecules | $^1H$ NMR | LC-MS $[M+H]^+$ |
|---|---|---|---|
| Replace **a2** with: 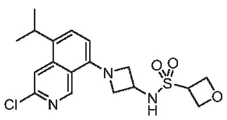 a31 Replace **b3(cis)** with: 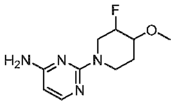 b9 (cis) | 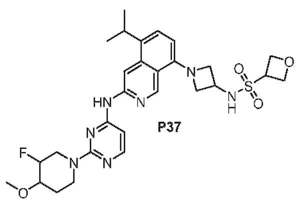 P37 | $^1H$ NMR (400 MHz, DMSO-$d_6$) $\delta$ 9.85 (s, 1H), 8.99 (s, 1H), 8.58 (s, 1H), 8.20 (s, 1H), 7.96 (d, $J$ = 5.7 Hz, 1H), 7.38 (d, $J$ = 8.0 Hz, 1H), 6.43 (dd, $J$ = 15.3, 6.9 Hz, 2H), 4.99 - 4.93 (m, 1H), 4.86 - 4.76 (m, 2H), 4.73 - 4.56 (m, 4H), 4.46 (t, $J$ = 7.5 Hz, 3H), 4.35 (s, 1H), 3.81 (t, $J$ = 6.9 Hz, 2H), 3.63 - 3.37 (m, 3H), 3.33 (s, 3H), 3.24 - 3.22 (m,1H), 1.81 - 1.68 (m, $J$ = 32.1, 14.8, 7.1 Hz, 2H), 1.26 (dd, $J$ = 6.7, 4.3 Hz, 6H). | 586.0 |
| Replace **a2** with: 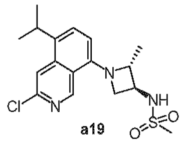 a19 Replace **b3(cis)** with: 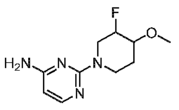 b9 (cis) | 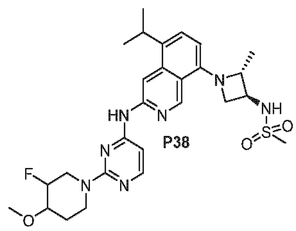 P38 | $^1H$ NMR (400 MHz, DMSO-$d_6$) $\delta$ 9.96 (s, 1H), 9.06 (s, 1H), 8.65 (s, 1H), 8.00 (d, $J$ = 5.5 Hz, 1H), 7.77 (d, $J$ = 8.5 Hz, 1H), 7.44 (d, $J$=7.8 Hz, 1H), 6.62 (d, $J$ = 7.9 Hz, 1H), 6.46 (d, $J$ = 5.3 Hz, 1H), 5.01 (s, 1H), 4.89 (s, 1H), 4.77 - 4.74 (m, 2H), 4.49 (d, $J$ = 12.7 Hz, 1H), 4.17 - 4.10 (m, 1H), 3.95 - 3.90 (m, 1H), 3.62 - 3.49 (m, 4H), 3.37 (s, 3H), 2.96 (s, 3H), 1.85 - 1.72 (m, 2H), 1.42 (d, $J$ = 5.8 Hz, 3H), 1.32 - 1.31 (m, 6H). | 558.1 |
| Replace **a2** with: 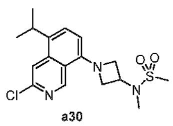 a30 Replace **b3(cis)** with x1: 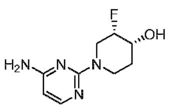 x1 (3s,4r) | 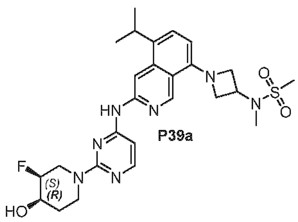 P39a | $^1H$ NMR (400 MHz, DMSO-$d_6$) $\delta$ 9.90 (s, 1H), 9.09 (s, 1H), 8.64 (s, 1H), 8.00 (d, $J$ = 5.8 Hz, 1H), 7.44 (d, $J$ = 8.0 Hz, 1H), 6.48 (d, $J$ = 8.0 Hz, 2H), 5.16 (d, $J$ = 5.1 Hz, 1H), 4.76 (s, 1H), 4.69 - 4.58 (m, 2H), 4.42 - 4.38 (m, 3H), 4.24 - 4.19 (m, 2H), 3.91 - 3.79 (m, 1H), 3.63 - 3.49 (m, 3H), 2.96 (s, 3H), 2.93 (s, 3H), 1.73 (d, $J$ = 4.8 Hz, 2H), 1.30 (dd, $J$ = 6.6, 4.8 Hz, 6H). | 544.2 |
| Replace **a2** with: 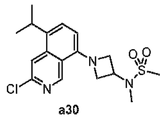 a30 Replace **b3(cis)** with **x2:** 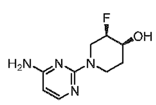 x2 (3r,4s) | 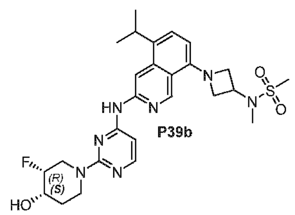 P39b | **The same as above** | 544.2 |

(continued)

| Replacement of structure | Target molecules | $^1H$ NMR | LC-MS $[M+H]^+$ |
|---|---|---|---|
| Replace **a2** with: 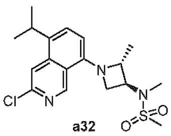 a32 Replace **b3(cis)** with **x3:** 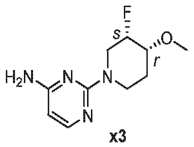 x3 | 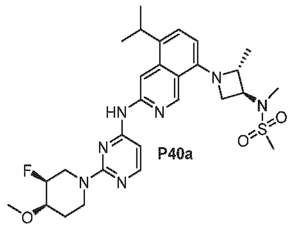 P40a | $^1H$ NMR (400 MHz, DMSO-$d_6$) $\delta$ 9.95 (s, 1H), 9.08 (s, 1H), 8.65 (s, 1H), 8.00 (d, $J$ = 5.7 Hz, 1H), 7.45 (d, $J$ = 8.0 Hz, 1H), 6.66 (d, $J$ = 8.1 Hz, 1H), 6.47 (d, $J$ =5.6 Hz, 1H), 5.00 (s, 1H), 4.88 (s, 1H), 4.74 (s, 1H), 4.64 (t, $J$ = 7.5 Hz, 1H), 4.52-4.41 (m,2H), 4.24-4.18 (m, 1H), 3.81 (t, $J$ = 7.2 Hz, 1H), 3.66-3.49 (m,3H), 3.37 (s, 3H), 3.27-3.25 (m,1H), 2.95 (s, 3H), 2.82 (s, 3H), 1.84-1.81 (m, $J$ = 20.0, 16.6 Hz, 2H), 1.40 (d, $J$ = 6.0 Hz, 3H), 1.37-1.27 (m, 6H). | 572.3 |
| Replace **a2** with: 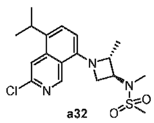 a32 Replace **b3(cis)** with **x4:** 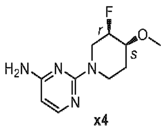 x4 | 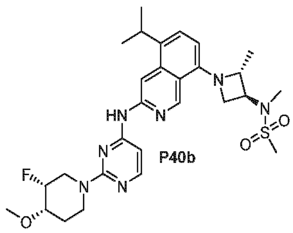 P40b | **The same as above** | 572.3 |
| Replace **a2** with: 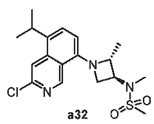 a32 Replace **b3(cis)** with **x1:** 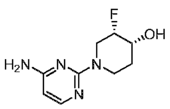 x1 (3s,4r) | 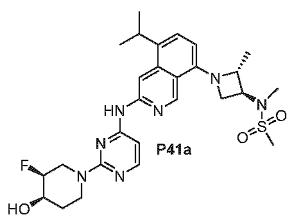 P41a | $^1H$ NMR (400 MHz, DMSO-$d_6$) $\delta$ 9.96(s, 1H), 9.09 (s, 1H), 8.67 (s, 1H), 8.00 (d, $J$ = 5.6 Hz, 1H), 7.46 (d, $J$ = 7.7 Hz, 1H), 6.68 (d, $J$ = 7.5 Hz, 1H), 6.49 (s, 1H), 5.18 (s, 1H), 4.77 (s, 1H), 4.65 - 4.63 (m, 2H), 4.43 - 4.39 (m, 2H), 4.27 - 4.12 (m, 2H), 3.84 - 3.81 (m, 2H), 3.68 - 3.48 (m, 2H), 2.95 (s, 3H), 2.82 (s, 3H), 1.74 - 1.64 (m, 2H), 1.49 - 1.13 (m, 9H). | 558.1 |
| Replace **a2** with: 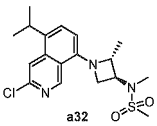 a32 Replace **b3(cis)** with **x2:** 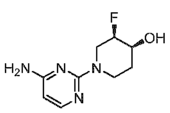 x2 (3r,4s) | 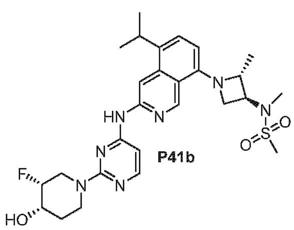 P41b | $^1H$ NMR (400 MHz, DMSO-$d_6$) $\delta$ 9.95 (s, 1H), 9.08 (s, 1H), 8.68 (s, 1H), 8.00 (d, $J$ = 5.7 Hz, 1H), 7.45 (d, $J$ = 8.0 Hz, 1H), 6.66 (d, $J$ = 8.1 Hz, 1H), 6.45 (d, $J$ = 5.7 Hz, 1H), 5.16 (d, $J$ = 5.1 Hz, 1H), 4.77 - 4.61 (m, 3H), 4.45 - 4.36 (m, 2H), 4.24 - 4.20 (m, 1H), 3.94 - 3.84(m, 1H), 3.84 - 3.78 (m, 1H), 3.62 - 3.54 (m, 1H), 3.51 (d, $J$ = 6.2 Hz, 1H), 3.40 - 3.35 (m, 1H), 2.95 (s, 3H), 2.82 (s, 3H), 1.77 - 1.69 (m, 2H), 1.40 (d, $J$ = 6.0 Hz, 3H), 1.31 (dd, $J$ = 11.6, 6.8 Hz, 6H). | 558.1 |

(continued)

| Replacement of structure | Target molecules | $^1H$ NMR | LC-MS $[M+H]^+$ |
|---|---|---|---|
| Replace **a2** with: 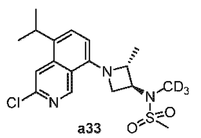 a33 Replace ***b*3(cis)** with: | 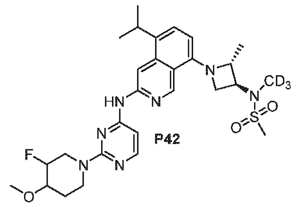 P42 | $^1H$ NMR (400 MHz, DMSO-$d_6$) $\delta$ 10.12 (s, 1H), 9.10 (s, 1H), 8.64 (s, 1H), 8.00 (d, *J* = 5.8 Hz, 1H), 7.46 (d, *J* = 7.9 Hz, 1H), 6.68 (d, *J* = 8.0 Hz, 1H), 6.52 (s, 1H), 4.97 (d, *J* = 48.8 Hz, 1H), 4.77 - 4.68 (m, 1H), | 575.1 |
| 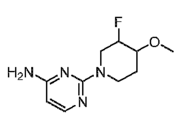 b9 (cis) | | 4.65 (t, *J* = 7.4 Hz, 1H), 4.51 -4.40 (m, 2H), 4.20 (dd, *J* = 13.5, 6.8 Hz, 1H), 3.81 (t, *J* = 7.1 Hz, 1H), 3.67 - 3.59 (m, 1H), 3.59 - 3.53 (m, 1H), 3.53 - 3.42 (m, 2H), 3.37 (s, 3H), 2.95 (s, 3H), 1.89 - 1.81 (m, 1H), 1.78 - 1.70 (m, 1H), 1.40 *(d, J* = 6.0 Hz, 3H), 1.31 (dd, *J* = 11.9, 6.7 Hz, 6H). | |
| Replace **a2** with: 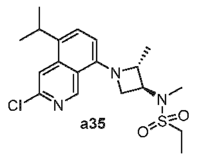 a35 Replace **b3(cis)** with: 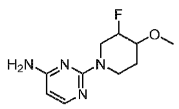 b9 (cis) | 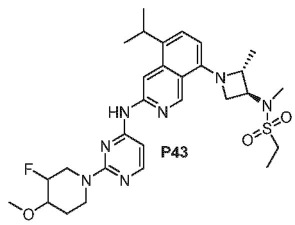 P43 | $^1H$ NMR (400 MHz, DMSO-$d_6$) $\delta$ 9.96 (s, 1H), 9.08 (s, 1H), 8.65 (s, 1H), 8.00 (d, *J* = 5.6 Hz, 1H), 7.44 (d, *J* = 8.0 Hz, 1H), 6.67 (d, *J* = 8.1 Hz, 1H), 6.47 (d, *J* = 5.5 Hz, 1H), 4.95 (dd, *J* = 49.6, 1.3 Hz, 1H), 4.79 - 4.69 (m, 1H), 4.62 (t, *J* = 7.4 Hz, 1H), 4.53 - 4.41 (m, 2H), 4.31 (*q*, *J* = 6.9 Hz, 1H), 3.86 (t, *J* = 7.1 Hz, 1H), 3.67 - 3.57 (m, 1H), 3.57 - 3.48 (m, 2H), 3.35 - 3.24 (m, 4H), 3.12 (q, *J* = 7.3 Hz, 2H), 2.86 (s, 3H), 1.87 - 1.78 (m, 1H), 1.78 - 1.68 (m, 1H), 1.39 (d, *J* = 6.0 Hz, 3H), 1.31 (dd, *J* = 11.0, 6.3 Hz, 6H), 1.21 (t, *J* = 7.3 Hz, 3H). | 585.9 |
| Replace **a2** with: 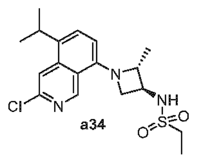 a34 Replace **b3(cis)** with: 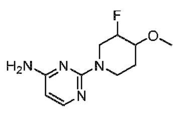 b9 (cis) | 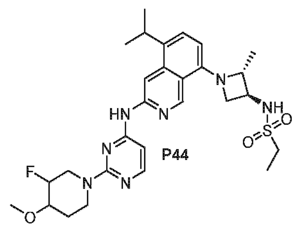 P44 | $^1H$ NMR (400 MHz, DMSO-$d_6$) $\delta$ 10.28 (s, 1H), 9.09 (s, 1H), 8.63 (s, 1H), 8.00 (d, *J* = 6.0 Hz, 1H), 7.81 (d, *J* = 8.7 Hz, 1H), 7.47 (d, *J* = 8.0 Hz, 1H), 6.66 (*d, J* = 8.1 Hz, 1H), 6.55 (s, 1H), 4.99 (d, *J* = 48.6 Hz, 1H), 4.77 (t, *J* = 7.2 Hz, 1H), 4.74 - 4.66 (m, 1H), 4.52 - 4.40 (m, 1H), 4.17 - 4.10 (m, 1H), 3.89 (dt, *J* = 14.3, 7.1 Hz, 1H), 3.68 - 3.59 (m, 1H), 3.59 - 3.42 (m, 4H), 3.37 (s, 3H), 3.04 (q, *J* = 7.3 Hz, 2H), 1.90 - 1.81 (m, 1H), 1.79 - 1.70 (m, 1H), 1.41 (d, *J* = 6.1 Hz, 3H), 1.31 (dd, J = 10.3, 4.7 Hz, 6H), 1.21 (t, *J* = 7.3 Hz, 3H). | 572.2 |

(continued)

| Replacement of structure | Target molecules | $^1$H NMR | LC-MS $[M+H]^+$ |
|---|---|---|---|
| Replace **a2** with: 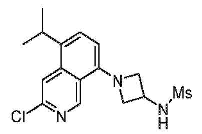 a16 Replace **b3(cis)** with: 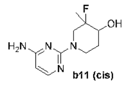 b11 (cis) = 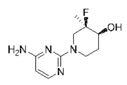 + 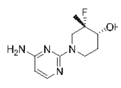 | 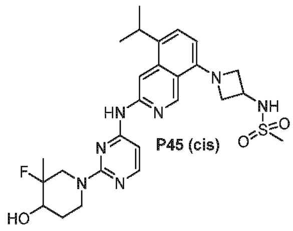 P45 (cis) | $^1$H NMR (400 MHz, DMSO-$d_6$) $\delta$ 10.42 (s, 1H), 9.09 (s, 1H), 8.56 (s, 1H), 7.99 (d, *J* = 6.2 Hz, 1H), 7.84 (d, *J* = 8.5 Hz, 1H), 7.47 (d, *J* = 8.0 Hz, 1H), 6.61 (s, 1H), 6.50 (d, *J* = 8.0 Hz, 1H), 5.12 (s, 1H), 4.62 (d, *J* = 2.6 Hz, 1H), 4.54 (td, *J* = 7.1, 3.3 Hz, 2H), 4.37 (dd, *J* = 14.3, 7.1 Hz, 1H), 3.93 (t, *J* = 5.7 Hz, 2H), 3.65 -3.60 (m, 1H), 3.58 - 3.54 (m, 1H), 3.51 - 3.46 (m, 2H), 3.24 - 3.19 (m, 1H), 2.97 (s, 3H), 1.83 - 1.71 (m, 2H), 1.38 (d, *J* = 21.3 Hz, 3H), 1.30 (dd, *J* = 6.4, 4.8 Hz, 6H). | 543.8 |
| Replace **a2** with: 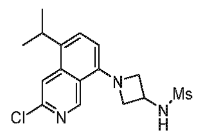 a16 Replace **b3(cis)** with: 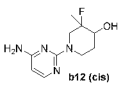 b12 (cis) = 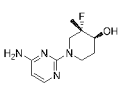 + 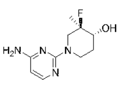 | 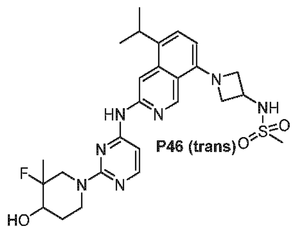 P46 (trans) | $^1$H NMR (400 MHz, DMSO-$d_6$) $\delta$ 9.90 (s, 1H), 9.04 (s, 1H), 8.63 (s, 1H), 8.31 (s, 1H), 7.99 (d, *J* = 5.6 Hz, 1H), 7.85 (d, *J* = 8.6 Hz, 1H), 7.43 (d, J = 8.0 Hz, 1H), 6.46 (t, J = 6.2 Hz, 2H), 5.36 (s, 1H), 4.53 (t, J = 7.2 Hz, 2H), 4.36 (dd, J = 14.5, 7.1 Hz, 1H), 3.97 - 3.86 (m, 5H), 3.80 - 3.69 (m, 1H), 3.58 - 3.44 (m, 2H), 2.97 (s, 3H), 1.99 - 1.83 (m, 1H), 1.54 (td, J = 11.9, 6.1 Hz, 1H), 1.38 - 1.17 (m, 9H). | 543.8 |
| Replace **a2** with: 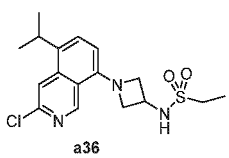 a36 | 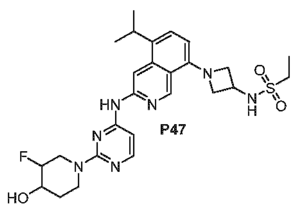 P47 | $^1$H NMR (400 MHz, DMSO-$d_6$) $\delta$ 10.15 (s, 1H), 9.06 (s, 1H), 8.63 (s, 1H), 8.00 (d, *J* = 5.9 Hz, 1H), 7.86 (d, *J* = 8.6 Hz, 1H), 7.45 (d, *J* = 8.0 Hz, 1H), 6.53 (s, 1H), 6.48 (d, *J* = 8.0 Hz, 1H), 5.18 (s, 1H), 4.72 (d, *J* = 50.7 Hz, 1H), 4.63 - 4.55 (m, 1H), 4.52 (t, *J* = 7.4 Hz, 2H), 4.40 - 4.30 (m, 2H), 3.95 - 3.83 (m, 3H), 3.67 - 3.57 (m, 1H), 3.56 - 3.44 (m, 2H), 3.05 (dd, *J* = 14.6, 7.3 Hz, 2H), 1.78 - 1.70 (m, 2H), 1.30 (dd, *J* = 6.0, 5.4 Hz, 6H), 1.22 (t, *J* = 7.3 Hz, 3H). | 544.2 |
| Replace **a2** with: 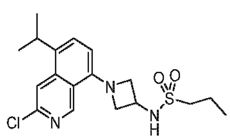 a37 | 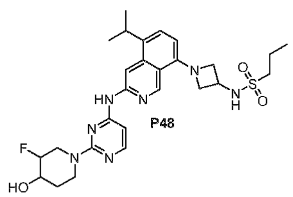 P48 | $^1$H NMR (400 MHz, DMSO-$d_6$) $\delta$ 9.93 (s, 1H), 9.04 (s, 1H), 8.65 (s, 1H), 8.14 (s, 0.2H), 8.00 (d, *J* = 5.7 Hz, 1H), 7.87 (d, *J* = 8.7 Hz, 1H), 7.43 (d, *J* = 8.0 Hz, 1H), 6.50 - 6.42 (m, 2H), 5.16 (d, *J* = 4.6 Hz, 1H), 4.77 - 4.54 (m, 1H), 4.63 - 4.57 (m, 1H), 4.51 (t, *J* = 7.4 Hz, 2H), 4.41 - 4.31 (m, 2H), 3.92 - 3.82 (m, 3H), 3.64 - 3.56 (m, 1H), 3.56 - 3.45 (m, 2H), 3.05 - 2.99 (m, 2H), 1.76 - 1.64 (m, 4H), 1.30 (dd, *J* = 6.5, 4.8 Hz, 6H), 1.00 (t, *J* = 7.4 Hz, 3H). | 557.9 |

(continued)

| Replacement of structure | Target molecules | $^1$H NMR | LC-MS [M+H]$^+$ |
|---|---|---|---|
| Replace **a2** with: 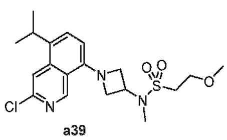 a39 Replace **b3(cis)** with: 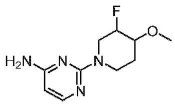 b9 (cis) | 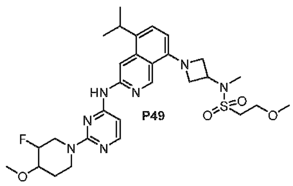 P49 | $^1$H NMR (400 MHz, DMSO-$d_6$) $\delta$ 9.92 (s, 1H), 9.08 (s, 1H), 8.63 (s, 1H), 8.00 (d, $J$ = 5.6 Hz, 1H), 7.43 (d, $J$ = 8.0 Hz, 1H), 6.54 - 6.40 (m, 2H), 4.94 (d, $J$ = 51.4 Hz, 1H), 4.79 - 4.71 (m, 2H), 4.50 (d, $J$ = 12.9 Hz, 1H), 4.40 - 4.35 (m, 2H), 4.27 - 4.20 (m, 2H), 3.68 - 3.64 (m, 2H), 3.564 - 3.55 (m, 1H), 3.54 - 3.42 (m, 2H), 3.42 - 3.37 (m, 2H), 3.37 (s, 3H), 3.36 - 3.34 (m,1H), 3.30 (s,3H), 2.96 (s, 3H), 1.86 - 1.78 (m, 1H), 1.74 - 1.66 (m, 1H), 1.38 - 1.16 (m, 6H). | 602.0 |
| Replace **a2** with: 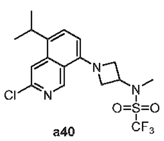 a40 Replace **b3(cis)** with: 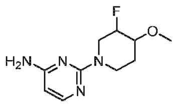 b9 (cis) | 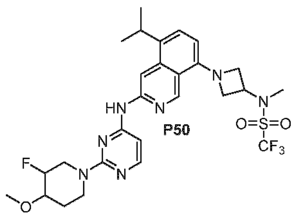 P50 | $^1$H NMR (400 MHz, DMSO-$d_6$) $\delta$ 9.91 (s, 1H), 9.09 (s, 1H), 8.63 (s, 1H), 8.39 (s, 1H), 8.01 (d, $J$ = 5.6 Hz, 1H), 7.43 (d, $J$ = 8.0 Hz, 1H), 6.55 - 6.39 (m, 2H), 5.03 - 4.87 (m, 2H), 4.73 - 4.70 (m, 1H), 4.53 - 4.47 (m, 1H), 4.43 (d, $J$ = 6.2 Hz, 4H), 3.66 - 3.57 (m, 1H), 3.54 - 3.41 (m, 3H), 3.37 (s, 3H), 3.30 - 3.22 (m, 3H), 1.85 - 1.78 (m, 1H), 1.74 - 1.66 (m, 1H), 1.40 - 1.18 (m, 6H). | 612.2 |
| Replace **a2** with: 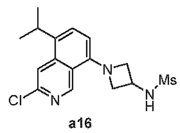 a16 Replace **b3(cis)** with: 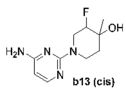 b13 (cis) = 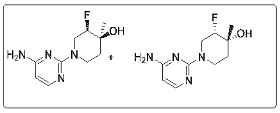 | 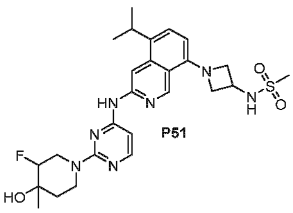 P51 | $^1$H NMR (400 MHz, DMSO-$d_6$) $\delta$ 9.93 (s, 1H), 9.04 (s, 1H), 8.65 (s, 1H), 8.33 (s, 1H), 8.01 (d, J = 5.7 Hz, 1H), 7.84 (d, J = 8.7 Hz, 1H), 7.42 (d, J = 8.0 Hz, 1H), 6.46 (dd, J = 12.0, 6.7 Hz, 2H), 4.87 (s, 1H), 4.52 (t, J = 8.1 Hz, 2H), 4.48 - 4.25 (m, 3H), 4.21 - 4.17 (m, 1H), 3.91 (t, J = 8.1 Hz, 2H), 3.64 - 3.59 (m, 1H), 3.52 -3.46 (m, 2H), 2.97 (s, 3H), 1.7 2 - 1.68 (m, 1H), 1.60 - 1.56 (m, 1H), 1.33 - 1.26 (m, 6H), 1.26 (s, 3H). | 544.2 |

**Example 3**

**Preparation of target molecule P6**

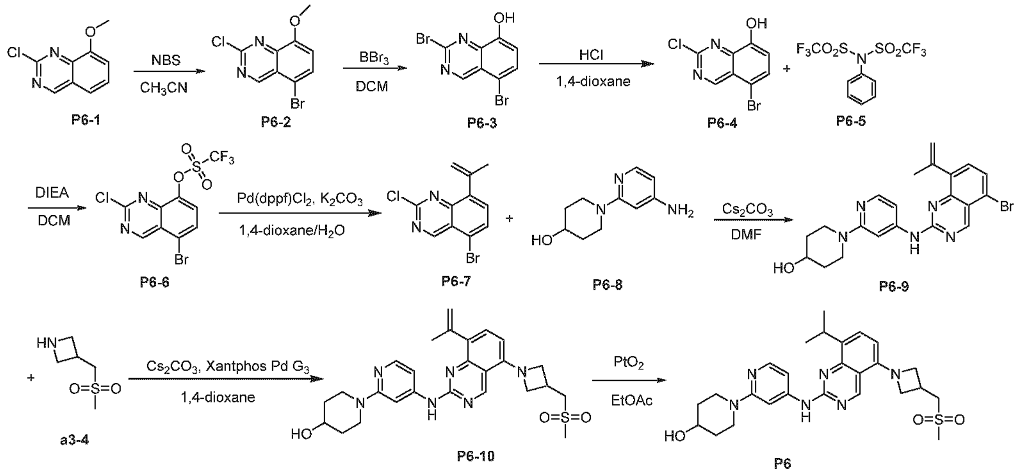

P6-1 P6-2 P6-3 P6-4 P6-5 P6-6 P6-7 P6-8 P6-9 a3-4 P6-10 P6

2-Chloro-8-methoxyquinazoline **P6-1** (2.0 g, 10.3 mmol) was dissolved in 50 mL of acetonitrile, and N- bromosuccinimide (3.67 g, 20.6 mmol) was added. The mixture was stirred at room temperature for 2 hours. The reaction was completed by LC-MS monitoring. The solvent was removed by evaporation under reduced pressure, and 100 mL of water was added to the system. The mixture was extracted with ethyl acetate, dried over anhydrous sodium sulfate, and filtered. The filtrate was concentrated under reduced pressure, and separated by column chromatography (PE/EA=1/10) to obtain 5-bromo-2-chloro-8-methoxyquinazoline P6-2 (2.85 g), yield: 80%, LCMS: ESI-MS (m/z): 272.9 $[M + H]^+$.

$^1H$ NMR (400 MHz, $CDCl_3$) $\delta$ 9.54 (s, 1H), 7.78 (d, *J*= 8.4 Hz, 1H), 7.15 (d, *J*= 8.4 Hz, 1H), 4.08 (s, 3H).

The intermediate **P6-2** (2.0 g, 10.3 mmol) from the previous step was dissolved in 30 mL of dichloromethane, and boron tribromide (2.74 g, 10.9 mmol) was added. The mixture was reacted at room temperature for 12 hours. The reaction was completed by LC-MS monitoring. Aqueous saturated sodium bicarbonate solution was slowly added to the reaction solution to quench the reaction. The mixture was extracted with dichloromethane, dried over anhydrous sodium sulfate, and filtered. The filtrate was concentrated under reduced pressure, and separated by column chromatography (DCM/MeOH=20/1) to obtain 2,5-dibromo-8-hydroxyquinazoline **P6-3** (2.8 g), yield: 81%, LCMS: ESI-MS (m/z): 302.8 $[M + H]^+$.

The intermediate **P6-3** (2.8 g, 9.2 mmol) from the previous step was dissolved in 46 mL of a solution of 1M hydrogen chloride in 1,4-dioxane and the mixture was reacted at room temperature for 1 hour. The reaction was completed by LC-MS monitoring. The reaction solution was concentrated under reduced pressure, and aqueous saturated sodium bicarbonate solution was slowly added to the reaction solution to adjust the pH to about 8. The solution was extracted with dichloromethane, dried over anhydrous sodium sulfate, and filtered. The filtrate was concentrated under reduced pressure to obtain a crude product **P6-4** (2.7 g), LCMS: ESI-MS (m/z): 258.8 [M + H]+.

The crude product **P6-4** (2.7 g) from the previous step and DIEA **(2.7** g, 20.8 mmol) were dissolved in 30 mL of dichloromethane, and N-phenylbistrifluoromethylsulfonamide **P6-5** (5.57 g, 15.6 mmol) was added. The mixture was allowed to react at room temperature for 12 hours. The reaction was completed by LC-MS monitoring. The reaction solution was concentrated under reduced pressure. 100 mL of water was added to the reaction solution. The mixture was extracted with dichloromethane, dried over anhydrous sodium sulfate, and filtered. The filtrate was concentrated under reduced pressure, and separated by column chromatography (PE/EA=1/1) to obtain compound **P6-6** (3.2 g), yield: 71%, LCMS: ESI-MS (m/z): 392.8 $[M + H]^+$.

Under nitrogen protection, the intermediate **P6-6** (1.0 g, 2.6 mmol) from the previous step, $K_2CO_3$ (706 mg, 5.2 mmol) and isopropenylboronic acid pinacol ester **a1-3** (515 mg, 3.1 mmol) were dissolved in 11 mL of a mixed solution of 1,4-dioxane and water (v/v=10/1). Pd(dppf)$Cl_2$ (187 mg, 0.25 mmol) was added. The mixture was heated to 45°C for 2 hours. The reaction was completed by LC-MS monitoring. The mixture was filtered. The filtrate was concentrated, and separated by column chromatography (PE/EA=10/1) to obtain a brown oil **P6-7** (100 mg), yield: 8%, LCMS: ESI-MS (m/z): 282.8 $[M + H]^+$.

The intermediate **P6-7** (100 mg, 0.21 mmol) from the previous step and $Cs_2CO_3$ (649 mg, 0.63 mmol) were dissolved in 5 mL of DMF. 1-(4-aminopyridin-2-yl)piperidin-4-ol **P6-8** (49 mg, 0.25 mmol) was added. The mixture was heated to 110°C and reacted for 12 hours. The reaction was completed by LC-MS monitoring. After cooling to room temperature, 50 mL of water was added to the reaction solution. The mixture was extracted with dichloromethane, dried over anhydrous sodium sulfate, and filtered. The filtrate was concentrated under reduced pressure, and separated by column chromatography (DCM/MeOH=20/1) to obtain compound **P6-9** (40 mg), yield: 39%, LCMS: ESI-MS (m/z): 439.7 $[M + H]^+$.

Under nitrogen protection, the intermediate **P6-9** (40 mg, 0.091 mmol) from the previous step, $Cs_2CO_3$ (89 mg,

0.27 mmol) and intermediate **a3-4** (36 mg, 0.14 mmol) were dissolved in 2 mL of 1,4-dioxane. XantPhos PdG3 (17 mg, 0.018 mmol) was added. The mixture was heated to 100°C and reacted for 6 hours. The reaction was completed by LC-MS monitoring. The mixture was filtered. The filtrate was concentrated, and separated by HPLC preparative chromatography to obtain a yellow solid **P6-10** (30 mg), yield: 59%, LCMS: ESI-MS (m/z): 509.1 $[M + H]^+$.

The intermediate **P6-10** (20 mg, 0.04 mmol) from the previous step was dissolved in 5 mL of ethyl acetate, and platinum dioxide (45 mg, 0.19 mmol) was added. The mixture was reacted at room temperature for 6 hours under a hydrogen atmosphere. The reaction solution was filtered. The filtrate was concentrated, and separated by HPLC preparative chromatography to obtain the target compound **P6** (1.0 mg), yield: 5%, LC-MS: ESI-MS (m/z): 510.9 $[M + H]^+$.

## Example 4

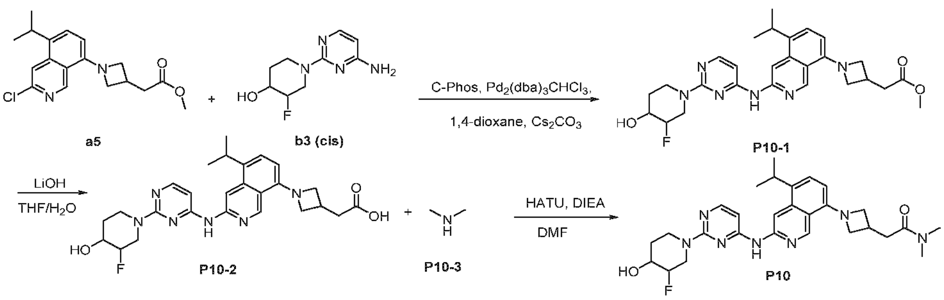


a5 + b3 (cis) → P10-1 → P10-2 + P10-3 → P10

Under nitrogen protection, intermediate **a5** (100 mg, 0.30 mmol), $Cs_2CO_3$ (196 mg, 0.60 mmol) and intermediate **b3 cis** (64 mg, 0.30 mmol) were mixed in 3 mL of 1,4-dioxane. $Pd_2(dba)_3CHCl_3$ (31 mg, 0.03 mmol) was added. The mixture was heated to 100°C and reacted for 16 hours. The reaction was completed by LC-MS monitoring. The mixture was filtered. The filtrate was concentrated, and separated by flash column chromatography (DCM/MeOH=10/1) to obtain a yellow solid **P10-1** (100 mg), yield: 59%, LCMS: ESI-MS (m/z): 508.9 $[M + H]^+$.

The intermediate **P10-1** (100 mg, 0.60 mmol) from the previous step was dissolved in 1 mL of tetrahydrofuran, and 3 N aqueous LiOH solution (0.5 mL) was added. The mixture was reacted at room temperature for 3 hours. The reaction was stopped. The reaction solution was concentrated under reduced pressure. Acetic acid was added to the system to adjust the pH to about 6. 10 mL of water was added. The mixture was extracted with ethyl acetate and concentrated under reduced pressure to obtain crude product **P10-2** (50 mg), yield: 23%, LCMS: ESI-MS (m/z): 495.1 $[M + H]^+$.

The crude product **P10-1** (10 mg, 0.039 mmol) and a solution of dimethylamine in tetrahydrofuran (1 mL, 1M) were dissolved in 1 mL of DMF. DIEA (16 mg, 0.12 mmol) and HATU (3.0 mg, 0.06 mmol) were added. The mixture was stirred at room temperature for 16 hours. 5 mL of water was added to the system. The mixture was extracted with ethyl acetate, concentrated under reduced pressure, and separated by thin layer chromatography to obtain compound P10 (1.5 mg), yield: 24%. [cis configuration]

$^1H$ NMR (400 MHz, DMSO-$d_6$) $\delta$ 9.08 (s, 1H), 8.41 (s, 1H), 7.92 (d, $J$ = 8.0 Hz, 1H), 7.42 (d, $J$ = 8.0 Hz, 1H), 6.39 (d, $J$ = 8.2 Hz, 1H), 6.33 (d, $J$ = 6.3 Hz, 1H), 4.86 (s, 1H), 4.66 - 4.56 (m, 1H), 4.32 - 4.40 (m, 3H), 3.89 - 4.00 (m, 1H), 3.85-3.89 (m, 2H), 3.57 -3.65(m, 2H), 3.45-3.50 (m, 1H), 3.10 (s, 3H), 3.09-3.13 (m, 3H), 2.89 (s, 3H), 2.60(d, $J$ = 6.0 Hz, 2H), 1.84-1.90(m, 1H), 1.80-1.82(m, 1H), 1.33 (dd, $J$= 6.7, 4.5 Hz, 6H).

Referring to the synthetic route of compound **P10, the** following target molecule was synthesized using a similar skeleton structure (unless otherwise stated, the compound is in cis configuration).

| Replacement of structure | Target molecule | $^1$H NMR | LC-MS $[M+H]^+$ |
|---|---|---|---|
| Replace **P10**-3 with: **$MeNH_2$ (HCl)** | 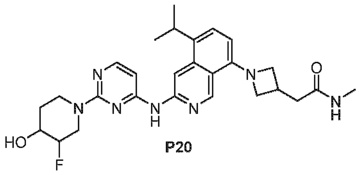 P20 | $^1$H NMR (400 MHz, $CD_3OD$) $\delta$ 9.06 (s, 1H), 8.64 (s, 1H), 7.93 (d, $J$ = 5.9 Hz, 1H), 7.43 (d, $J$ = 8.0 Hz, 1H), 6.46 (d, $J$ = 8.0 Hz, 1H), 6.34 (d, $J$ = 5.8 Hz, 1H), 4.78 - 4.58 (m, 2H), 4.45 - 4.35 (m, 1H), 4.29 (t, $J$ = 7.5 Hz, 2H), 3.96 (dd, $J$ = 22.7, 7.2 Hz, 1H), 3.83 (t, $J$ = 6.5 Hz, 2H), 3.70 - 3.52 (m, 2H), 3.43 (t, $J$ = 9.1 Hz, 1H), 3.09 (dt, $J$ = 13.5, 6.8 Hz, 1H), 2.71 (s, 3H), 2.60 (d, $J$ = 7.7 Hz, 2H), 1.94 - 1.78 (m, 2H), 1.35 - 1.31 (m, 6H). | 508.2 |

## Example 5

### Preparation of target molecules H1-H2, H4-H5, H12, H23, H25, and H33-H34

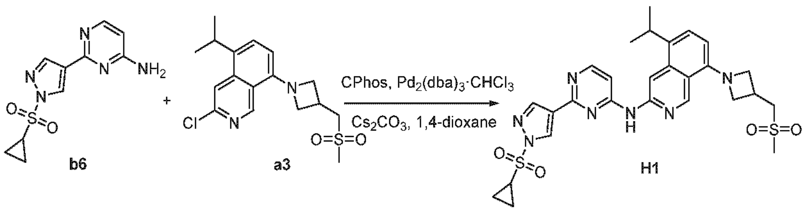

b6 a3 H1

Under nitrogen protection, intermediate **a3** (35 mg, 0.10 mmol), $Cs_2CO_3$ (48 mg, 0.15 mmol) and intermediate **b6** (32 mg, 0.12 mmol) were mixed in 3 mL of 1,4-dioxane. $Pd_2(dba)_3CHCl_3$ (10 mg, 0.01 mmol) and C-phos (4.5 mg, 0.01 mmol) were added. The mixture was heated to 100°C and reacted for 12 hours. The reaction was completed by LC-MS monitoring. The mixture was filtered. The filtrate was concentrated, and separated by HPLC preparative chromatography to obtain a yellow solid **H1** (2.7 mg), yield: 5%, LCMS: ESI-MS (m/z): 582.1 $[M + H]^+$.

$^1$H NMR (400 MHz, DMSO-$d_6$) $\delta$ 10.37 (d, $J$ = 8.4 Hz, 1H), 9.10 (s, 1H), 8.77 (s, 1H), 8.70 (s, 1H), 8.51 (s, 1H), 8.43 (d, $J$ = 5.9 Hz, 1H), 7.45 (d, $J$ = 8.0 Hz, 1H), 7.25 (s, 1H), 6.45 (d, $J$ = 8.1 Hz, 1H), 4.41 (t, $J$ = 7.7 Hz, 2H), 3.98 (t, $J$ = 6.9 Hz, 2H), 3.64 - 3.52 (m, 3H), 3.31 - 3.23 (m, 2H), 3.02 (s, 3H), 1.37 (d, $J$ = 6.8 Hz, 6H), 1.30 (ddd, $J$ = 21.4, 16.3, 9.3 Hz, 4H).

Referring to the synthetic route of compound H1, the following target molecules were synthesized using a similar skeleton structure.

| Replacement of structure | Target molecule | $^1$H NMR | LC-MS $[M+H]^+$ |
|---|---|---|---|
| Replace intermediate **b6** with: 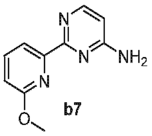 b7 | 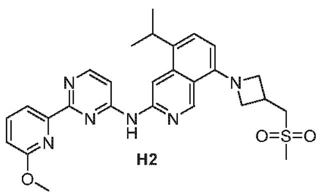 H2 | $^1$H NMR (400 MHz, DMSO-$d_6$) $\delta$ 10.38 (s, 1H), 9.10 (s, 1H), 8.86 (s, 1H), 8.56 (d, $J$ = 5.8 Hz, 1H), 8.28 (s, 1H), 8.00 (d, $J$ = 6.7 Hz, 1H), 7.93 - 7.87 (m, 1H), 7.44 - 7.32 (m, 2H), 7.00 (d, $J$ = 7.5 Hz, 1H), 6.42 (d, $J$ = 8.0 Hz, 1H), 4.41 (t, $J$ = 7.7 Hz, 2H), 4.08 - 3.89 (m, 5H), 3.58 (m, 3H), 3.28 - 3.24 (m, 1H), 3.02 (s, 3H), 1.28 (d, $J$ = 6.8 Hz, 6H). | 519.2 |

(continued)

| Replacement of structure | Target molecule | $^1$H NMR | LC-MS [M+H]+ |
|---|---|---|---|
| Replace intermediate **a3** with: 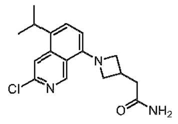 a6 | 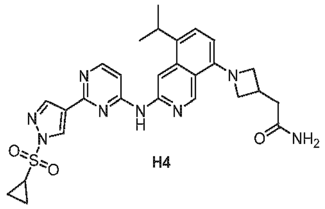 H4 | $^1$H NMR (400 MHz, DMSO-$d_6$) $\delta$ 10.37 (s, 1H), 9.11 (s, 1H), 8.76 (s, 1H), 8.69 (s, 1H), 8.51 (s, 1H), 8.43 (d, $J$ = 5.9 Hz, 1H), 7.43 (d, $J$ = 8.0 Hz, 1H), 7.38 (s, 1H), 7.23 (s, 1H), 6.86 (s, 1H), 6.41 (d, $J$ = 8.1 Hz, 1H), 4.32 (t, $J$ = 7.6 Hz, 2H), 3.84 (t, $J$ = 6.6 Hz, 2H), 3.61 - 3.50 (m, 1H), 3.31 - 3.26 (m, 1H), 3.07 - 2.98 (m, 1H), 2.49 - 2.47 (m, 2H), 1.37 (d, $J$ = 6.8 Hz, 6H), 1.34 - 1.21 (m, 4H). | 547.0 |
| Replace intermediate **a3** with: 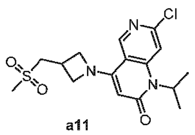 a11 | 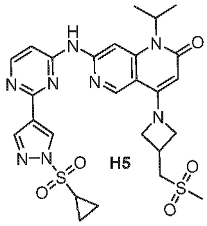 H5 | $^1$H NMR (400 MHz, DMSO-$d_6$) $\delta$ 10.43 (s, 1H), 8.63 (d, $J$ = 8.5 Hz, 1H), 8.57 (s, 1H), 8.47 (d, $J$ = 5.9 Hz, 1H), 8.42 (s, 1H), 7.94 (s, 1H), 7.67 (s, 1H), 5.43 - 5.18 (m, 1H), 5.09 (s, 1H), 4.44 (t, $J$ = 8.3 Hz, 2H), 4.05 (dd, $J$ = 8.1, 6.4 Hz, 2H), 3.55 (d, $J$ = 7.4 Hz, 2H), 3.27 - 3.14 (m, 2H), 2.97 (s, 3H), 1.51 (d, $J$ = 7.1 Hz, 6H), 1.35 - 1.28 (m, 2H), 1.25 - 1.20 (m, 2H). | 598.8 |
| Replace intermediate a3 with: 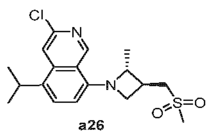 a26 | 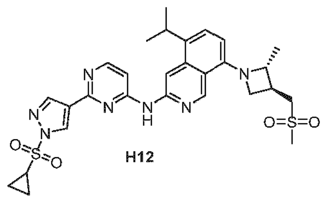 H12 | $^1$H NMR (400 MHz, DMSO-$d_6$) $\delta$ 10.42 (s, 1H), 9.11 (s, 1H), 8.80 (s, 1H), 8.70 (s, 1H), 8.52 (s, 1H), 8.43 (d, $J$ = 5.9 Hz, 1H), 8.38 (s, 1H), 7.46 (d, $J$ = 8.0 Hz, 1H), 7.23 (s, 1H), 6.60 (d, $J$ = 8.1 Hz, 1H), 4.69 (t, $J$ = 7.4 Hz, 1H), 4.24 - 4.17 (m, 1H), 3.66 (t, $J$ = 7.1 Hz, 1H), 3.62 - 3.47 (m, 4H), 3.00 (s, 3H), 2.90 (dd, $J$ = 14.1, 7.2 Hz, 1H), 1.43 (d, $J$ = 6.0 Hz, 3H), 1.38 (dd, $J$ = 6.6, 2.4 Hz, 6H), 1.35 - 1.28 (m, 2H), 1.28 - 1.23 (m, 2H). | 596.2 |
| Replace intermediate **a3** with: 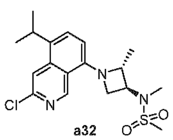 a32 | 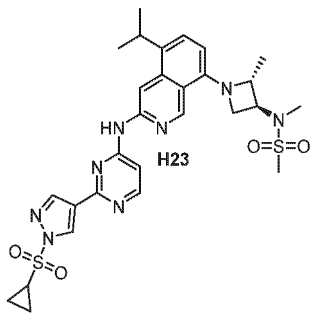 H23 | $^1$H NMR (400 MHz, DMSO-$d_6$) $\delta$ 10.42 (s, 1H), 9.13 (s, 1H), 8.81 (s, 1H), 8.70 (s, 1H), 8.52 (s, 1H), 8.44 (d, $J$ = 5.9 Hz, 1H), 7.48 (d, $J$ = 8.0 Hz, 1H), 7.24 (s,lH), 6.70 (d, $J$ = 8.1 Hz, 1H), 4.66 (t, $J$ = 7.3 Hz, 1H), 4.48 - 4.43 (m, 1H), 4.24 - 4.19 (m, 1H), 3.83 (t, $J$ = 7.1 Hz, 1H), 3.62 - 3.56 (m, 1H), 3.29 - 3.27(m,1H), 2.96 (s, 3H), 2.83 (s, 3H), 1.42 -1.35 (m, 9H), 1.34 - 1.16 (m, 4H). | 611.3 |
| Replace intermediate **a3** with: 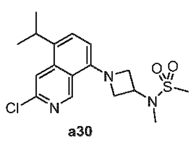 a30 | 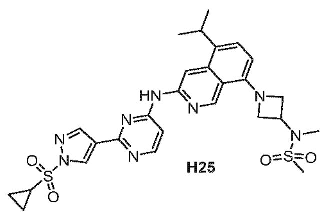 H25 | $^1$H NMR (400 MHz, DMSO-$d_6$) $\delta$ 10.37 (s, 1H), 9.14 (s, 1H), 8.76 (s, 1H), 8.69 (s, 1H), 8.51 (s, 1H), 8.44 (d, $J$ = 5.9 Hz, 1H), 8.40 (s, 1H), 7.46 (d, $J$ = 8.0 Hz, 1H), 7.26 (d, $J$ = 4.7 Hz, 1H), 6.50 (d, $J$ = 8.0 Hz, 1H), 4.71 - 4.64 (m, 1H), 4.42 (t, $J$ = 7.9 Hz, 2H), 4.26 - 4.21 (m, 2H), 3.60 - 3.56 (m, 1H), 3.27 - 3.26 (m, 1H), 2.96 (s, 3H), 2.94 (s, 3H), 1.40 - 1.35 (m, 6H), 1.34 - 1.25 (m, 4H). | 597.0 |

(continued)

| Replacement of structure | Target molecule | $^1$H NMR | LC-MS [M+H]+ |
|---|---|---|---|
| Replace intermediate **a3** with: 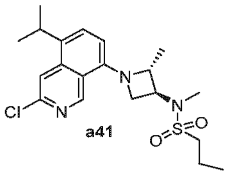 a41 | 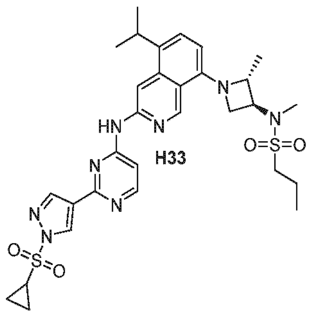 H33 | $^1$H NMR (400 MHz, DMSO-$d_6$) $\delta$ 10.36 (s, 1H), 9.09 (s, 1H), 8.68 (s, 1H), 8.56 (s, 1H), 8.47 (s, 1H), 8.40 (d, $J$ = 5.9 Hz, 1H), 7.35 (d, $J$ = 7.8 Hz, 1H), 7.25 (s, 1H), 6.60 (d, $J$ = 8.0 Hz, 1H), 4.61 (t, $J$ = 7.5 Hz, 1H), 4.43 (t, $J$ = 6.1 Hz, 1H), 4.28 - 4.23 (m, 1H), 3.82 (t, $J$ = 7.2 Hz, 1H), 3.25 - 3.20 (m, 2H), 3.06 - 3.01 (m, 2H), 2.91 (t, $J$ = 7.4 Hz, 2H), 2.82 (s, 3H), 1.69 - 1.64 (m, 3H), 1.35 (d, $J$ = 6.0 Hz, 6H), 0.97 (t, $J$ = 7.4 Hz, 4H), 0.88 (t, $J$ = 7.3 Hz, 3H). | 639.8 |
| Replace intermediate **a3** with: 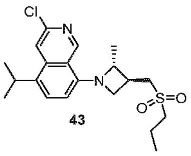 43 | 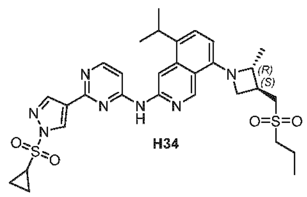 H34 | $^1$H NMR (400 MHz, DMSO-$d_6$) $\delta$ 10.40 (s, 1H), 9.11 (s, 1H), 8.79 (s, 1H), 8.70 (s, 1H), 8.51 (s, 1H), 8.43 (d, $J$ = 5.9 Hz, 1H), 7.46 (d, $J$ = 8.0 Hz, 1H), 7.23 (s,1H), 6.60 (d, $J$ = 8.2 Hz, 1H), 4.68 (t, $J$ = 7.4 Hz, 1H), 4.23 - 4.18 (m, 1H), 3.68 - 3.48 (m, 5H), 3.30 - 3.26 (m, 1H), 3.08 (dd, $J$ = 9.0, 6.7 Hz, 2H), 2.92 - 2.87(m, 1H), 1.72 (dd, $J$ = 15.4, 7.5 Hz, 2H), 1.43 (d, $J$ = 6.0 Hz, 3H), 1.38 (dd, $J$ = 6.8, 2.2 Hz, 6H), 1.30 - 1.19 (m, 1H), 1.00 (t, $J$ = 7.4 Hz, 3H). | 624.8 |

**Example 6**

**Preparation of target molecules H3, H6-H11, H13-H22, H24, and H26-H32**

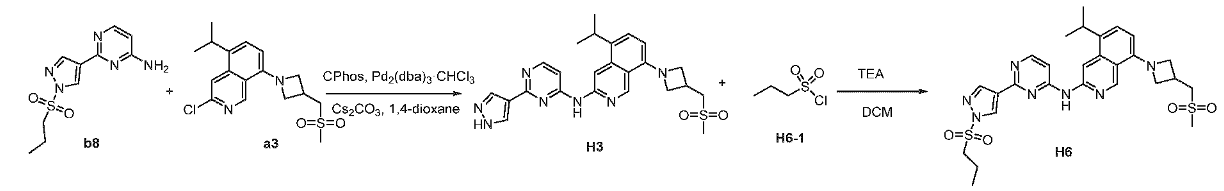

b8 a3 H3 H6-1 H6

Under nitrogen protection, intermediate **a3** (9 mg, 0.026 mmol), $Cs_2CO_3$ (17 mg, 0.052 mmol) and intermediate **b8** (7 mg, 0.026 mmol) were mixed in 1.5 mL of 1,4-dioxane. $Pd_2(dba)_3CHCl_3$ (81 mg, 0.008 mmol) and C-phos (3.0 mg, 0.008 mmol) were added. The mixture was heated to 100°C and reacted for 20 hours. The reaction was completed by LC-MS monitoring. The mixture was filtered. The filtrate was concentrated, and separated by HPLC preparative chromatography to obtain a yellow solid **H3** (5.0 mg), yield: 44%, LCMS: ESI-MS (m/z): 437.3 [M + H]$^+$.

In an ice bath, compound **H3** (5 mg, 0.011 mmol) was dissolved in 1.5 mL of dichloromethane. Triethylamine (3 mg, 0.021 mmol) and n-propylsulfonyl chloride (3 mg, 0.021 mmol) were added. The mixture was warmed to room temperature and reacted for 1 hour. The reaction was stopped. 10 mL of water was added to the reaction solution. The mixture was extracted with ethyl acetate, concentrated, and separated by HPLC preparative chromatography to obtain compound **H6** (2.1 mg), LCMS: ESI-MS (m/z): 584.0 [M + H]$^+$.

1H NMR (400 MHz, DMSO-$d_6$) $\delta$ 10.43 (s, 1H), 9.11 (s, 1H), 8.77 (s, 1H), 8.70 (s, 1H), 8.53 (s, 1H), 8.44 (d, $J$= 5.9 Hz, 1H), 7.46 (d, $J$ = 8.0 Hz, 1H), 7.25 (bs, 1H), 6.45 (d, $J$= 8.1 Hz, 1H), 4.43 - 4.39 (m, 2H), 3.98 -3.82 (m, 2H), 3.586-3.52 (m, 2H), 3.60 -3.57 (m, 3H), 3.29 -3.26 (m, 1H), 3.02 (s, 3H), 1.64 - 1.58 (m, 2H), 1.38 (d, $J$ = 6.8 Hz, 6H),

0.96 - 0.90 (m, 3H).

Referring to the synthetic route of compound **H6,** the following target molecules were synthesized using a similar skeleton structure.

| Replacement of structure | Target molecule | $^1$H NMR | LC-MS $[M+H]^+$ |
|---|---|---|---|
| Replace compound **H6-1** with: 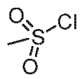 | 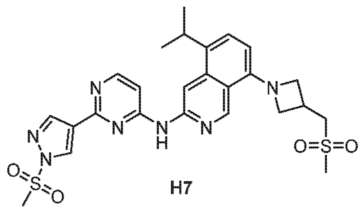 H7 | HNMR (400 MHz, DMSO-$d_6$) δ 10.38 (s, 1H), 9.10 (s, 1H), 8.79 (s, 1H), 8.69 (s, 1H), 8.50 (s, 1H), 8.43 (d, *J* = 5.9 Hz, 1H), 7.45 (d, *J* = 8.0 Hz, 1H), 7.24 (s, 1H), 6.45 (d, *J* = 8.1 Hz, 1H), 4.39-4.41 (m, 2H), 3.98-4.01 (m, 2H), 3.69 (s, 3H), 3.58-3.60 (m, 3H), 3.30 - 3.25 (m, 1H), 3.02 (s, 3H), 1.38 (d, *J* = 6.8 Hz, 6H). | 555.9 |
| Replace compound **H6-1** with: $Tf_2O$ | 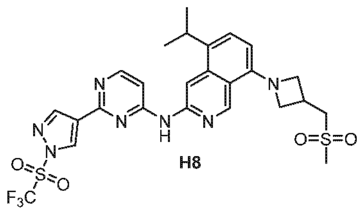 H8 | 1H NMR (400 MHz, DMSO-$d_6$) δ10.47 (s, 1H), 9.11 (s, 1H), 8.96 (s, 1H), 8.84 (s, 1H), 8.72 (s, 1H), 8.48 (d, *J* = 5.9 Hz, 1H), 8.25 (s, 2H), 7.45 (d, *J* = 8.0 Hz, 1H), 7.33 (s, 1H), 6.45 (d, *J* = 8.1 Hz, 1H), 4.39-4.43 (m, 2H), 4.01 - 3.96 (m, 2H), 3.60 (d, *J* = 7.4 Hz, 2H), 3.55 (d, *J* = 6.4 Hz, 1H), 3.28 - 3.26 (m, 1H), 3.02 (s, 3H), 1.35 (d, J = 7.9 Hz, 6H). | 609.9 |
| Replace compound **H6-1** with: 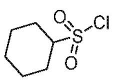 | 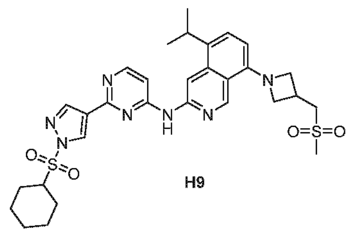 H9 | 1H NMR (400 MHz, DMSO-$d_6$) δ10.40 (s, 1H), 9.10 (s, 1H), 8.77 (s, 1H), 8.66 (s, 1H), 8.53 (s, 1H), 8.44 (d, *J* = 5.9 Hz, 1H), 7.45 (d, *J* = 8.0 Hz, 1H), 7.24 (s, 1H), 6.45 (d, *J* = 8.1 Hz, 1H), 4.39-4.43 (m, 2H), 3.96-4.00 (m, 2H), 3.91 - 3.84 (m, 1H), 3.59 (d, *J* = 8.0 Hz, 2H), 3.56 (d, *J* = 6.8 Hz, 1H), 3.28-3.31 (m, 1H), 3.02 (s, 3H), 1.90 (d, *J* = 10.0 Hz, 2H), 1.77 (d, *J* = 13.2 Hz, 2H), 1.59 (d, *J* = 13.0 Hz, 1H), 1.44 (dd, *J* = 12.4, 3.1 Hz, 2H), 1.38 (d, *J* = 6.8 Hz, 6H), 1.32 - 1.24 (m, 2H), 1.15 - 1.08 (m, 1H). | 614.0 |

(continued)

| Replacement of structure | Target molecule | $^1$H NMR | LC-MS $[M+H]^+$ |
|---|---|---|---|
| Replace compound **H6-1** with: 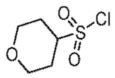 | 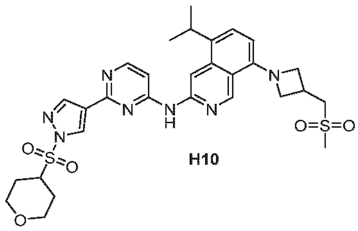 H10 | $^1$H NMR (400 MHz, DMSO-$d_6$) $\delta$ 10.40 (s, 1H), 9.10 (s, 1H), 8.76 (s, 1H), 8.68 (s, 1H), 8.55 (s, 1H), 8.44 (d, $J$ = 5.9 Hz, 1H), 7.46 (d, $J$ = 8.0 Hz, 1H), 7.24 (s, 1H), 6.45 (d, $J$ = 8.1 Hz, 1H), 4.41 (t, $J$ = 7.7 Hz, 2H), 4.24 - 4.20 (m, 1H), 3.98 (t, $J$ = 6.9 Hz, 2H), 3.91 (dd, $J$ = 11.6, 3.3 Hz, 2H), 3.60 (d, $J$ = 7.4 Hz, 2H), 3.57 - 3.53 (m, 1H), 3.32 - 3.26 (m, 3H), 3.02 (s, 3H), 1.78 - 1.65 (m, 4H), 1.38 (*d*, $J$ = 6.8 Hz, 6H). | 626.0 |
| Replace compound **H6-1** with: 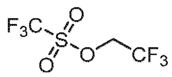 | 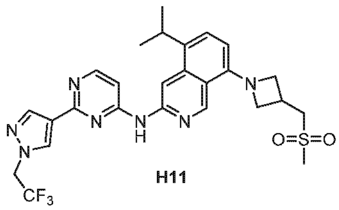 H11 | $^1$H NMR (400 MHz, DMSO-$d_6$) $\delta$ 10.27 (s, 1H), 9.10 (s, 1H), 8.67 (s, 1H), 8.44 (s, 1H), 8.38 (d, $J$ = 5.9 Hz, 1H), 8.20 (s, 1H), 7.45 (d, $J$ = 8.0 Hz, 1H), 7.23 (s, 1H), 6.44 (d, $J$ = 8.1 Hz, 1H), 5.31-5.24 (m, 2H), 4.41 (t, $J$ = 7.7 Hz, 2H), 3.98 (t, $J$ = 6.9 Hz, 2H), 3.60 (d, $J$ = 7.3 Hz, 2H), 3.58-3.55 (m, 1H), 3.29-3.27 (m, 1H), 3.02 (s, 3H), 1.34 (d, $J$ = 6.8 Hz, 6H). | 560.0 |
| Replace compound **H6-1** with: 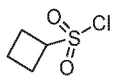 | 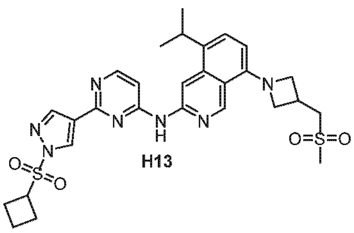 H13 | $^1$H NMR (400 MHz, DMSO-$d_6$) $\delta$ 0.36 (s, 1H), 9.10 (s, 1H), 8.77 (s, 1H), 8.68 (s, 1H), 8.51 (s, 1H), 8.43 (d, $J$ = 5.9 Hz, 1H), 7.45 (d, $J$ = 8.0 Hz, 1H), 7.24 (d, $J$ = 5.2 Hz, 1H),6.45 (d, $J$ = 8.1 Hz, 1H), 4.65-4.60 (m, 1H), 4.43-4.39 (m, 2H), 3.98-3.97 (m, 2H), 3.58 (d, $J$ = 7.4 Hz, 3H), 3.27 (m, 1H), 3.02 (s, 3H), 2.34-2.28 (m, 2H), 2.00-1.93 (m, 2H), 1.38 (d, $J$ = 6.8 Hz, 6H). | 596.0 |
| Replace compound **H6-1** with: 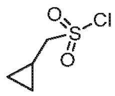 | 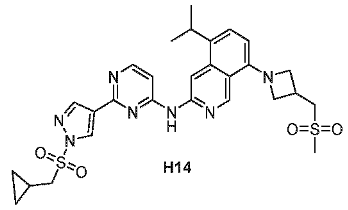 H14 | $^1$H NMR (400 MHz, DMSO-$d_6$) $\delta$ 10.39 (s, 1H), 9.10 (s, 1H), 8.79 (s, 1H), 8.70 (s, 1H), 8.52 (s, 1H), 8.43 (d, $J$ = 5.9 Hz, 1H), 7.45 (d, $J$ = 8.1 Hz, 1H), 7.23 (s, 1H), 6.45 (d, $J$ = 8.0 Hz, 1H), 4.41 (t, $J$ = 7.6 Hz, 2H), 3.98 (t, $J$ = 6.9 Hz, 2H), 3.82 (d, J = 7.1 Hz, 2H), 3.58 (t, $J$ = 9.2 Hz, 4H), 3.31 - 3.27 (m, 1H), 3.02 (s, 3H), 1.40 - 1.37 (m, 6H), 0.92 - 0.83 (m, 1H), 0.46 (d, $J$ = 4.7 Hz, 2H), 0.12 (d, $J$ = 5.9 Hz, 2H). | 596.1 |

(continued)

| Replacement of structure | Target molecule | $^1$H NMR | LC-MS [M+H]$^+$ |
|---|---|---|---|
| Replace compound **H6-1** with: 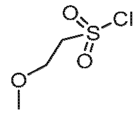 | 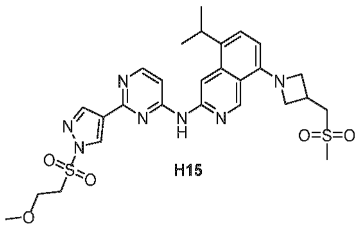 H15 | $^1$H NMR (400 MHz, DMSO-$d_6$) $\delta$ 10.38 (s, 1H), 9.10 (s, 1H), 8.78 (s, 1H), 8.65 (s, 1H), 8.49 (s, 1H), 8.43 (d, $J$ = 5.9 Hz, 1H), 7.46 (d, $J$ = 7.9 Hz, 1H), 7.23 (s, 1H), 6.45 (d, $J$ = 8.1 Hz, 1H), 4.41 (t, $J$ = 7.6 Hz, 2H), 4.10 (t, $J$ = 5.4 Hz, 2H), 3.98 (t, $J$ = 6.9 Hz, 2H), 3.69 (t, $J$ = 5.4 Hz, 2H), 3.60 (d, $J$ = 7.4 Hz, 3H), 3.33- 3.28 (m, 1H), 3.09 (s, 3H), 3.02 (s, 3H), 1.39 (d, $J$ = 6.8 Hz, 6H). | 600.1 |
| Replace compound **H6-1** with: 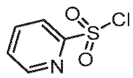 | 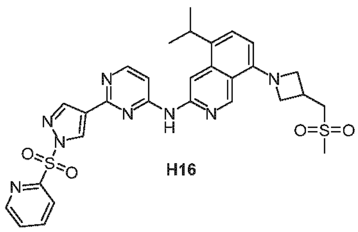 H16 | $^1$H NMR (400 MHz, DMSO-$d_6$) $\delta$ 10.38 (s, 1H), 9.25 (d, $J$ = 2.0 Hz, 1H), 9.10 (s, 1H), 8.98 (dd, $J$ = 4.8, 1.5 Hz, 1H), 8.94 (s, 1H), 8.74 (s, 1H), 8.52 - 8.49 (m, 1H), 8.48 (s, 1H), 8.42 (d, $J$ = 5.9 Hz, 1H), 7.75 (dd, $J$ = 7.7, 4.8 Hz, 1H), 7.46 (d, $J$ = 8.0 Hz, 1H), 7.26 (s, 1H), 6.45 (d, $J$ = 8.1 Hz, 1H), 4.41 (t, $J$ = 7.7 Hz, 2H), 3.98 (t, $J$ = 6.9 Hz, 2H), 3.61 (t, $J$ = 7.0 Hz, 3H), 3.31 - 3.28 (m, 1H), 3.02 (s, 3H), 1.38 (d, $J$ = 6.8 Hz, 6H). | 619.0 |
| Replace compound H6-1 with: 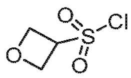 | 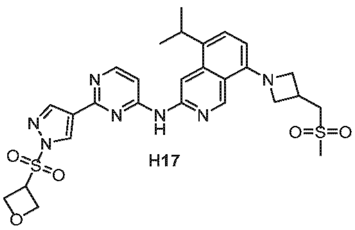 H17 | $^1$H NMR (400 MHz, DMSO-$d_6$) $\delta$ 10.38 (s, 1H), 9.10 (s, 1H), 8.77 (d, $J$ = 14.3 Hz, 2H), 8.54 (s, 1H), 8.44 (d, $J$ = 5.9Hz, 1H), 7.45 (d, $J$ =7.9Hz, 1H), 7.26 (s, 1H), 6.45 (d, $J$ = 8.0 Hz, 1H), 4.92 (t, $J$ = 7.8 Hz, 2H), 4.89 (d, $J$ = 5.9 Hz, 2H), 4.41 (t, $J$ = 7.8 Hz, 2H), 3.98 (t, $J$ = 7.8 Hz, 2H), 3.60 (d, $J$ = 7.4 Hz, 3H), 3.02 (s, 3H), 1.37 (d, $J$ = 6.8 Hz, 6H). | 598.0 |
| Replace compound **H6-1** 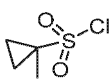 with: | 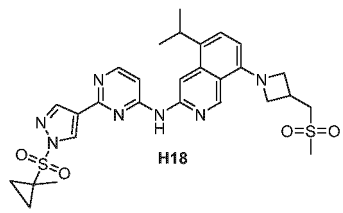 H18 | $^1$H NMR (400 MHz, DMSO-$d_6$) $\delta$ 10.39 (s, 1H), 9.10 (s, 1H), 8.77 (s, 1H), 8.70 (s, 1H), 8.54 (s, 1H), 8.44 (d, $J$ = 5.9 Hz, 1H), 7.45 (d, $J$ = 8.0 Hz, 1H), 7.25 (s, 1H), 6.45 (d, $J$ = 8.1 Hz, 1H), 4.41 (t, $J$ = 7.7 Hz, 2H), 3.98 (t, $J$ = 6.9 Hz, 3H), 3.58 (dd, $J$ = 15.2, 7.3 Hz, 4H), 3.31- 3.28 (m, 1H), 3.02 (s, 4H), 1.61 (d, $J$ = 4.6 Hz, 2H), 1.38 (d, $J$ = 6.0 Hz, 9H), 1.24 - 1.21 (m, 2H). | 596.1 |

(continued)

| Replacement of structure | Target molecule | $^1$H NMR | LC-MS $[M+H]^+$ |
|---|---|---|---|
| Replace compound **H6-1** with: 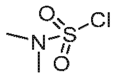 | 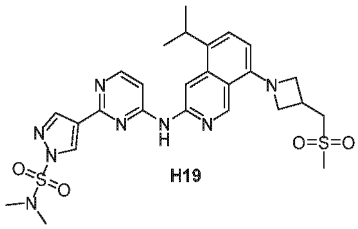 H19 | $^1$H NMR (400 MHz, DMSO-$d_6$) $\delta$ 10.36 (s, 1H), 9.10 (s, 1H), 8.78 (s, 1H), 8.64 (s, 1H), 8.46 (s, 1H), 8.42 (d, *J* = 5.9 Hz, 1H), 7.45 (d, *J* = 8.0 Hz, 1H), 7.23 (s, 1H), 6.45 (d, *J* = 8.1 Hz, 1H), 4.41 (t, *J* = 7.7 Hz, 2H), 3.98 (t, *J* = 6.8 Hz, 2H), 3.58 (dd, *J* = 12.3, 7.0 Hz, 3H), 3.31 - 3.28 (m, 1H), 3.02 (s, 3H), 2.94 (s, 6H), 1.37 (d, *J* = 6.8 Hz, 6H). | 585.0 |
| Replace compound **H6-1** with: 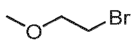 | 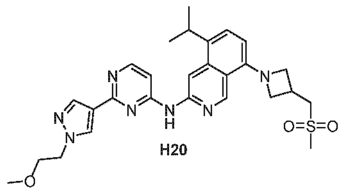 H20 | $^1$H NMR (400 MHz, DMSO-$d_6$) $\delta$ 10.19 (s, 1H), 9.09 (s, 1H), 8.77 (s, 1H), 8.35 (d, *J* =5.8Hz, 1H), 8.28 (s, 1H), 8.09 (s, 1H), 7.45 (d, *J* =8.0Hz, 1H), 7.14 (d, *J* =5.6 Hz, 1H), 6.43 (d, *J* = 8.1 Hz, 1H), 4.41 (t, *J* = 7.7 Hz, 2H), 3.98 (t, *J* = 6.9 Hz, 2H), 3.74 (t, *J* = 5.2 Hz, 3H),3.60 (d, *J*=7.4Hz, 3H), 3.31 - 3.28 (m, 1H), 3.24 (s, 3H), 3.02 (s, 3H), 1.36 (d, *J* = 6.8 Hz, 6H). | 536.1 |
| Replace intermediate **a3** with: 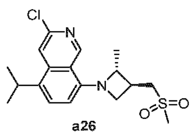 a26 Replace compound **H6-1** with: 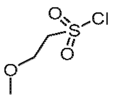 | 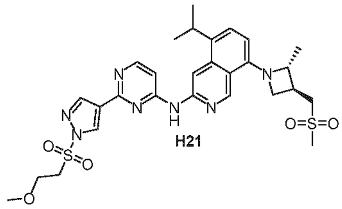 H21 | $^1$H NMR (400 MHz, DMSO-$d_6$) $\delta$ 10.43 (s, 1H), 9.11 (s, 1H), 8.81 (s, 1H), 8.66 (s, 1H), 8.50 (s, 1H), 8.43 (d, *J* =5.9 Hz, 1H), 7.47 (d, *J* =8.0Hz, 1H), 7.21 (s, 1H), 6.60 (d, *J* =8.0 Hz, 1H), 4.70 (t, *J* = 7.8 Hz, 1H), 4.21 (t, *J* = 7.8 Hz, 1H), 4.10 (t, *J* = 5.5 Hz, 3H), 3.69 (t, *J* = 5.5 Hz, 3H), 3.56-3.54 (m, 5H), 3.09 (s, 3H), 3.00 (s, 3H), 1.43 (d, *J* = 6.0 Hz, 3H), 1.39 (d, *J* = 6.7 Hz, 6H). | 614.1 |
| Replace intermediate **a3** with: 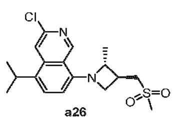 a26 Replace compound **H6-1** with: 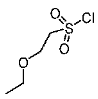 | 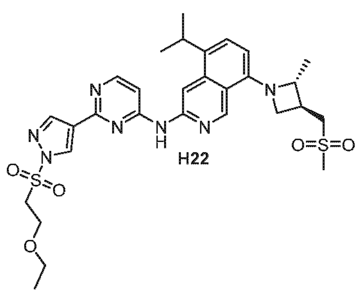 H22 | $^1$H NMR (400 MHz, DMSO-$d_6$) $\delta$ 10.41 (s, 1H), 9.11 (s, 1H), 8.82 (s, 1H), 8.64 (s, 1H), 8.48 (s, 1H), 8.42 (d, *J* = 5.8 Hz, 1H), 7.47 (d, *J* = 7.9 Hz, 1H), 7.19 (s,1H), 6.60 (*d, J* = 8.0 Hz, 1H), 4.70 - 4.67 (m, 1H), 4.23 - 4.18 (m, 1H), 4.12-4.05 (m, 2H), 3.76 - 3.70 (m, 2H), 3.68 - 3.66 (m, 1H), 3.58-3.52 (m, 2H), 3.26 - 3.20 (m, 2H), 3.17 - 3.16 (m, 1H), 3.00 (s, 3H), 2.91 - 2.89 (m, 1H), 1.55 - 1.29 (m, 9H), 0.84 (t, *J* = 6.8 Hz, 3H). | 628.0 |

(continued)

| Replacement of structure | Target molecule | $^1$H NMR | LC-MS [M+H]$^+$ |
|---|---|---|---|
| Replace compound **H6-1** with: 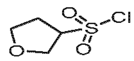 | 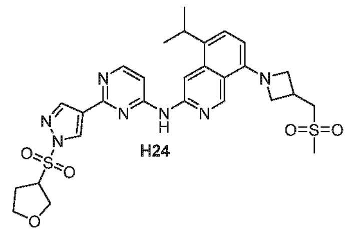 H24 | $^1$H NMR (400 MHz, DMSO-$d_6$) $\delta$ 10.40 (s, 1H), 9.10 (s, 1H), 8.76 - 8.73 (m, 2H), 8.54 (s, 1H), 8.44 (d, *J* = 5.9 Hz, 1H), 7.45 (d, *J* = 8.0 Hz, 1H), 7.25 (s, 1H), 6.45 (*d, J* = 8.1 Hz, 1H), 4.74 - 4.69 (m, 1H), 4.41 (t, *J* = 7.7 Hz, 2H), 4.22 - 4.18 (m, 1H), 4.02 - 3.88 (m, 4H), 3.80 - 3.74 (m, 1H), 3.67 - 3.63 (m, 4H), 3.02 (s, 3H), 2.325 - 2.28 (m, 2H), 1.37 (dd, *J* = 6.7, 2.7 Hz, 6H). | 612.0 |
| Replace compound **H6-1** with: 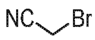 | 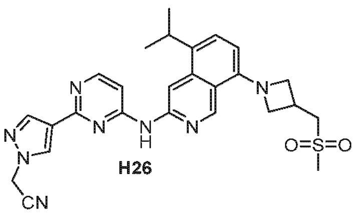 H26 | $^1$H NMR (400 MHz, DMSO-$d_6$) $\delta$ 10.25 (s, 1H), 9.10 (s, 1H), 8.70 (s, 1H), 8.48 (s, 1H), 8.44 (s, 1H), 8.38 (d, *J* = 5.8 Hz, 1H), 8.19 (s, 1H), 7.45 *(d, J* = 7.9 Hz, 1H), 7.22 (s, 1H), 6.44 *(d, J* = 8.0 Hz, 1H), 5.60 (s, 2H), 4.41 (t, *J* = 7.5 Hz, 2H), 3.98 (t, *J* = 6.8 Hz, 3H), 3.60 (d, *J* = 7.3 Hz, 2H), 3.58 - 3.54 (m, 1H), 3.30 - 3.27 (m, 1H), 3.02 (s, 3H), 1.36 (d, *J* = 6.7 Hz, 6H). | 517.9 |
| Replace compound **H6-1** with: 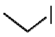 | 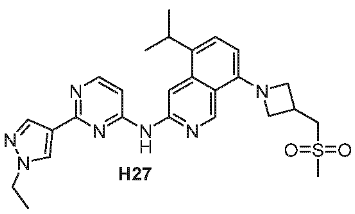 H27 | $^1$H NMR (400 MHz, DMSO-$d_6$) $\delta$ 10.19 (s, 1H), 9.09 (s, 1H), 8.78 (s, 1H), 8.36 - 8.30 (m, 2H), 8.08 (s, 1H), 7.45 (d, *J* = 8.0 Hz, 1H), 7.12 (*d, J* = 4.8 Hz, 1H), 6.43 (d, *J* = 8.0 Hz, 1H), 4.41 (t, *J* = 7.5 Hz, 2H), 4.23 (dd, *J* = 14.3, 7.1 Hz, 2H), 3.98 (t, *J* = 6.7 Hz, 2H), 3.64 - 3.56 (m, 3H), 3.30 - 3.25 (m, 1H), 3.02 (s, 3H), 1.44 (t, *J* = 7.2 Hz, 3H), 1.36 (d, *J* = 6.7 Hz, 6H). | 506.2 |
| Replace intermediate **a3** with: 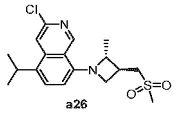 a26 Replace compound **H6-1** with: 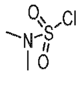 | 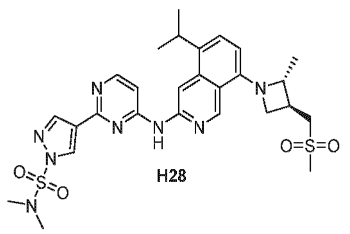 H28 | $^1$H NMR (400 MHz, DMSO-$d_6$) $\delta$ 10.39 (s, 1H), 9.07 (s, 1H), 8.76 (s, 1H), 8.61 (s, 1H), 8.43 - 8.36 (m, 2H), 7.42 (d, *J* = 8.0 Hz, 1H), 7.18 (s, 1 H), 6.56 (d, *J* = 8.1 Hz, 1H), 4.65 (*t, J* = 7.4 Hz, 1H), 4.20 - 4.13 (m, 1H), 3.62 (t, *J* = 7.1 Hz, 1H), 3.56 - 3.50 (m, 3H), 2.96 (s, 3H), 2.90 (s, 6H), 2.87 - 2.83 (m, 1H), 1.39 (d, *J* = 6.0 Hz, 3H), 1.34 (dd, *J* = 6.6, 2.8 Hz, 6H). | 599.8 |

(continued)

| Replacement of structure | Target molecule | ¹H NMR | LC-MS [M+H]⁺ |
|---|---|---|---|
| Replace compound **H6-1** with: Methyl iodide MeI | 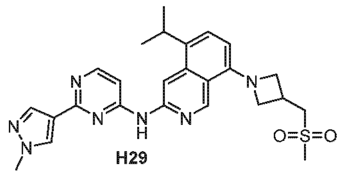<br>H29 | ¹H NMR (400 MHz, DMSO-$d_6$) $\delta$ 10.32 (s, 1H), 9.06 (s, 1H), 8.74 (s, 1H), 8.36 - 8.27 (m, 2H), 8.05 (s, 1H), 7.42 (d, $J$ = 8. 0 Hz, 1H), 7.11 (s, 1H), 6.41 (d, $J$ = 8.0 Hz, 1H), 4.37 (t, $J$ = 7.6 Hz, 2H), 3.96 - 3.92 (m, 2H), 3.91 (s, 3H), 3.56 (d, $J$ = 7.2 Hz, 3H), 3.27 - 3.23 (m, 1H), 2.98 (s, 3H), 1.32 (d, $J$ = 6.7 Hz, 6H). | 492.1 |
| Replace intermediate **a3** with:<br>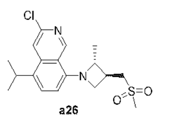<br>a26<br>Replace compound **H6-1** with:<br>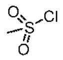 | 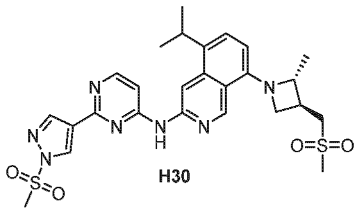<br>H30 | ¹H NMR (400 MHz, DMSO-$d_6$) $\delta$ 10.41 (s, 1H), 8.81 (s, 1H), 8.69 (s, 1H), 8.69 (s, 1H), 8.51 (s, 1H), 8.43 (d, $J$ = 5.9 Hz, 1H), 7.46 (d, $J$ = 8.0 Hz, 1H), 7.21 (d, $J$ = 4.6 Hz, 1H), 6.60 (d, $J$ = 8.1 Hz, 1H), 4.69 (t, $J$ = 7.5 Hz, 1H), 4.24 - 4.18 (m, 1H), 3.69 (s, 3H), 3.67 - 3.62 (m, 1H), 3.54 (ddd, $J$ = 22.6, 13.0, 7.5 Hz, 3H), 3.00 (s, 3H), 2.90 (dd, $J$ = 14.5, 7.2 Hz, 1H), 1.43 (d, $J$ = 6.0 Hz, 3H), 1.38 (dd, $J$ = 6.6, 2.8 Hz, 6H). | 570.0 |
| Replace intermediate **a3** with:<br>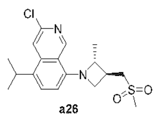<br>a26<br>Replace compound **H6-1** with:<br>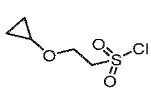 | 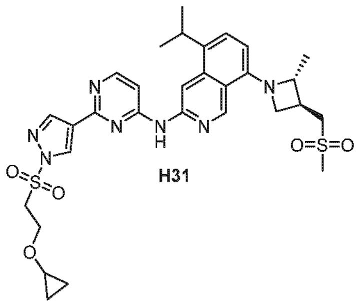<br>H31 | ¹H NMR (400 MHz, DMSO-$d_6$) $\delta$ 10.62 (s, 1H), 9.13 (s, 1H), 8.75 (d, $J$ = 18.2 Hz, 1H), 8.69 (s, 1H), 8.51 (s, 1H), 8.45 (d, $J$ = 6.0 Hz, 1H), 7.48 (d, $J$ = 8.0 Hz, 1H), 7.26 (s, 1H), 6.62 (d, $J$ = 8.2 Hz, 1H), 4.70 (t, $J$ = 7.5 Hz, 1H), 4.22 (t, $J$ = 6.6 Hz, 1H), 4.11 (t, $J$ = 5.3 Hz, 2H), 3.77 (t, $J$ = 5.3 Hz, 2H), 3.67 (t, $J$ = 7.2 Hz, 1H), 3.61 - 3.50 (m, 3H), 3.17 (dt, $J$ = 8.9, 4.6 Hz, 1H), 3.00 (s, 3H), 2.95 - 2.87 (m, 1H), 1.44 (d, $J$ = 6.1 Hz, 3H), 1.39 (dd, $J$ = 6.7, 3.3 Hz, 6H), 0.29 (t, $J$ = 4.6 Hz, 4H). | 640.0 |

(continued)

| Replacement of structure | Target molecule | $^1$H NMR | LC-MS $[M+H]^+$ |
|---|---|---|---|
| Replace intermediate **a3** with: 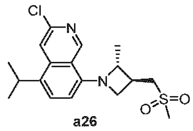 a26 Replace compound **H6-1** with: 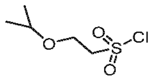 | 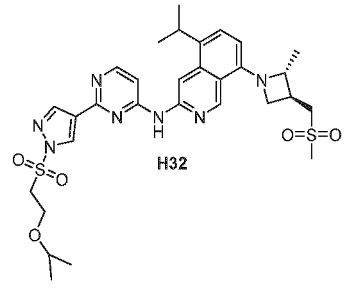 H32 | $^1$H NMR (400 MHz, DMSO-$d_6$) $\delta$ 10.39 (s, 1H), 9.11 (s, 1H), 8.81 (s, 1H), 8.64 (s, 1H), 8.48 (s, 1H), 8.42 (d, $J$ = 5.7 Hz, 1H), 7.47 (d, $J$ = 7.9 Hz, 1H), 7.20 (s, 1H), 6.60 (d, $J$ = 7.9 Hz, 1H), 4.71 - 4.66 (m, 1H), 4.23 - 4.18 (m, 1H), 4.05- 4.03 (m, $J$ = 4.6 Hz, 2H), 3.74 - 3.69 (m, 3H), 3.57 - 3.49 (m, 4H), 2.95-2.89 (m,1H), 3.00 (s, 3H), 1.45 - 1.38 (m, 6H), 1.23 (s, 3H), 0.86 (d, $J$ = 5.9 Hz, 6H). | 642.1 |

**Example 7**

The inhibitory effect of the compounds of the present disclosure on kinase EGFR activity:
The MSA (Mobility Shift Assay) method was used to detect the effects of small molecule inhibitors on the kinase activity of EGFR wild type and its mutants (EGFR$^{wt,}$ EGFR-L858R, EGFR-L858R-T790M-C797S, EGFR-L858R-C797S).

10 μL of kinase solution at 2.5 times the final concentration was added to the compound wells and positive control Max wells; 10 μL of 1 × kinase buffer was added to the negative control Min wells. The mixture was centrifuged at 1000 rpm for 30 seconds, and the reaction plate was shaken to mix the solution well and then incubated at room temperature for 10 minutes. A mixed solution of ATP and kinase substrate at 5/3 times the final concentration was prepared in 1 × kinase buffer. 15 μL of a mixed solution of ATP and substrate at 5/3 times the final concentration was added to initiate the reaction. The 384-well plate was centrifuged at 1000 rpm for 30 seconds, and shaken to mix the solution well and then incubated at room temperature for the corresponding time. 30 μL of stop detection solution was added to stop the kinase reaction, and the mixture was centrifuged at 1000 rpm for 30 seconds, and then to mixed well by shaking. The conversion rate was read using Caliper EZ Reader.

Calculation formula:

$$\%\ \text{Inhibition} = \frac{\text{Conversion\%_max} - \text{Conversion\%_sample}}{\text{Conversion\%_max} - \text{Conversion\%_min}} \times 100$$

wherein: Conversion%_sample is the conversion rate reading of the sample; Conversion%_min is the mean of the negative control wells, representing the conversion rate reading of the wells without enzyme activity; Conversion%_max is the mean of the positive control wells, representing the conversion rate reading of the wells without compound inhibition.

Fitting dose-effect curve:

The log (inhibitor) vs. response -Variable slope of the analysis software GraphPad Prism 5 was used to fit the dose-effect curve, where the log value of the concentration was used as the X-axis and the percentage inhibition rate was used as the Y-axis, to obtain the IC $_{50}$ value of each compound on the enzyme activity.

The calculation formula is Y=Bottom + (Top-Bottom)/(1+10^((LogIC$_{50}$-X)*HillSlope))

**Table 1: Inhibitory test results of compounds on EGFR (wild-type), EGFR (L858R), EGFR (L858R/C797S) and EGFR (L858R/T790M/C797S)**

| Compound | EGFR wt/ $IC_{50}$/nM | EGFR (L858R)/ $IC_{50}$/nM | EGFR (L858R/C797S)/ $IC_{50}$/nM | EGFR (L858R/T790M/C797S)/ $IC_{50}$/nM |
|---|---|---|---|---|
| **P1** | **>100** | **2.78** | **58.3** | **0.47** |

(continued)

| Compound | EGFR wt/ $IC_{50}$/nM | EGFR (L858R)/ $IC_{50}$/nM | EGFR (L858R/C797S)/ $IC_{50}$/nM | EGFR (L858R/T790M/C797S)/ $IC_{50}$/nM |
|---|---|---|---|---|
| P2 | >100 | 43.0 | >100 | 6.7 |
| P3 | 17.9 | 5.8 | 10.6 | 0.71 |
| P4 | >100 | 43.3 | >100 | 4.6 |
| P5 | >100 | >100 | >100 | 7.1 |
| P6 | >100 | 45.2 | 89.8 | 6.1 |
| P7 | 50.5 | 14.0 | 22.0 | 0.21 |
| P8 | >100 | 67.8 | 83.3 | 0.97 |
| P9 | >100 | >100 | >100 | 3.4 |
| P10 | >100 | 43.2 | 41.0 | 0.56 |
| P11 | >100 | >100 | >100 | 4.8 |
| P12 | >100 | >100 | >100 | 1.9 |
| P13 | >100 | 31.2 | 34.5 | 1.0 |
| P14 | 80.7 | 9.5 | 11.8 | 0.12 |
| P15 | >100 | 98.9 | >100 | 1.7 |
| P16 | >100 | N.D. | 24.1 | 0.28 |
| P17 | >100 | 73.4 | >100 | 1.6 |
| P18 | >100 | N.D. | >100 | 9.9 |
| P19 | >100 | >100 | >100 | 3.4 |
| P20 | 90.9 | 20.5 | 43.1 | 0.42 |
| P21 | 8.4 | 1.9 | 2.3 | 0.06 |
| P22 | >100 | >100 | >100 | 3.2 |
| P23 | 33.8 | 7.8 | 8.2 | 0.11 |
| P24 | 41.2 | N.D. | 10.4 | 0.13 |
| P25 | 5.1 | 0.77 | 1.22 | 0.08 |
| P26 | >100 | >100 | >100 | 30.9 |
| P28 | 64 | 13 | 27 | 0.34 |
| P29 | 8.4 | 1.3 | 2.5 | 0.07 |
| P30 | 18.1 | 3.5 | 4.8 | 0.10 |
| P39a | 9.4 | 1.1 | 2.0 | 0.05 |
| P45 | 20.7 | 4.1 | 7.6 | 0.18 |
| P46 | 41.8 | 6.2 | 9.3 | 0.61 |
| P51 | 13.4 | 2.5 | 4.2 | 0.12 |
| H1 | 3.9 | 1.6 | 1.5 | 0.4 |
| H2 | >100 | >100 | >100 | 71.6 |
| H3 | 11.7 | 5.6 | 4.7 | 0.7 |
| H4 | 6.1 | 2.1 | 2.9 | 0.3 |

(continued)

| Compound | EGFR wt/ $IC_{50}$/nM | EGFR (L858R)/ $IC_{50}$/nM | EGFR (L858R/C797S)/ $IC_{50}$/nM | EGFR (L858R/T790M/C797S)/ $IC_{50}$/nM |
|---|---|---|---|---|
| H5 | 10.2 | 4.6 | 1.9 | 0.43 |
| H6 | 5.2 | 1.5 | 2.2 | 0.3 |
| H7 | 2.4 | N.D. | 0.77 | 0.14 |
| H8 | >100 | N.D. | 6.4 | 3.5 |
| H9 | >100 | N.D. | 12.0 | 6.3 |
| H10 | 30.3 | 8.1 | 7.2 | 0.34 |
| H11 | 24.3 | 5.8 | 5.1 | 0.44 |
| H12 | 1.9 | 0.51 | 0.60 | 0.43 |
| H13 | 11.4 | 1.8 | 2.5 | 0.91 |
| H18 | 7.3 | 2.6 | 5.0 | 0.60 |
| H19 | 7.4 | 2.9 | 4.6 | 0.40 |
| Control A | 30.2 | 7.1 | 11.0 | 0.19 |
| N.D. = Not Detected | | | | |

The above results show that the molecules of the present disclosure have a good inhibitory effect on osimertinib-resistant mutations in EGFR, and have a weaker inhibition on wild-type EGFR, thus having high selectivity.

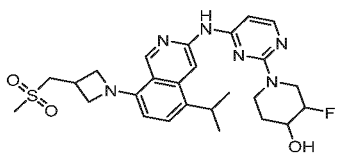

(**Control A**; WO2021133809)

**Example 8**

Promega CellTiter-Glo reagent was used to detect the effects of small molecule inhibitors on the proliferation of five Ba/F3 cell lines (Ba/F3-FL-EGFR, Ba/F3-FL-EGFR-L858R, Ba/F3-FL-EGFR-L858R-T790M-C797S, Ba/F3-TEL-EGFR-L858R-C797S and Ba/F3-FL-EGFR-del19-C797S).

The cell lines were cultured in an incubator at 37°C and 5% $CO_2$. Cells were passaged regularly and cells in the logarithmic phase were used for plating. 95 μL of cell suspension was added to each well of the cell plate, and culture medium without cells (containing 0.1% DMSO) was added to the Min control wells. Substance addition for compound detection cell plate: 5 μL of 20× compound working solution was added to the cell culture plate as shown in the table below. 5 μL of DMSO-cell culture medium mixture was added to the Max control, with the final DMSO concentration of 0.1%. 5 μL of DMSO-cell culture medium mixture was added to the Max control. The final DMSO concentration was 0.1%.

The compounds to be tested were added as per the table below

| 1 ×Compound solution | C-1 | C-2 | C-3 | C-4 | C-5 | C-6 | C-7 | C-8 | C-9 | C-10 |
|---|---|---|---|---|---|---|---|---|---|---|
| Concentration (μM) | 100000 | 2.5000 | 0.6250 | 0.1563 | 0.0391 | 0.0098 | 0.0024 | 0.00061 | 0.00015 | 10.0000 |
| C1 to C9 (μL) | 5 | 5 | 5 | 5 | 5 | 5 | 5 | 5 | 5 | 5 |
| Culture medium (μL) | 95 | 95 | 95 | 95 | 95 | 95 | 95 | 95 | 95 | 95 |

The culture plate was incubated in a 37 °C, 5% $CO_2$ incubator for 72 hours. Cell viability was detected by CellTiter-Glo luminescence assay. Data analysis:

The cell proliferation inhibition rate (Inhibition Rate) data was processed using the following formula:

$$\text{Inhibition Rate(Inh\%)}=100-(RLU_{Drug}-RLU_{Min})/(RLU_{Max}-RLU_{Min})*100\%.$$

wherein: $RLU_{Drug}$ represents the relative luminescence unit of the cells with the addition of drug, $RLU_{Min}$ represents the luminescence unit of the culture medium, and $RLU_{Max}$ represents the relative luminescence unit of the cells with the addition of DMSO.

The inhibition rates corresponding to different concentrations of compounds were calculated in EXCEL, and then the inhibition rate curve was plotted using GraphPad Prism software and related parameters including the maximum and minimum inhibition rates of cells and $IC_{50}$ values were calculated.

**Table 2: Antiproliferative inhibitory effects of representative compounds on BaF3 cells transfected with kinase EGFRwt, EGFR(L858R), EGFR(L858R/C797S), EGFR(L858R/T790M/C797S) and EGFR(del19/C797S)**

| Compound | Ba/F3-FL-EGFR $IC_{50}$/nM | Ba/F3-FL-EGFR-L858R $IC_{50}$/nM | Ba/F3-TEL-EGFR-L858R-C797S $IC_{50}$/nM | Ba/F3-FL-EGFR-L858R-T790M-C797S $IC_{50}$/nM | Ba/F3-FL-EGFR-del19-C797S $IC_{50}$/nM |
|---|---|---|---|---|---|
| P1 | 1425 | 656 | 389 | 340 | N.D. |
| P3 | 889 | 55 | 43 | 248 | N.D. |
| P7 | 7377 | 771 | 271 | 554 | N.D. |
| P21 | 1511 | 76 | 38.6 | 36 | 205 |
| P24 | 3436 | 128 | 63 | 57 | 283 |
| P25 | 618 | 42 | 15 | 11 | 71 |
| P27 | 2198 | 169 | 74 | 64 | 226 |
| P29 | 1088 | 41 | 13 | 13 | 202 |
| P30 | 2339 | 111 | 48 | 47 | 200 |
| P31 | 1461 | 86 | 19 | 18 | 295 |
| P32 | 565 | 22 | 16 | 8.9 | 123 |
| P33 | 1504 | 218 | 115 | 91 | 351 |
| P34 | 903 | 206 | 83 | 78 | 234 |
| P35 | 8761 | 2518 | 1695 | 1659 | 3121 |
| P36 | 1086 | 33 | 8.3 | 14 | 159 |
| P37 | 1142 | 83 | 33 | 34 | 339 |
| P38 | 540 | 15 | 7.0 | 5.4 | 76 |
| P39a | 670 | 18 | 14 | 8.8 | 52 |
| P39b | 760 | 24 | 16 | 14 | 60 |
| P40a | 421 | 20 | 5.8 | 2.5 | 53 |
| P40b | 465 | 25 | 5.9 | 4.8 | 81 |
| P41a | 693 | 22 | 7.1 | 4.6 | 57 |
| P41b | 464 | 15 | 5.6 | 3.7 | 35 |
| P42 | 761 | 16 | 6.6 | 5.8 | 90 |
| P43 | 593 | 36 | 9.8 | 5.6 | 142 |

(continued)

| Compound | Ba/F3-FL-EGFR $IC_{50}$/nM | Ba/F3-FL-EGFR-L858R $IC_{50}$/nM | Ba/F3-TEL-EGFR-L858R-C797S $IC_{50}$/nM | Ba/F3-FL-EGFR-L858R-T790M-C797S $IC_{50}$/nM | Ba/F3-FL-EGFR-del19-C797S $IC_{50}$/nM |
|---|---|---|---|---|---|
| P44 | 775 | 21 | 10 | 6.7 | 110 |
| P45 | 2777 | 181 | 61 | 38 | 265 |
| P46 | 3107 | 208 | 88 | 271 | 308 |
| P47 | 1610 | 113 | 38 | 42 | 179 |
| P48 | 1167 | 76 | 29 | 31 | 144 |
| P49 | 1072 | 121 | 36 | 38 | 316 |
| P50 | 6625 | 1891 | 542 | 720 | 3510 |
| P51 | 1748 | 86 | 28 | 63 | 181 |
| H1 | 101 | 5.9 | 3.1 | 16 | 10 |
| H3 | 854 | N.D. | 188 | 462 | 216 |
| H4 | 628 | 46 | 39 | 146 | N.D. |
| H6 | 559 | N.D. | 40 | 90 | 48 |
| H7 | 365 | 13 | 11 | 25 | 12 |
| H10 | 1082 | 94 | 54 | 219 | 101 |
| H11 | >10000 | 40 | 37 | 123 | 97 |
| H12 | 209 | 7.7 | 5.8 | 16 | 4.3 |
| H13 | 640 | 44 | 18 | 158 | 23 |
| H14 | 721 | 71 | 28 | 136 | 34 |
| H15 | 148 | 5.7 | 4.3 | 8.6 | 6.6 |
| H16 | 1359 | 235 | 122 | 652 | 264 |
| H17 | 858 | 56 | 33 | 127 | 58 |
| H18 | 658 | 73 | 46 | 150 | 69 |
| H19 | 559 | 64 | 36 | 123 | 57 |
| H20 | 2113 | 43 | 22 | 188 | 53 |
| H21 | 76 | 2.9 | 1.8 | 4.4 | 4.3 |
| H22 | 229 | 17 | 13 | 14 | 14 |
| H23 | 121 | 3.7 | 4.3 | 10 | 3.1 |
| H24 | 458 | 42 | 12 | 94 | 20 |
| H25 | 208 | 14 | 9.6 | 26 | 11 |
| H26 | 1421 | 74 | 56 | 336 | 128 |
| H27 | >10000 | 49 | 21 | 322 | 53 |
| H28 | 595 | 50 | 23 | 68 | 48 |
| H29 | 739 | 91 | 34 | 235 | 59 |
| H30 | 259 | 10 | 8.9 | 21 | 9.2 |
| H31 | 503 | 50 | 55 | 64 | 39 |
| H32 | 3292 | 510 | 226 | 397 | 340 |

(continued)

| Compound | Ba/F3-FL-EGFR $IC_{50}$/nM | Ba/F3-FL-EGFR-L858R $IC_{50}$/nM | Ba/F3-TEL-EGFR-L858R-C797S $IC_{50}$/nM | Ba/F3-FL-EGFR-L858R-T790M-C797S $IC_{50}$/nM | Ba/F3-FL-EGFR-del19-C797S $IC_{50}$/nM |
|---|---|---|---|---|---|
| H33 | 202 | 14 | 5.9 | 13 | 7.1 |
| H34 | 656 | 65 | 28 | 73 | 37 |
| Control A | 2022 | 176 | 52 | 38 | N.D. |
| Osimertinib | 13 | 1.5 | 2135 | 1747 | 906 |
| N.D. = Not Detected | | | | | |

The above results show that the compounds of the present disclosure have good antiproliferative effects on L858R-related mutations and osimertinib-resistant mutations in EGFR, and have weaker inhibitory effects on wild-type EGFR. Some compounds are significantly superior to the control molecules.

**Example 9**

Promega CellTiter-Glo reagent was used to detect the effects of small molecule inhibitors on the proliferation of three Ba/F3 cell lines (Ba/F3-FL-EGFR-*del19*, Ba/F3-FL-EGFR-*del19*-T790M, Ba/F3-FL-EGFR-*del19*-T790M-C797S).

The cell lines were cultured in an incubator at 37°C and 5% $CO_2$. Cells were passaged regularly and cells in the logarithmic phase were used for plating. 95 μL of cell suspension was added to each well of the cell plate, and culture medium without cells (containing 0.1% DMSO) was added to the Min control wells. Substance addition for compound detection cell plate: 5 μL of 20× compound working solution was added to the cell culture plate. 5 μL of DMSO-cell culture medium mixture was added to the Max control, with the final DMSO concentration of 0.1%. 5 μL of DMSO-cell culture medium mixture was added to the Max control. The final DMSO concentration was 0.1%.

The culture plate was incubated in a 37 °C, 5% $CO_2$ incubator for 72 hours. Cell viability was detected by CellTiter-Glo luminescence assay. Data analysis:

The cell proliferation inhibition rate (Inhibition Rate) data was processed using the following formula:

$$\text{Inhibition Rate(Inh\%)}=100-(RLU_{Drug}-RLU_{Min})/(RLU_{Max}-RLU_{Min})*100\%.$$

wherein: $RLU_{Drug}$ represents the relative luminescence unit of the cells with the addition of drug, $RLU_{Min}$ represents the luminescence unit of the culture medium, and $RLU_{Max}$ represents the relative luminescence unit of the cells with the addition of DMSO.

The inhibition rates corresponding to different concentrations of compounds were calculated in EXCEL, and then the inhibition rate curve was plotted using GraphPad Prism software and related parameters including the maximum and minimum inhibition rates of cells and $IC_{50}$ values were calculated.

Table 3: Antiproliferative inhibitory effects of representative compounds on BaF3 cells transfected with kinase EGFR (del19), EGFR(del19/T790M) and EGFR(de119/T790M/C797S)

| Compound | Ba/F3-FL-EGFR-Del19 (E746-A750) | Ba/F3-FL-EGFR-del19/T790M $IC_{50}$/nM | Ba/F3-FL-EG-FR-del19-T790M-C797S $IC_{50}$/nM |
|---|---|---|---|
| H12 | 15 | 5.1 | 7.8 |
| H23 | 3.1 | 0.3 | 3.1 |
| H30 | 15 | 5.1 | 7.8 |
| P39a | 41 | 4.4 | 8.4 |
| P41a | 54 | 3.2 | 5.9 |
| P41b | 36 | 2.3 | 3.7 |

(continued)

| Compound | Ba/F3-FL-EGFR-Del19 (E746-A750) | Ba/F3-FL-EGFR-del19/T790M $IC_{50}$/nM | Ba/F3-FL-EG-FR-del19-T790M-C797S $IC_{50}$/nM |
|---|---|---|---|
| **Osimertinib** | **5.8** | **2.0** | **1002** |

The above results indicate that the compounds of the present disclosure have good antiproliferative effects on *del19*-related mutations and **osimertinib**-resistant mutations in EGFR.

**Example 10:**

The liver microsome stability test of the compounds was as follows:

The compounds of the present disclosure were subjected to a liver microsome stability test. The compounds to be tested were co-incubated with liver microsomes of different species with or without the addition of NADPH. The final concentration of the compounds to be tested in the test system was 1 μM. The final concentration of NADPH was 1 mM, and the final concentration of liver microsomes was 0.5 mg/ml. The compound concentrations in the incubation supernatant at different time points within 60 minutes were detected and pharmacokinetic parameters (such as clearance Clint) were calculated.

This result indicates that the molecules of the present disclosure have good metabolic stability (especially in the human body).

| Compound | Human Clint/(mL/min/kg) | Mouse Clint/(mL/min/kg) |
|---|---|---|
| **P21** | 13.6 | 396 |
| **P29** | 23.4 | 440 |
| **P30** | <8.6 | 230 |
| **P39a** | <8.6 | 231 |
| **H1** | <8.6 | 174 |
| **H7** | 21.3 | 151 |
| **H12** | 14.2 | 196 |
| **H19** | 17.2 | 61.8 |
| **H23** | <8.6 | 448 |
| **H30** | 11.7 | 145 |

**Example 11:**

In vivo efficacy assay in BALB/c nude mice. Details were as follows:

BaF3 (*del19* /T790M/C797S) tumor cells were cultured and inoculated into 6-8 weeks old female BALB/c nude mice (weighing about 20 g), with all mice being subcutaneously inoculated. The mice were cultured in an SPF-grade experimental environment, and all mice had free access to commercially certified standard diets. When the average tumor volume of the mice grew to about 150 $mm^3$, the test compound was orally administered daily. The dosage was as follows: blank group vehicle (DMSO/Solutol/$H_2O$, 5/10/85). The dosage of the administration group was 100 mg/kg, twice a day; the dosage of the osimertinib group was 25 mg/kg, once a day. The tumor volume was measured three times a week with a two-dimensional caliper, and the animals were weighed every day. After 13 consecutive days of administration, the inhibition rate (TGI/100%) was calculated based on the final tumor volume. The volume calculation formula is: $V=1/2a*b^2$, where a represents the long diameter of the tumor, and b represents the short diameter of the tumor.

| Test drug | Dosage | Tumor volume ($mm^3$) | TGI |
|---|---|---|---|
| Blank group | 0 | 3870 | 0% |
| Osimertinib | 25 mg/kg, QD | 3110 | 20% |
| **H12** | 100 mg/kg$^{\$}$, BID | 1341 | 68% |

(continued)

| Test drug | Dosage | Tumor volume ($mm^3$) | TGI |
|---|---|---|---|
| **H23** | 100 mg/kg$, BID | 28 | 103% |
| **P39a** | 100 mg/kg, BID | 945 | 79% |
| **P41a** | 100 mg/kg, BID | 693 | 85% |

$ means that the dosage was 50 mg/kg for the first 4 days and 100 mg/kg starting from the 5th day.

The results show that the molecules of the present disclosure have good in vivo efficacy, have excellent inhibitory effects on osimertinib-resistant tumor cells, can overcome drug resistance, and bring clinical benefits.

## Claims

1. A compound of formula (I), or a pharmaceutically acceptable salt, isotopic variant, tautomer, stereoisomer, prodrug, polymorph, hydrate or solvate thereof

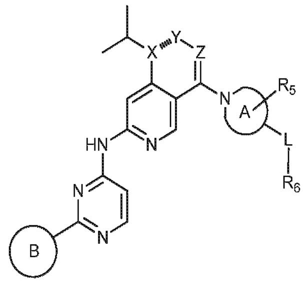

(I)

wherein:

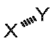 represents 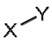 or 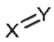; wherein when 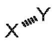 is 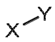, X is selected from CH and N, and Y is selected from $CH_2$, C=O and C=S; and when 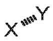 is 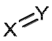, X is C, and Y is CH;
Z is selected from CH and N;
ring A is 4- to 8-membered heterocyclyl;
ring B is selected from

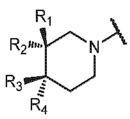 , 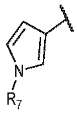 and 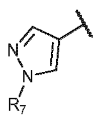 ;

L is selected from a chemical bond, -S(=O)(=M)-, -N($R_N$)-S(=O)(=M)-, -S(=O)(=M)-N($R_N$)-, - N($R_N$)-C(=O)-, -C(=O)-N($R_N$)-, -CH($R_N$)-C(=O)-, -C(=O)-CH($R_N$)-, -CH($R_N$)-S(=O)(=M)- and - S(=O)(=M)-CH($R_N$)-;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from -$C_{0-6}$ alkylene-$OR_a$, -$C_{0-6}$ alkylene-$NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, -$C_{1-6}$ alkylene-CN, -$C_{1-6}$ alkylene-$OR_a$, -$C_{1-6}$ alkylene-$NR_bR_c$, $C_{1-6}$ alkyl, $C_{16}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from -$C_{0-6}$ alkylene-$OR_a$, -$C_{0-6}$ alkylene-$NR_bR_c$, $C_{1-6}$ alkyl, -$C_{1-6}$ alkylene-$C_{3-6}$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which

are optionally substituted with one or more $R_a$;

$R_a$, $R_b$ and $R_c$ are independently selected from H, $C_{1-6}$ alkyl, $C_{1-6}$ haloalkyl, $C_{3-6}$ cycloalkyl and 4-to 6-membered heterocyclyl; or $R_b$ and $R_c$ together with the nitrogen atom to which they are attached form 4- to 7-membered heterocyclyl;

each group in X, Y, Z, ring A, ring B, L, Ri-Rs, $R_N$, $R_a$, $R_b$ and $R_c$ may be substituted by one or more deuterium atoms, up to full deuteration;

provided that, when L is -CH($R_N$)-S(=O)(=M)-, and the ring where X, Y and Z are located is a benzene ring, B is

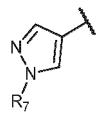.

2. The compound of formula (I) according to claim 1, or a pharmaceutically acceptable salt, isotopic variant, tautomer, stereoisomer, prodrug, polymorph, hydrate or solvate thereof, wherein the ring where X, Y and Z are located is selected from:

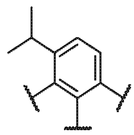, 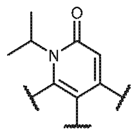 and 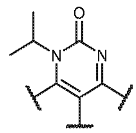.

3. The compound of formula (I) according to claim 1 or 2, or a pharmaceutically acceptable salt, isotopic variant, tautomer, stereoisomer, prodrug, polymorph, hydrate or solvate thereof, wherein ring A is selected from:

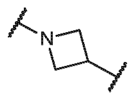, 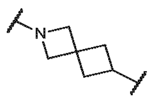 and 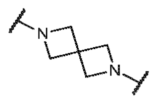.

4. The compound of formula (I) according to any one of claims 1 to 3, or a pharmaceutically acceptable salt, isotopic variant, tautomer, stereoisomer, prodrug, polymorph, hydrate or solvate thereof, wherein ring B is

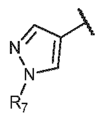.

5. The compound of formula (I) according to any one of claims 1 to 3, or a pharmaceutically acceptable salt, isotopic variant, tautomer, stereoisomer, prodrug, polymorph, hydrate or solvate thereof, wherein ring B is

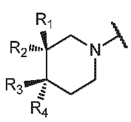.

6. The compound of formula (I) according to any one of claims 1 to 5, or a pharmaceutically acceptable salt, isotopic variant, tautomer, stereoisomer, prodrug, polymorph, hydrate or solvate thereof, wherein L is selected from -S(=O)(=M)-, -N($R_N$)-S(=O)(=M)-, -N($R_N$)-C(=O)-, -CH($R_N$)-C(=O)- and -CH($R_N$)-S(=O)(=M)-; alternatively, L is -N($R_N$)-S(=O)(=M)- or -CH($R_N$)-S(=O)(=M)-.

7. The compound of formula (I) according to any one of claims 1 to 6, or a pharmaceutically acceptable salt, isotopic variant, tautomer, stereoisomer, prodrug, polymorph, hydrate or solvate thereof, wherein one of Ri and $R_2$ is halogen, and the other is selected from H, halogen and $C_{1-6}$ alkyl; alternatively, one of $R_1$ and $R_2$ is F, and the other is selected from H, F and Me; alternatively, one of $R_1$ and $R_2$ is F, and the other is H.

8. The compound of formula (I) according to any one of claims 1 to 7, or a pharmaceutically acceptable salt, isotopic

variant, tautomer, stereoisomer, prodrug, polymorph, hydrate or solvate thereof, wherein one of $R_3$ and $R_4$ is $OR_a$, and the other is selected from H and $C_{1-6}$ alkyl; alternatively, one of $R_3$ and $R_4$ is OH or OMe, and the other is selected from H and Me.

**9.** The compound of formula (I) according to any one of claims 1 to 8, or a pharmaceutically acceptable salt, isotopic variant, tautomer, stereoisomer, prodrug, polymorph, hydrate or solvate thereof, wherein$R_5$ is selected from H and Me.

**10.** The compound of formula (I) according to any one of claims 1 to 9, or a pharmaceutically acceptable salt, isotopic variant, tautomer, stereoisomer, prodrug, polymorph, hydrate or solvate thereof, having the following structures:

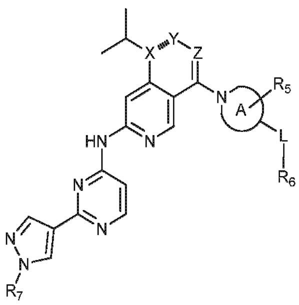
(II),
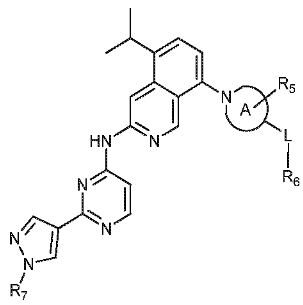
(III),
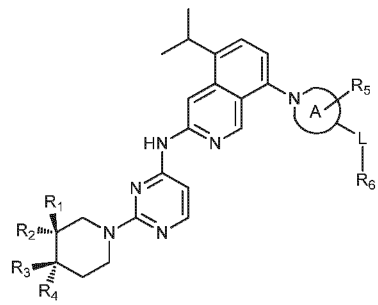
(IV),
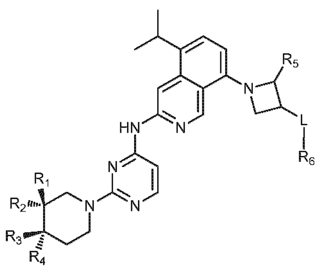
(V),
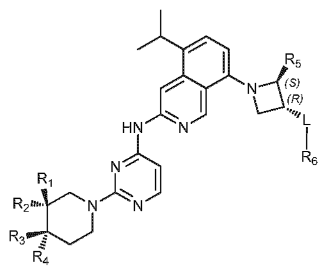
(V-1),
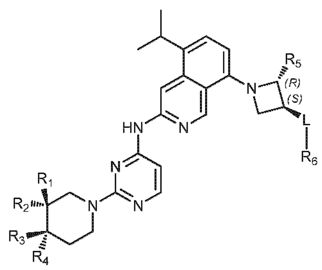
(V-2),
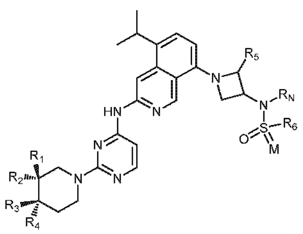
(VI),
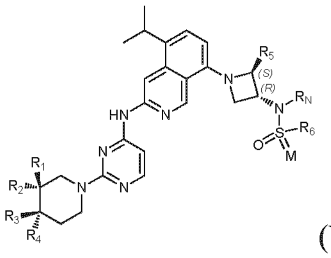
(VI-1),
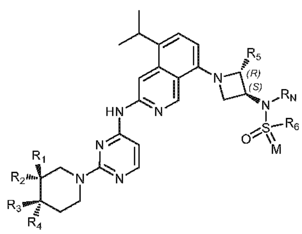
(VI-2),
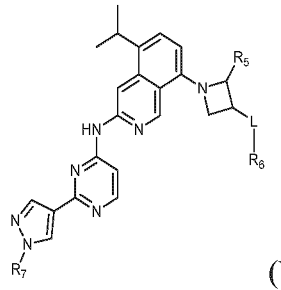
(VII),
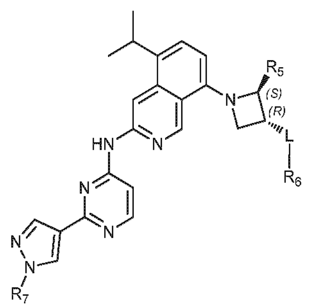
(VII-1) or
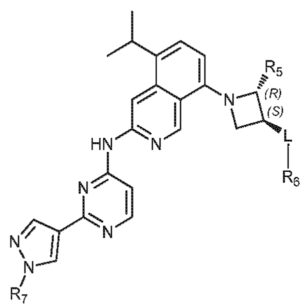
(VII-2),

wherein each group is as defined in any one of claims 1 to 9.

**11.** The compound of claim 10, or a pharmaceutically acceptable salt, isotopic variant, tautomer, stereoisomer, prodrug, polymorph, hydrate or solvate thereof, which is a compound of formula (**II**):

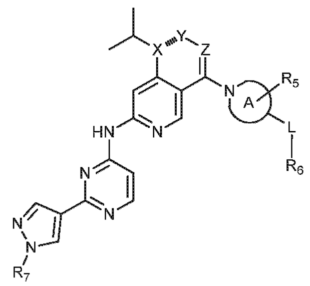

(II)

wherein: 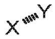

represents 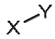 or 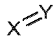; wherein when 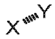 is 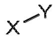, X is selected from CH and N, and Y is selected from $CH_2$, C=O and C=S; and when 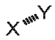 is 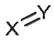, X is C, and Y is CH;
Z is selected from CH and N;
ring A is 4- to 8-membered heterocyclyl;
L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)-, -C(=O)-CH($R_N$)-, -CH($R_N$)-S(=O)(=M)- and -S(=O)(=M)-CH($R_N$)-;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, -$CH_2$-$C_3$-$_6$ cycloalkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

12. The compound of claim 10, or a pharmaceutically acceptable salt, isotopic variant, tautomer, stereoisomer, prodrug, polymorph, hydrate or solvate thereof, which is a compound of formula (III):

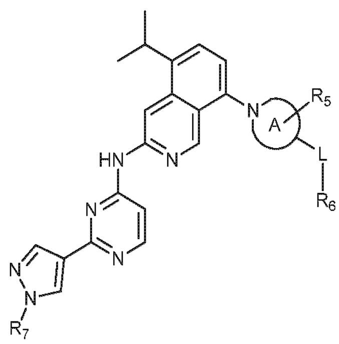

(III)

wherein:

ring A is 4- to 8-membered heterocyclyl;
L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)-, -C(=O)-CH($R_N$)-, -CH($R_N$)-S(=O)(=M)- and -S(=O)(=M)-CH($R_N$)-;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;

wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
Rs is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**13.** The compound of claim 12, or a pharmaceutically acceptable salt, isotopic variant, tautomer, stereoisomer, prodrug, polymorph, hydrate or solvate thereof, wherein:

ring A is selected from

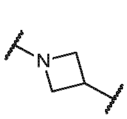, 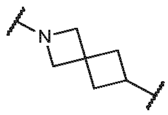 and 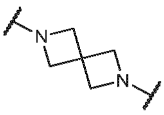;

L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)-, -C(=O)-CH($R_N$)-, -CH($R_N$)-S(=O)(=M)- and -S(=O)(=M)-CH($R_N$)-;
$R_5$ is selected from H and Me;
$R_6$ is selected from Me, Et, Pr, $CH_2CF_3$, cyclopropyl, OH, $NH_2$, NHMe, $NMe_2$, furan-2-yl and pyridin-4-yl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
Rs is selected from Me, Et, Pr, $CH_2CF_3$, cyclopropyl, OH, $NH_2$, NHMe, $NMe_2$, pyran-4-yl, furan-2-yl and pyridin-4-yl;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**14.** The compound of claim 12, or a pharmaceutically acceptable salt, isotopic variant, tautomer, stereoisomer, prodrug, polymorph, hydrate or solvate thereof, wherein:

ring A is 4- to 8-membered heterocyclyl;
L is selected from -S(=O)(=M)-, -CH($R_N$)-C(=O)- and -CH($R_N$)-S(=O)(=M)-;
$R_5$ is H, $C_{1-6}$ alkyl or $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl and 4- to 6-membered heterocyclyl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, $C_{1-6}$ haloalkyl, $C_{3-6}$ cycloalkyl and 4- to 6-membered heterocyclyl;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**15.** The compound of claim 12, or a pharmaceutically acceptable salt, isotopic variant, tautomer, stereoisomer, prodrug, polymorph, hydrate or solvate thereof, wherein:

ring A is 4- to 8-membered heterocyclyl;
L is selected from -S(=O)(=M)-, -CH($R_N$)-C(=O)- and -CH($R_N$)-S(=O)(=M)-;
$R_5$ is selected from H and $C_{1-6}$ alkyl;
$R_6$ is selected from $NR_bR_c$, $C_{1-6}$ alkyl and $C_{1-6}$ haloalkyl;
$R_7$ is selected from H, trifluoroethyl and -S(=O)(=M)-Rs;
wherein M is O or NH;
$R_N$ is selected from H, $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and pyran-4-yl;
$R_b$ and $R_c$ are independently selected from H, $C_{1-6}$ alkyl and $C_{1-6}$ haloalkyl.

**16.** The compound of claim 12, or a pharmaceutically acceptable salt, isotopic variant, tautomer, stereoisomer, prodrug, polymorph, hydrate or solvate thereof, wherein:

ring A is selected from

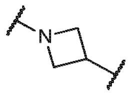 and 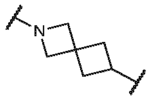;

L is selected from -S(=O)(=M)-, -CH($R_N$)-C(=O)- and -CH($R_N$)-S(=O)(=M)-;
$R_5$ is H;
$R_6$ is selected from Me and $NH_2$;
$R_7$ is selected from H and -S(=O)(=M)-Rs;
wherein M is O;
$R_N$ is selected from H, $C_{1-6}$ alkyl and $C_{1-6}$ haloalkyl;
Rs is selected from Me, Et, Pr, cyclopropyl and pyran-4-yl.

**17.** The compound of claim 10, or a pharmaceutically acceptable salt, isotopic variant, tautomer, stereoisomer, prodrug, polymorph, hydrate or solvate thereof, which is a compound of formula (IV):

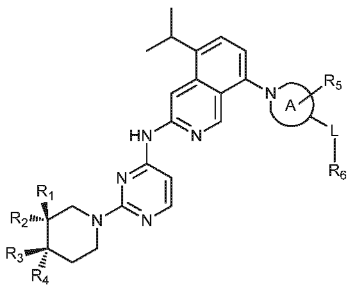

(IV)

wherein:

ring A is 4- to 8-membered heterocyclyl;
L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)- and -C(=O)-CH($R_N$)-;
Ri and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**18.** The compound of claim 17, or a pharmaceutically acceptable salt, isotopic variant, tautomer, stereoisomer, prodrug, polymorph, hydrate or solvate thereof, wherein:

ring A is selected from

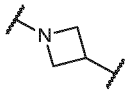, 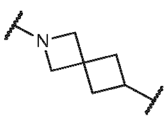 and 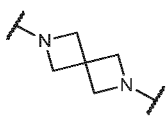;

L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)- and -C(=O)-CH($R_N$)-;
Ri and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**19.** The compound of claim 10, or a pharmaceutically acceptable salt, isotopic variant, tautomer, stereoisomer, prodrug, polymorph, hydrate or solvate thereof, which is a compound of formula (V), (V-1) or (V-2):

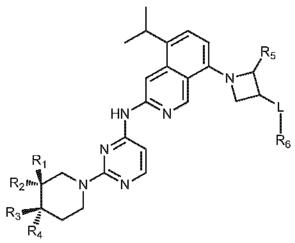
(V),
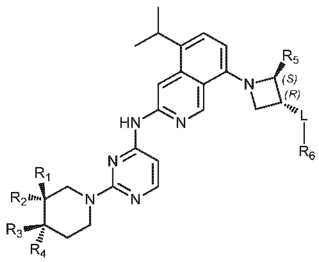
(V-1) or
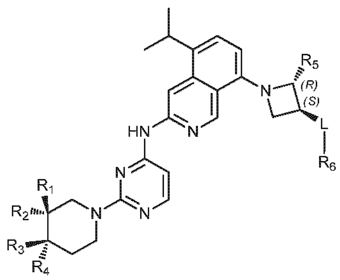
(V-2)

wherein:

L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)- and -C(=O)-CH($R_N$)-;
Ri and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**20.** The compound of claim 19, or a pharmaceutically acceptable salt, isotopic variant, tautomer, stereoisomer, prodrug, polymorph, hydrate or solvate thereof, wherein:

L is selected from -S(=O)(=M)-, -N($R_N$)-S(=O)(=M)-, -N($R_N$)-C(=O)- and -CH($R_N$)-C(=O)-;
one of $R_1$ and $R_2$ is halogen, and the other is selected from H, halogen and $C_{1-6}$ alkyl;
one of $R_3$ and $R_4$ is $OR_a$, and the other is selected from H and $C_{1-6}$ alkyl;
$R_5$ is selected from H, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl and 5- to 6-membered heteroaryl;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**21.** The compound of claim 10, or a pharmaceutically acceptable salt, isotopic variant, tautomer, stereoisomer, prodrug, polymorph, hydrate or solvate thereof, which is a compound of formula (VI), (VI-1) or (VI-2):

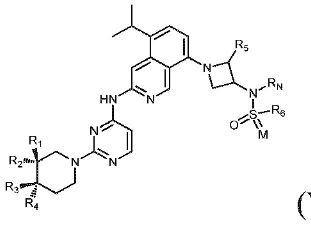 (VI), 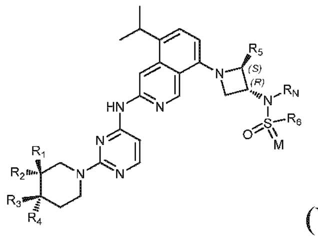 (VI-1) or 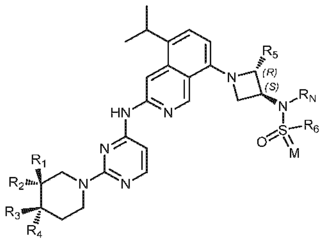 (VI-2)

wherein:

M is O or NH;
$R_1$ and $R_2$ are independently selected from H, halogen, $C_{1-6}$ alkyl and $C_{1-6}$ haloalkyl;
$R_3$ and $R_4$ are independently selected from H, $OR_a$, $C_{1-6}$ alkyl and $C_{1-6}$ haloalkyl;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
wherein $R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**22.** The compound of claim 21, or a pharmaceutically acceptable salt, isotopic variant, tautomer, stereoisomer, prodrug, polymorph, hydrate or solvate thereof, wherein:

M is O or NH;
one of $R_1$ and $R_2$ is halogen, and the other is selected from H, halogen and $C_{1-6}$ alkyl;
one of $R_3$ and $R_4$ is $OR_a$, and the other is selected from H and $C_{1-6}$ alkyl;
$R_5$ is selected from H and $C_{1-6}$ alkyl;
$R_6$ is selected from $OR_a$, $NR_bR_c$, $C_{1-6}$ alkyl, $C_{1-6}$ haloalkyl, $C_{3-6}$ cycloalkyl and 5- to 6-membered heteroaryl;
$R_N$ is selected from H and $C_{1-6}$ alkyl;
or $R_N$ and $R_6$ are linked to form $C_{1-6}$ alkylene;
wherein $R_a$, $R_b$ and $R_c$ are independently selected from H, $C_{1-6}$ alkyl and $C_{1-6}$ haloalkyl.

**23.** The compound of claim 21, or a pharmaceutically acceptable salt, isotopic variant, tautomer, stereoisomer, prodrug, polymorph, hydrate or solvate thereof, wherein:

M is O;
one of $R_1$ and $R_2$ is F, and the other is selected from H, F and Me;
one of $R_3$ and $R_4$ is OH or OMe, and the other is selected from H and Me;
$R_5$ is selected from H and Me;
$R_6$ is selected from Me, Et, Pr, $CH_2CF_3$, cyclopropyl, $NMe_2$, furan-2-yl and pyridin-4-yl;
$R_N$ is selected from H and Me;
or $R_N$ and $R_6$ are linked to form $C_{1-4}$ alkylene.

**24.** The compound of claim 21, or a pharmaceutically acceptable salt, isotopic variant, tautomer, stereoisomer, prodrug, polymorph, hydrate or solvate thereof, wherein:

M is O or NH;
one of $R_1$ and $R_2$ is selected from halogen, $C_{1-6}$ alkyl and $C_{1-6}$ haloalkyl, and the other is H;
one of $R_3$ and $R_4$ is selected from $OR_a$, $C_{1-6}$ alkyl and $C_{1-6}$ haloalkyl, and the other is H;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
wherein $R_a$, $R_b$ and $R_c$ are independently selected from H, $C_{1-6}$ alkyl and $C_{1-6}$ haloalkyl; or $R_b$ and

$R_c$ together with the nitrogen atom to which they are attached form 4- to 7-membered heterocyclyl.

**25.** The compound of claim 21, or a pharmaceutically acceptable salt, isotopic variant, tautomer, stereoisomer, prodrug, polymorph, hydrate or solvate thereof, wherein:

M is O or NH;
one of $R_1$ and $R_2$ is selected from halogen and $C_{1-6}$ alkyl, and the other is H;
one of $R_3$ and $R_4$ is $OR_a$, and the other is H;
$R_5$ is selected from H, $C_{1-6}$ alkyl and $C_{1-6}$ haloalkyl;
$R_6$ is selected from $C_{1-6}$ alkyl, $C_{1-6}$ haloalkyl, $C_{3-6}$ cycloalkyl and 4- to 6-membered heterocyclyl;
$R_N$ is selected from H, $C_{1-6}$ alkyl and $C_{1-6}$ haloalkyl;
wherein $R_a$ is selected from H, $C_{1-6}$ alkyl and $C_{1-6}$ haloalkyl.

**26.** The compound of claim 21, or a pharmaceutically acceptable salt, isotopic variant, tautomer, stereoisomer, prodrug, polymorph, hydrate or solvate thereof, wherein:

M is O;
one of $R_1$ and $R_2$ is halogen (alternatively F), and the other is H;
one of $R_3$ and $R_4$ is $OR_a$, and the other is H;
$R_5$ is selected from H and $C_{1-4}$ alkyl;
$R_6$ is selected from $C_{1-4}$ alkyl and $C_{1-4}$ haloalkyl;
$R_N$ is selected from H, $C_{1-4}$ alkyl and $C_{1-4}$ haloalkyl; alternatively $C_{1-4}$ alkyl;
wherein $R_a$ is selected from H and $C_{1-4}$ alkyl.

**27.** The compound of claim 10, or a pharmaceutically acceptable salt, isotopic variant, tautomer, stereoisomer, prodrug, polymorph, hydrate or solvate thereof, which is a compound of formula (VII), (VII-1) or (VII-2):

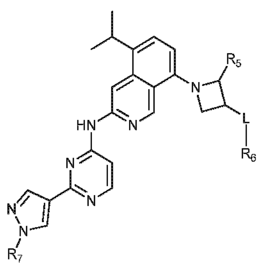
(VII),
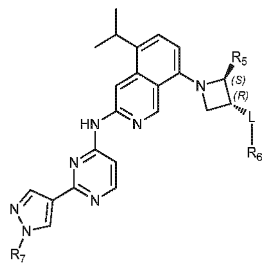
(VII-1) or
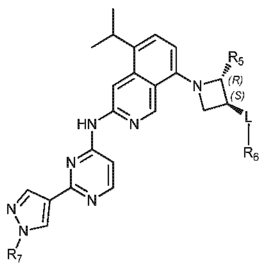
(VII-2)

wherein:

L is selected from -S(=O)(=M)-, -N($R_N$)-S(=O)(=M)-, -S(=O)(=M)-N($R_N$)-, -N($R_N$)-C(=O)-, - C(=O)-N($R_N$)-, -CH($R_N$)-C(=O)-, -C(=O)-CH($R_N$)-, -CH($R_N$)-S(=O)(=M)- and -S(=O)(=M)-CH($R_N$)-;
$R_5$ is selected from H, halogen, $C_{1-6}$ alkyl and $C_{1-6}$ haloalkyl;
$R_6$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl;
$R_7$ is selected from H, $C_{1-6}$ alkyl, $C_{1-6}$ haloalkyl and -S(=O)(=M)-Rs;
wherein M is O or NH;
$R_N$ is selected from H, $C_{1-6}$ alkyl and $C_{1-6}$ haloalkyl;
or $R_N$ and $R_6$ are linked to form $C_{1-6}$ alkylene, $C_{2-6}$ alkenylene or $C_{2-6}$ alkynylene;
$R_8$ is selected from $OR_a$, $NR_bR_c$, $C_{1-6}$ alkyl, $C_{1-6}$ haloalkyl, $C_{3-6}$ cycloalkyl, 4- to 6-membered heterocyclyl, $C_{6-10}$ aryl and 5- to 6-membered heteroaryl, which are optionally substituted with one or more $R_a$;
$R_a$, $R_b$ and $R_c$ are independently selected from H, $C_{1-6}$ alkyl and $C_{1-6}$ haloalkyl; or $R_b$ and $R_c$ together with the nitrogen atom to which they are attached form 4- to 7-membered heterocyclyl.

**28.** The compound of claim 27, or a pharmaceutically acceptable salt, isotopic variant, tautomer, stereoisomer, prodrug, polymorph, hydrate or solvate thereof, wherein:
wherein:

L is selected from -N($R_N$)-S(=O)(=M)- and -CH($R_N$)-S(=O)(=M)-;

$R_5$ is selected from $C_{1-6}$ alkyl and $C_{1-6}$ haloalkyl;
$R_6$ is selected from $C_{1-6}$ alkyl, $C_{1-6}$ haloalkyl, $C_{3-6}$ cycloalkyl and 4- to 6-membered heterocyclyl;
$R_7$ is -S(=O)(=M)-Rs;
wherein M is O or NH;
$R_N$ is selected from H, $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and 4- to 6-membered heterocyclyl, which are optionally substituted with one or more $R_a$;
$R_a$ is selected from H, $C_{1-6}$ alkyl and $C_{1-6}$ haloalkyl.

**29.** The compound of claim 27, or a pharmaceutically acceptable salt, isotopic variant, tautomer, stereoisomer, prodrug, polymorph, hydrate or solvate thereof, wherein:
wherein:

L is selected from $-N(R_N)-S(=O)(=M)-$ and $-CH_2-S(=O)(=M)-$;
$R_5$ is selected from $C_{1-6}$ alkyl and $C_{1-6}$ haloalkyl;
$R_6$ is selected from $C_{1-6}$ alkyl and $C_{1-6}$ haloalkyl;
$R_7$ is -S(=O)(=M)-Rs;
wherein M is O or NH;
$R_N$ is selected from $C_{1-6}$ alkyl and $C_{1-6}$ haloalkyl;
$R_8$ is selected from $C_{1-6}$ alkyl, $C_{1-6}$ haloalkyl, $C_{3-6}$ cycloalkyl and 4- to 6-membered heterocyclyl.

**30.** The compound of claim 27, or a pharmaceutically acceptable salt, isotopic variant, tautomer, stereoisomer, prodrug, polymorph, hydrate or solvate thereof, wherein:
wherein:

L is selected from $-N(R_N)-S(=O)_2-$ and $-CH_2-S(=O)_2-$;
$R_5$ is selected from $C_{1-6}$ alkyl and $C_{1-6}$ haloalkyl;
$R_6$ is selected from $C_{1-6}$ alkyl and $C_{1-6}$ haloalkyl;
$R_7$ is $-S(=O)_2-R_8$;
wherein $R_N$ is selected from $C_{1-6}$ alkyl and $C_{1-6}$ haloalkyl;
$R_8$ is selected from $C_{1-6}$ alkyl, $C_{1-6}$ haloalkyl and $C_{3-6}$ cycloalkyl.

**31.** The compound of claim 27, or a pharmaceutically acceptable salt, isotopic variant, tautomer, stereoisomer, prodrug, polymorph, hydrate or solvate thereof, wherein:
wherein:

L is selected from $-N(R_N)-S(=O)_2-$ and $-CH_2-S(=O)_2-$;
$R_5$ is $C_{1-4}$ alkyl;
$R_6$ is $C_{1-4}$ alkyl;
$R_7$ is $-S(=O)_2-R_8$;
wherein $R_N$ is $C_{1-4}$ alkyl;
$R_8$ is selected from $C_{1-4}$ alkyl and $C_{3-6}$ cycloalkyl.

**32.** A compound, or a tautomer, stereoisomer, prodrug, crystalline form, pharmaceutically acceptable salt, hydrate or solvate thereof, wherein the compound is selected from:

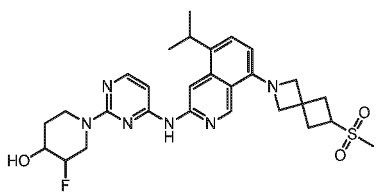,
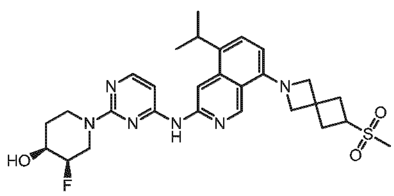,
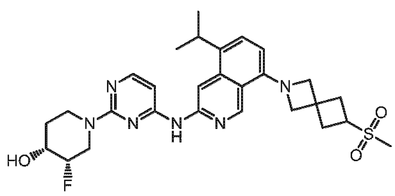,

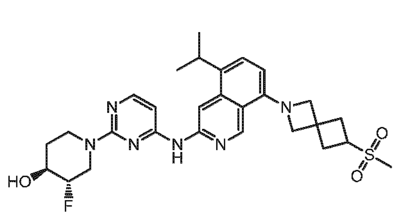,
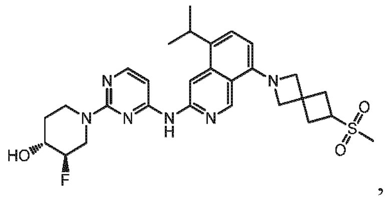,
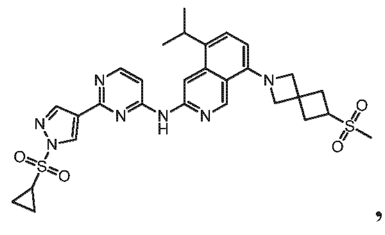,

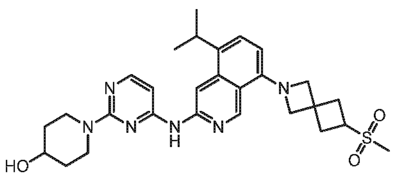,
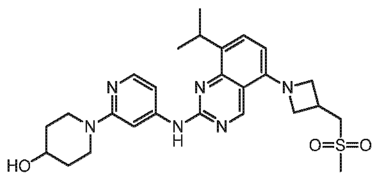,
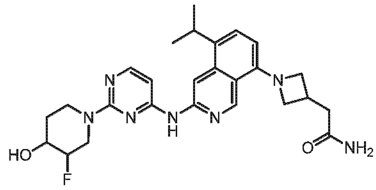,

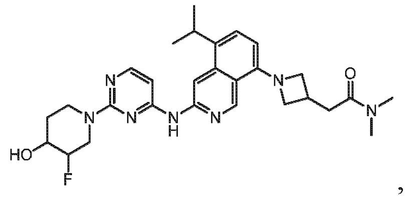,
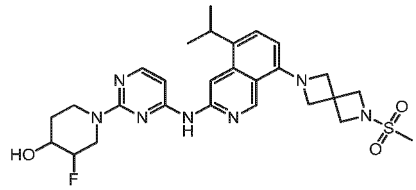,
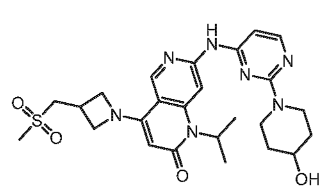,

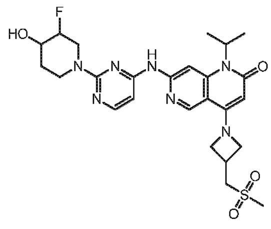,
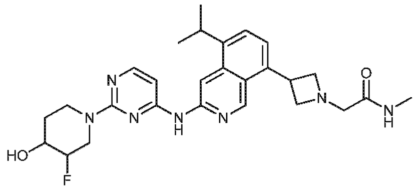,
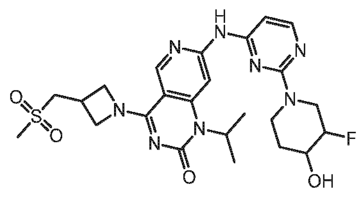,

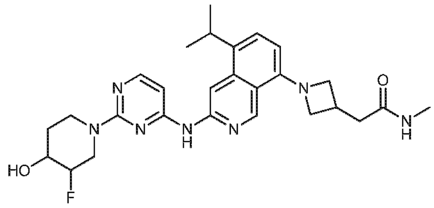,
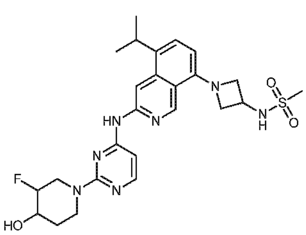,
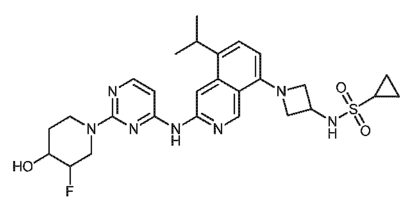,

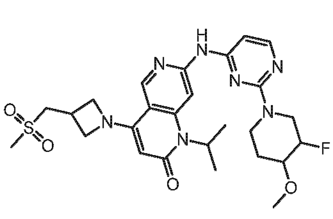,
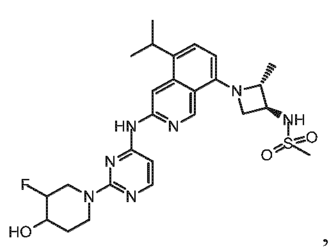,
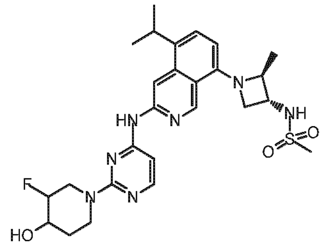,

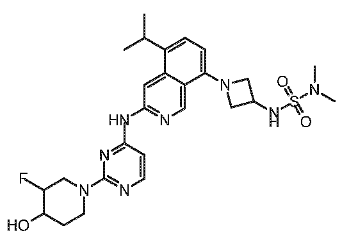, 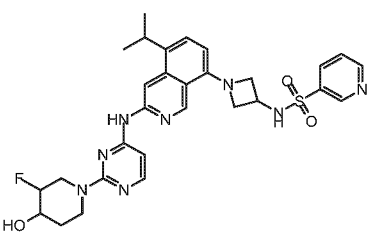, 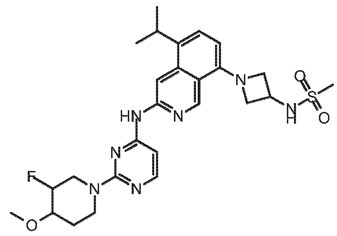,

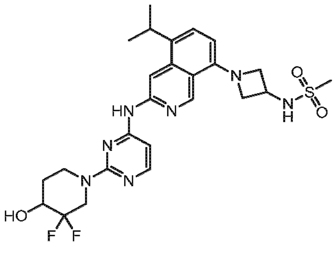, 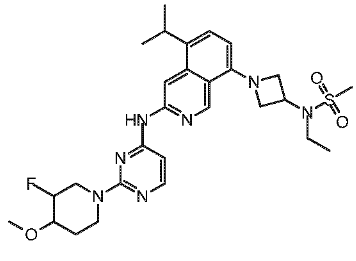, 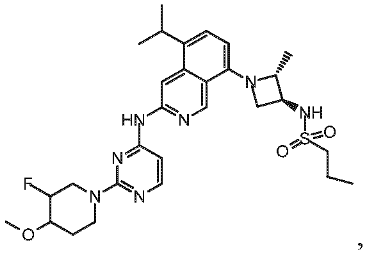,

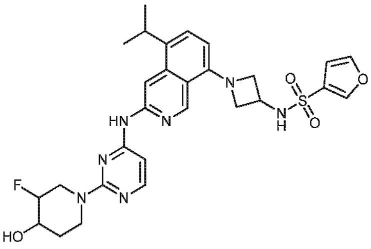, 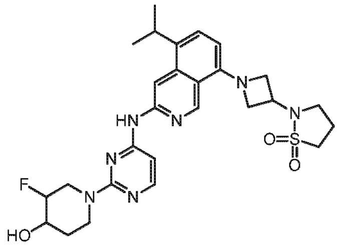, 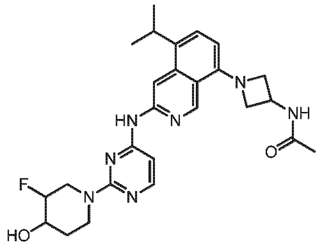,

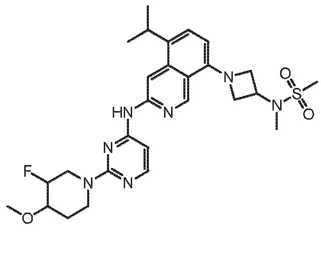, 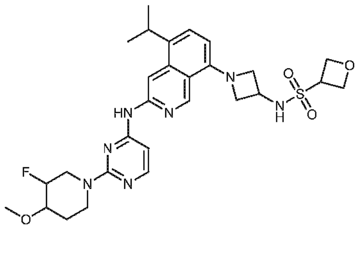, 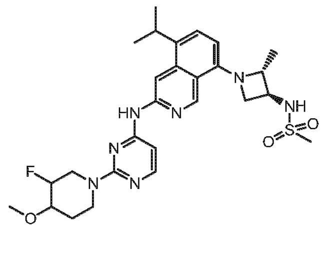,

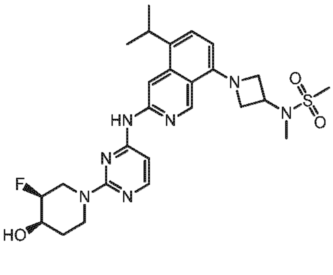, 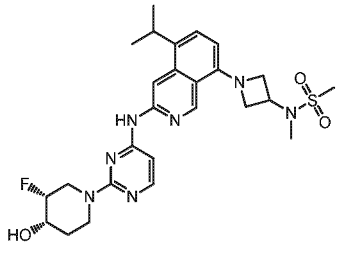, 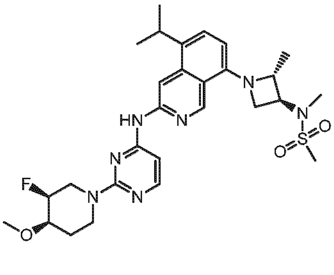,

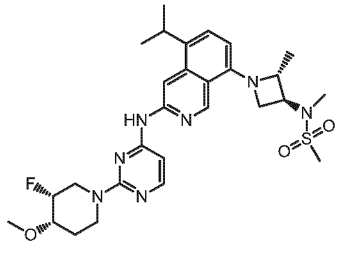, 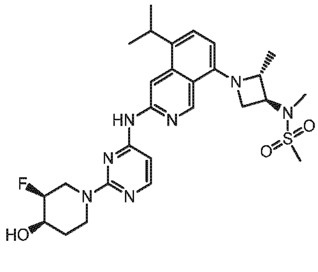, 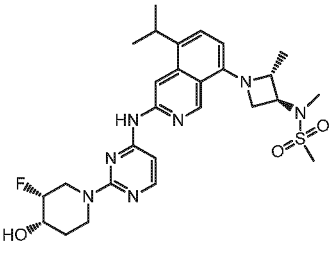,

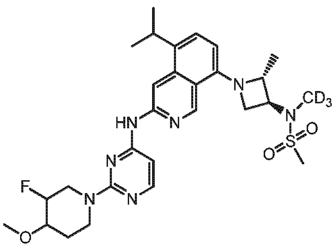 , 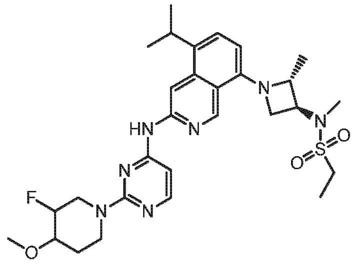 , 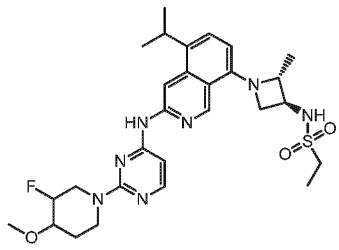 ,

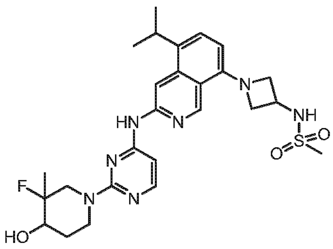 , 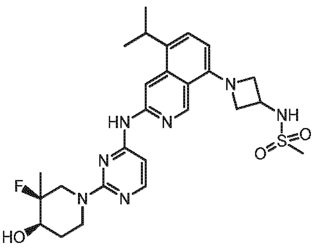 , 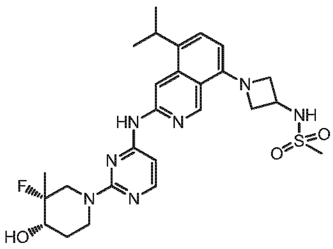 ,

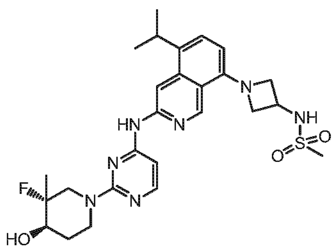 , 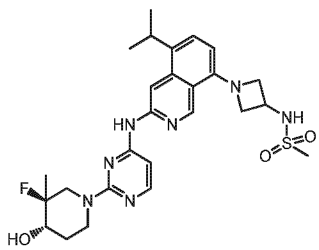 , 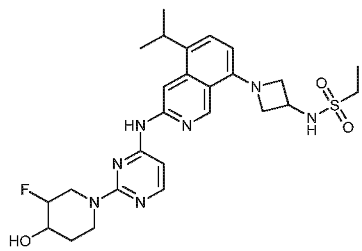 ,

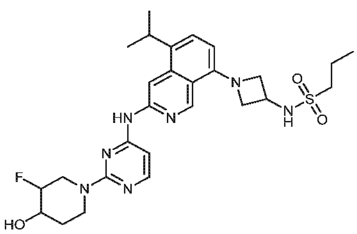 , 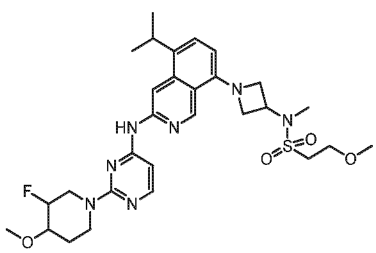 , 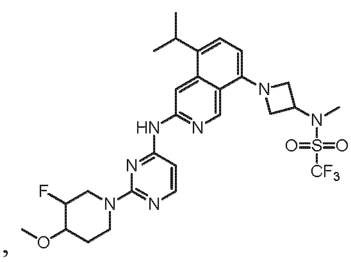 ,

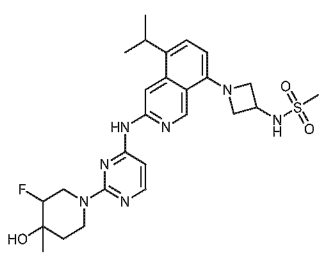 , 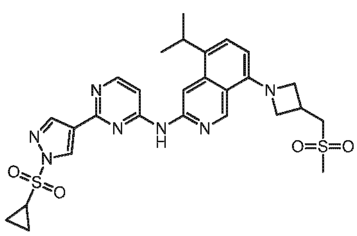 , 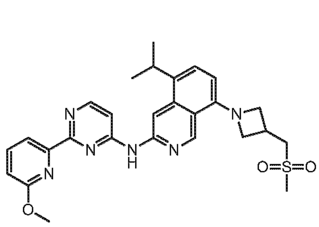 ,

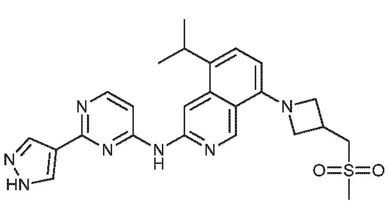 , 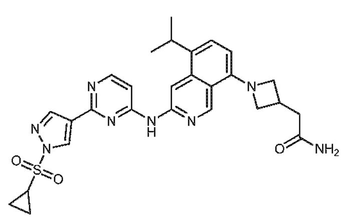 , 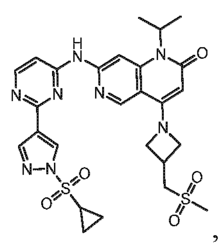 ,

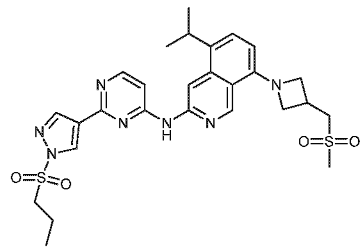 , 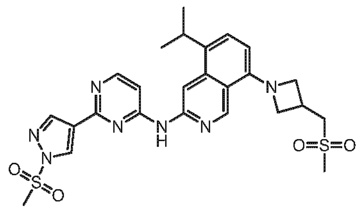 , 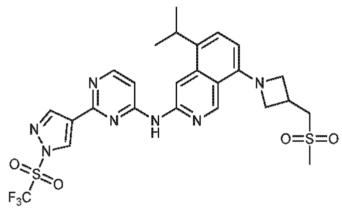 ,

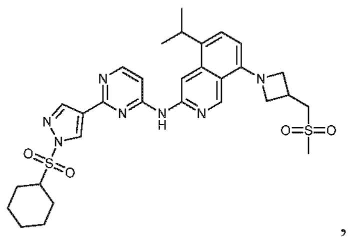 , 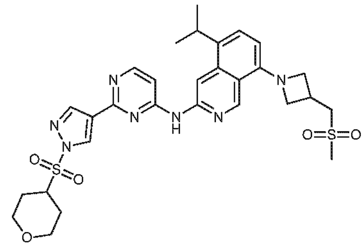 , 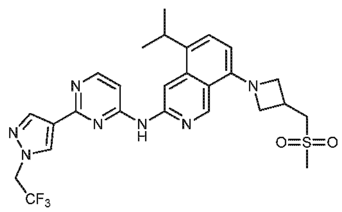 ,

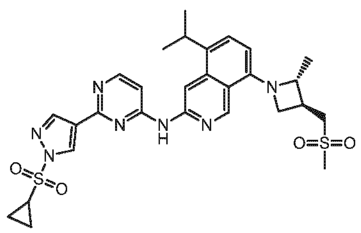 , 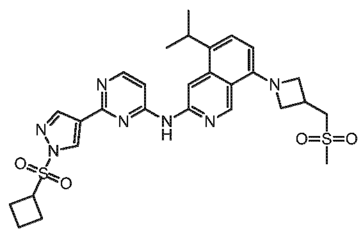 , 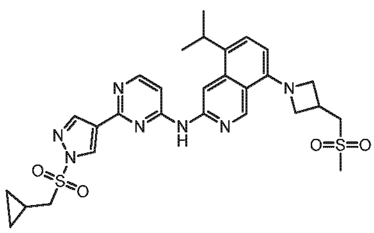 ,

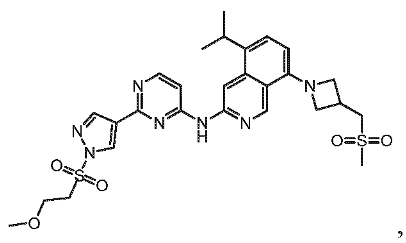 , 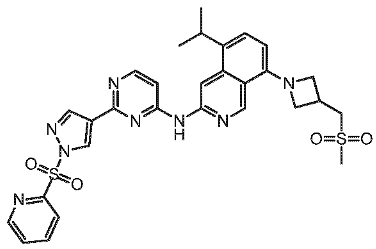 , 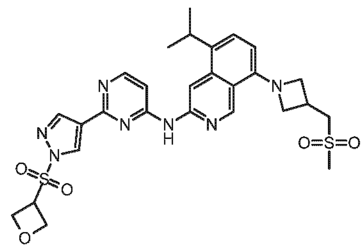 ,

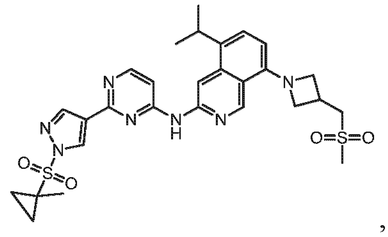 , 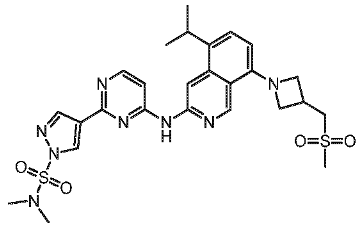 , 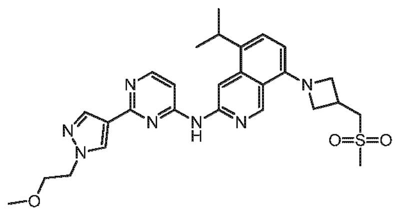 ,

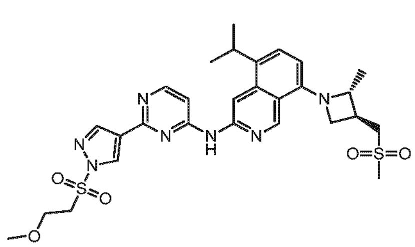 , 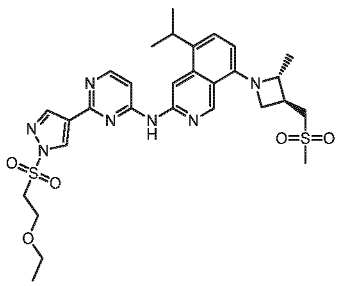 , 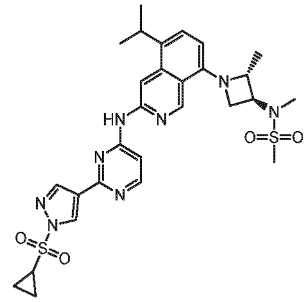 ,

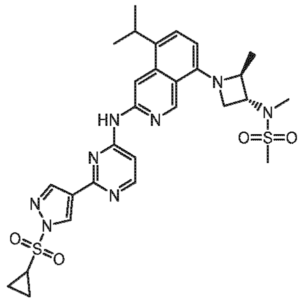, 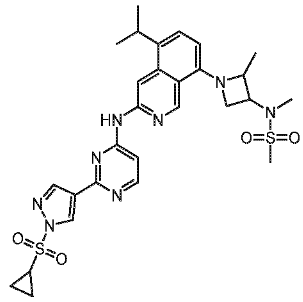, 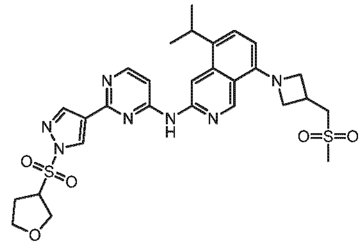,

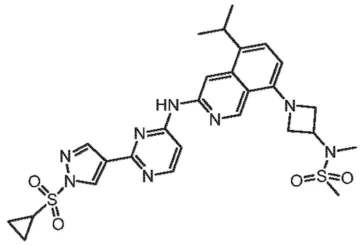, 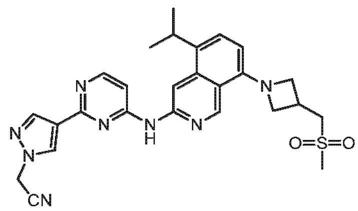, 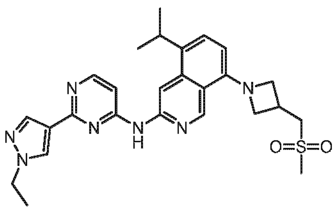,

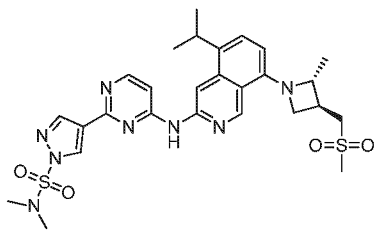, 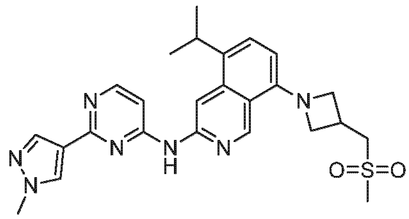, 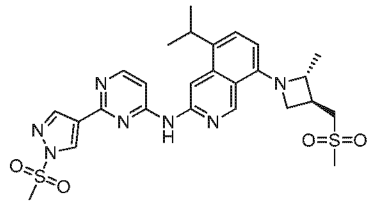,

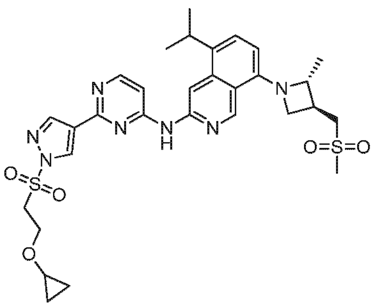, 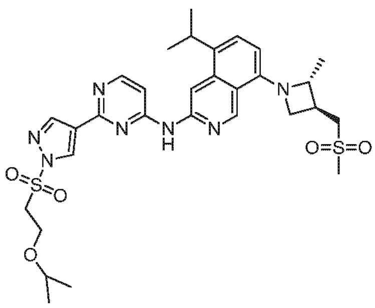, 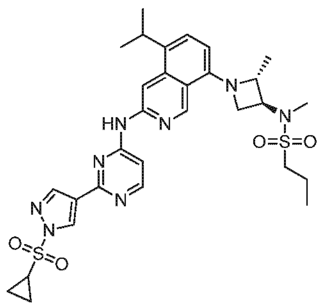,

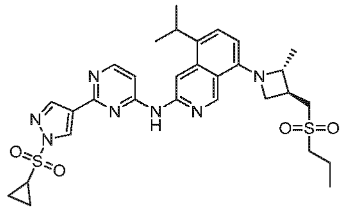 and 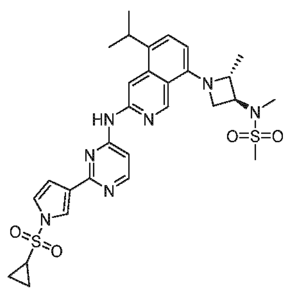.

**33.** A pharmaceutical composition comprising the compound according to any one of claims 1 to 32, or a pharmaceutically acceptable salt, enantiomer, diastereomer, solvate, hydrate or isotopic variant thereof, and a pharmaceutically acceptable excipient; alternatively, further comprising other therapeutic agent.

**34.** Use of the compound according to any one of claims 1 to 32 or a pharmaceutically acceptable salt, enantiomer, diastereomer, solvate, hydrate or isotopic variant thereof in the manufacture of a medicament for treating and/or

preventing a disease mediated by EGFR protein and mutants thereof.

**35.** A method for treating and/or preventing a disease mediated by EGFR protein and mutants thereof in a subject, the method comprising administering to the subject the compound according to any one of claims 1 to 32 or a pharmaceutically acceptable salt, enantiomer, diastereomer, solvate, hydrate or isotopic variant thereof or the pharmaceutical composition according to claim 33.

**36.** The compound according to any one of claims 1 to 32 or a pharmaceutically acceptable salt, enantiomer, diastereomer, solvate, hydrate or isotopic variant thereof or the pharmaceutical composition according to claim 33, for use in treating and/or preventing a disease mediated by EGFR protein and mutants thereof.

**37.** The use according to claim 34 or the method according to claim 35 or the compound or composition for use according to claim 36, wherein the EGFR mutant is selected from one or more of L858R mutant, del19 mutant, T790M mutant, and C797S mutant; optionally, the disease is selected from lung cancer, colon cancer, urothelial carcinoma, breast cancer, prostate cancer, brain cancer, ovarian cancer, gastric cancer, pancreatic cancer, head and neck cancer, bladder cancer and mesothelioma, alternatively non-small cell lung cancer.

## INTERNATIONAL SEARCH REPORT

International application No.
**PCT/CN2022/128264**

**A. CLASSIFICATION OF SUBJECT MATTER**

C07D 401/14(2006.01)i； C07D 471/04(2006.01)i； C07D 491/04(2006.01)i； C07D 491/08(2006.01)i； C07D 491/10(2006.01)i； C07D 519/00(2006.01)i； A61P 35/00(2006.01)i； A61K 31/506(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; STN; CNKI; ISI web of science: 苏州浦合医药科技有限公司, 刘彬, 高峰, 原帅, 赵卉, 彭星哲, 刘翀, 邵宁, 郭永起, 吴勇勇, 吴卓, 哌啶 10d 嘧啶, 吡唑 10d 嘧啶, 吡咯 10d 嘧啶, 异喹啉, 萘啶, 氮杂环丁烷, EGFR, 癌症, 肿瘤, 肺癌, +piperidin 10d pyrimidin+, +pyrazoly 10d pyrimidin+, +pyrr??yl 10d pyrimidin+, +isoquinolin+, +naphthyridin+, +azetidin+, tumor, tumour, cancer, lung cancer, 结构式I, structural formula I

**C. DOCUMENTS CONSIDERED TO BE RELEVANT**

| Category* | Citation of document, with indication, where appropriate, of the relevant passages | Relevant to claim No. |
|---|---|---|
| X | WO 2021133809 A1 (BLUEPRINT MEDICINES CORPORATION) 01 July 2021 (2021-07-01)<br>claims 1-26, description pages 5-27, pages 200-289 table 1 | 1-3, 5-10, 17-26, 32-37 |
| A | WO 2021133809 A1 (BLUEPRINT MEDICINES CORPORATION) 01 July 2021 (2021-07-01)<br>claims 1-26, description pages 5-27, pages 200-289 table 1 | 4, 11-16, 27-31 |
| PX | MEREDITH, S.E. et al. "Discovery of BLU-945, a Reversible, Potent, and Wild-Type-Sparing Next-Generation EGFR Mutant Inhibitor for Treatment-Resistant Non-Small-Cell Lung Cancer"<br>*Journal of Medicinal Chemistry,* Vol. vol. 65, 15 July 2022 (2022-07-15), ISSN: 0022-2623,<br>page 9663 figure 2, page 9665 table 2, page 9667 table 3, page 9668 table 5, page 9671 scheme 4 | 1-3, 5-10, 17-26, 32-37 |
| A | CN 104955824 A (GENENTECH, INC.) 30 September 2015 (2015-09-30)<br>entire document | 1-37 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2022** | **16 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No. **PCT/CN2022/128264**

**C. DOCUMENTS CONSIDERED TO BE RELEVANT**

| Category* | Citation of document, with indication, where appropriate, of the relevant passages | Relevant to claim No. |
|---|---|---|
| A | CN 101360746 A (KUDOS PHARMACEUTICALS LIMITED) 04 February 2009 (2009-02-04) entire document | 1-37 |
| A | WO 2021168074 A1 (THESEUS PHARMACEUTICALS INC.) 26 August 2021 (2021-08-26) entire document | 1-37 |

Form PCT/ISA/210 (second sheet) (January 2015)

**INTERNATIONAL SEARCH REPORT**

International application No.
**PCT/CN2022/128264**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **35, 37**
because they relate to subject matter not required to be searched by this Authority, namely:

[1] Claim 35 relates to a method for treatment and/or prevention of diseases mediated by an EGFR protein and a mutant thereof in a subject, which is a method for the treatment of diseases that is practiced on a living human or animal body for the direct purpose of disease treatment, and belongs to the cases set out in PCT Rule 39.1(iv) for which an international search is not required.

[2] When claim 37 refers to the method of claim 35, the subject matter thereof also falls within the scope of methods for treatment of a living human or animal body, and belongs to the cases set out in PCT Rule 39.1(iv) for which an international search is not required.

[3] The search regarding claims 35 and 37 is carried out on the basis of the following reasonably expected amendments:

[4] the subject matter of claim 35 is amended to "the use of the compound or the pharmaceutically acceptable salt thereof, enantiomer, diastereomer, solvate, and hydrate or isotopic variant of any one of claims 1-32, or the pharmaceutical composition of claim 33 in the preparation of a drug for the treatment and/or prevention of diseases mediated by the EGFR protein and the mutant thereof"; and the subject matter of claim 37 when referring to claim 35 is amended to "the use of claim 35".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/128264**

| Patent document cited in search report | Publication date (day/month/year) | Patent family member(s) | Publication date (day/month/year) |
|---|---|---|---|
| WO 2021133809 A1 | 01 July 2021 | CA 3163007 A1 | 01 July 2021 |
| | | AU 2020412710 A1 | 11 August 2022 |
| | | CN 115135642 A | 30 September 2022 |
| CN 104955824 A | 30 September 2015 | CA 2891655 A1 | 30 May 2014 |
| | | WO 2014081718 A1 | 30 May 2014 |
| | | KR 20150087850 A | 30 July 2015 |
| | | EP 2922851 A1 | 30 September 2015 |
| | | JP 2016500075 A | 07 January 2016 |
| | | MX 2015006152 A1 | 20 January 2016 |
| | | US 2016016948 A1 | 21 January 2016 |
| | | RU 2015118647 A | 10 January 2017 |
| | | EP 3181564 A1 | 21 June 2017 |
| | | BR 112015011456 A2 | 11 July 2017 |
| | | EP 2922851 B1 | 26 July 2017 |
| | | CN 104955824 B | 22 September 2017 |
| | | EP 2922851 B8 | 27 September 2017 |
| | | US 9890152 B2 | 13 February 2018 |
| | | HK 1238646 A0 | 04 May 2018 |
| | | JP 6430390 B2 | 28 November 2018 |
| | | EP 3181564 B1 | 18 September 2019 |
| | | HK 1238646 A1 | 23 October 2020 |
| CN 101360746 A | 04 February 2009 | US 2008194546 A1 | 14 August 2008 |
| | | CA 2628920 A1 | 31 May 2007 |
| | | WO 2007060404 A1 | 31 May 2007 |
| | | TW 200738706 A | 16 October 2007 |
| | | TW 200817395 A | 16 April 2008 |
| | | MX 2008006502 A1 | 31 May 2008 |
| | | AU 2006318948 A1 | 12 June 2008 |
| | | SG 142729 A1 | 27 June 2008 |
| | | KR 20080070079 A | 29 July 2008 |
| | | EP 1954699 A1 | 13 August 2008 |
| | | NO 20082101 A | 15 August 2008 |
| | | IN 200803973 P1 | 20 March 2009 |
| | | JP 2009516727 A | 23 April 2009 |
| | | ZA 200804104 A | 25 November 2009 |
| | | AU 2006318948 B2 | 24 February 2011 |
| | | SG 142729 B | 15 April 2011 |
| | | NZ 567787 A | 29 July 2011 |
| | | BRPI 0618874 A2 | 13 September 2011 |
| | | RU 2445315 C2 | 20 March 2012 |
| | | EP 1954699 B1 | 19 September 2012 |
| | | IL 191196 A | 24 September 2012 |
| | | MX 305846 B | 06 December 2012 |
| | | ES 2394470 T3 | 01 February 2013 |
| | | JP 5161102 B2 | 13 March 2013 |
| | | JP 2013107915 A | 06 June 2013 |
| | | HK 1124039 A1 | 07 June 2013 |
| | | PH 12011502370 B1 | 02 September 2013 |
| | | MX 313401 B | 18 September 2013 |
| | | CN 101360746 B | 11 December 2013 |

Form PCT/ISA/210 (patent family annex) (January 2015)

**INTERNATIONAL SEARCH REPORT**
**Information on patent family members**

International application No.
**PCT/CN2022/128264**

| Patent document cited in search report | Publication date (day/month/year) | Patent family member(s) | Publication date (day/month/year) |
|---|---|---|---|
| | | PH 12008501200 B1 | 06 April 2014 |
| | | TW I452047 B | 11 September 2014 |
| | | JP 5629343 B2 | 19 November 2014 |
| | | KR 101464384 B1 | 21 November 2014 |
| | | MY 153209 A | 29 January 2015 |
| | | CA 2628920 C | 29 December 2015 |
| | | IN 285629 B | 28 July 2017 |
| | | NO 341055 B1 | 14 August 2017 |
| WO 2021168074 A1 | 26 August 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *Nature Reviews Disease Primers,* 2015, vol. 1, 15009
- *Nature Medicine,* 2015, vol. 21, 560-562
- *Nature Cancer,* 2021, 377-391
- *The new England journal of medicine,* 2018, vol. 378, 113-125
- Prodrugs as Novel Delivery Systems, A.C.S. Symposium Series. **T. HIGUCHI ; V. STELLA.** Bioreversible Carriers in Drug Design. American Pharmaceutical Association and Pergamon Press, 1987, vol. 14
- **D. FLEISHER ; S. RAMON ; H. BARBRA.** Improved oral drug delivery: solubility limitations overcome by the use of prodrugs. *Advanced Drug Delivery Reviews,* 1996, vol. 19 (2), 115-130
- Remington's Pharmaceutical Sciences,. Mack Publishing Company, 1985